# EUROPÄISCHE PATENTSCHRIFT

(11) **EP 0 302 164 B1**
(45) Veröffentlichungstag und Bekanntmachung des Hinweises auf die Patenterteilung: **13.01.1993**
(21) Anmeldenummer: 88102371.7
(22) Anmeldetag: 18.02.1988
(51) Int. Cl.: C07D 401/12, C07D 233/24, C07D 417/12, C07D 417/14, A61K 31/155, A61K 31/415

(54) **Guanidincarbonsäureester, Verfahren zu ihrer Herstellung und diese Verbindungen enthaltende Arzneimittel**
Guanidine-carboxylic-acid esters, method for their preparation and medicines containing these compounds
Esters d'acides guanidinecarboxyliques, procédés pour leur préparation et médicaments contenant ces composés

(30) Priorität: 07.08.1987 DE 3726381
(43) Veröffentlichungstag der Anmeldung: 08.02.1989
(73) Patentinhaber: HEUMANN PHARMA GMBH & CO, D-90478 Nürnberg (DE)
(72) Erfinder: Mörsdorf, Peter, Dr. Dipl.-Chem., D-Langenzenn (DE); Schickaneder, Helmut, Dr. Dipl.-Chem., D-8501 Eckental (DE); Pfahlert, Volker, Dr. Pharm., D-8500 Nürnberg (DE); Engler, Heidrun, Dr.-Vet., D-8501 Cadolzburg (DE); Buschauer, Armin, Dr.-Pharm., D-1000 Berlin 41 (DE); Schunack, Walter, Prof.Dr.Dr., D-1000 Berlin 38 (DE)
(74) Vertreter: Kraus, Walter, Dr.

(56) Entgegenhaltungen:
- EP-A- 0 199 845
- DE-A- 2 053 175
- DE-A- 3 512 084
- DE-A- 3 528 214
- DE-A- 3 528 215
- GB-A- 1 305 547
- US-A- 3 881 015
- CHEMICAL ABSTRACTS, Band 103, Nr. 19, 11. November 1985, Columbus, Ohio, US; G.J. DURANT et al.: "The histamine H2-receptor agonist impromidine: synthesis and structure activity considerations" Seite 23, Spalte 2, Zusammenfassung-Nr. 153 321a

## Beschreibung

Histamin-H₂-Agonisten, wie zum Beispiel Impromidin (G.J. Durant et al., Nature (London) 1978, 276, 403), führen über eine Stimulierung der H₂-Rezeptoren des Herzens in vivo zu einer deutlichen Kontraktilitätssteigerung und wurden aufgrund ihrer positiv inotropen Wirkung zur Behandlung der Herzinsuffizienz und insbesondere der kongestiven Cardiomyopathie vorgeschlagen.

Die DE-OS'en 35 12 084, 35 28 214 und 35 28 215 und die EP-OS 0 199 845 beschrieben neue H₂-Agonisten, in denen durch den Einbau zusätzlicher Hi-antagonistischer Bausteine einerseits unerwünschte Nebenwirkungen des Impromidins beseitigt werden konnten, andererseits aber auch die Wirkstärke der Verbindungen erheblich gesteigert werden konnte. Diese Verbindungen zeigen bei parenteraler Applikation ausgezeichnete Wirkungen, sind aber bei oraler Verabreichung deutlich schwächer wirksam.

Der Erfindung liegt deshalb die Aufgabe zugrunde, H₂-Agonisten mit eventuell zusätzlicher Hi-antagonistischer Komponente zur Verfügung zu stellen, die sich durch eine verbesserte orale Wirksamkeit auszeichnen.

Diese Aufgabe wird durch die Erfindung gelöst.

Gegenstand der Erfindung sind Guanidincarbonsäureester der allgemeinen Formel I 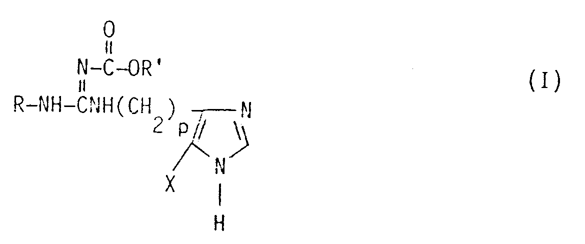 in der
(a) R die Gruppierung 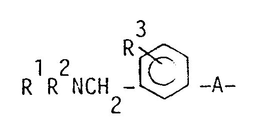 bedeutet, wobei R¹ und R², die gleich oder verschieden sein können, jeweils für Wasserstoff, lineares C₁-C₆-Alkyl oder C₅-C₆-Cycloalkyl stehen oder R¹ und R² zusammen mit dem Stickstoffatom, an das sie gebunden sind, einen 5- bis 10gliedrigen stickstoffhaltigen alicyclischen, heterocyclischen Ring bilden, R³ für ein Wasserstoffatom, ein Halogenatom oder eine C₁-C₃-Alkoxygruppe steht, A die Gruppierung -0-(CH₂)ₖ-, -O-CH₂CH(OH)CH₂-, -0-CH₂CH(CH₃)-CH₂-,-CH₂-0-CH₂-CH(OH)-CH₂- oder -O-CH₂-CH(OH)-CH(OH)-CH₂ bedeutet, worin k den Wert 3 oder 4 hat, oder
(b) R die Gruppierung 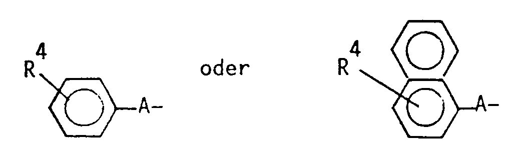 bedeutet, wobei R⁴ für ein Wasserstoffatom, ein vorzugsweise in para-Stellung zu A gebundenes Halogenatom, eine C₁-C₃-Alkoxy- oder C₁-C₃-Alkylgruppe steht und A die oben unter (a) genannte Bedeutung besitzt, oder
(c) R die Gruppierung 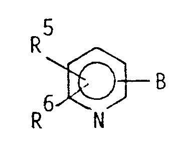 bedeutet, wobei R⁵ und R⁶, die gleich oder verschieden sein können, jeweils für ein Wasserstoffatom, ein Halogenatom oder eine lineare C₁-C₃-Alkyl- oder eine lineare C₁-C₃-Alkoxygruppe stehen, B in Position 2, 3 oder 4 des Pyridinrings gebunden sein kann und die Gruppierung 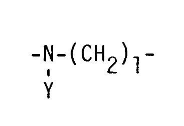 oder -(CH₂)ₘ- bedeutet, wobei I den Wert 2, 3 oder 4 und m den Wert 3, 4 oder 5 haben und Y für ein Wasserstoffatom oder für eine lineare C₁-C₃-Alkylgruppe steht, oder
(d) R die Gruppierung 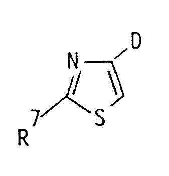 bedeutet, wobei R⁷ für die Gruppe (R¹ R²)N-CH₂-, (H₂N)₂C = N-, 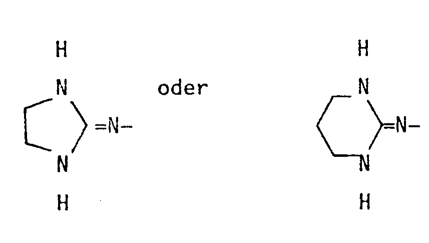 steht, wobei R¹ und R² die oben unter (a) angegebene Bedeutung besitzen, D für die Gruppierung -CH₂-S-(CH₂)ₙ- oder -(CH₂)ₒ- steht, wobei n den Wert 2 oder 3 und o den Wert 2, 3 oder 4 haben, oder
(e) R die Gruppierung 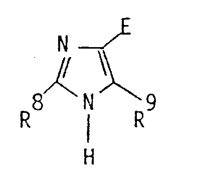 bedeutet, wobei R⁸ für ein Wasserstoffatom, eine gegebenenfalls durch ein Halogenatom substituierte Benzylgruppe, die Gruppierung (R¹ R²)N-CH₂ - oder eine Aminogruppe steht, wobei R¹ und R² die oben unter (a) genannten Bedeutungen haben, R⁹ ein Wasserstoffatom, eine lineare C₁-C₃-Alkyl- oder C₁-C₃-Alkylthiogruppe bedeutet, E für die Gruppierung 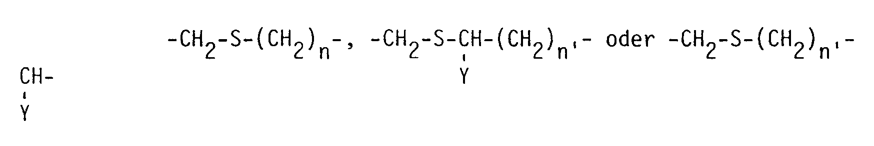 steht, wobei n den Wert 2 oder 3 und n' den Wert 1, 2 oder 3 hat und Y die oben unter (c) angegebene Bedeutung besitzt, oder
(f) R die Gruppierung 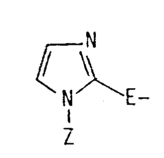 bedeutet, wobei Z ein Wasserstoffatom oder eine lineare C₁-C₃-Alkylgruppe bedeutet und E die oben unter (e) genannte Bedeutung besitzt, oder
(g) R die Gruppierung R"-A'-B'- bedeutet, wobei R" für eine unsubstituierte oder mit Halogen, linearem C₁-C₃-Alkyl oder linearem C₁-C₃-Alkoxy substituierte Phenylgruppe oder eine unsubstituierte oder mit Halogen, linearem C₁-C₃-Alkyl oder linearem C₁-C₃-Alkoxy substituierte Naphthylgruppe steht, A' für eine Einfachbindung oder für die Gruppierung -CR^{1'}R^{2'} oder für ein - mit einer Phenyl-, Naphthyl-, Benzyl-, Pyridinyl-, Furanyl- oder Thiophenylgruppe, die gegebenenfalls mit Halogen, linearem Cₗ-C₃-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Pyridinyl-, Furanyl- oder Thiophenylgruppe, die gegebenenfalls mit Halogen, linearem C₁-C₃-Alkyl oder linearem C₁-C₃-Alkoxy substituiert ist, steht, B' für die Gruppierung -CH(Y)-S-(CH2)m-, -CH₂-S-CH₂-CH(Y)-CH₂-, -CH₂-S-CH(Y)-CH₂-, -CH₂-S-CH₂-CH(Y)-, -(CH₂ )_{n"}-, -(CH₂)-CH(Y)-, -(CH₂)_{n,},-CH-(Y)-, -O-(CH₂)₂-, -CH2-0-(CH2)o-, -CH₂-O-CH₂-CH(Y)-CH₂-, -O-CH₂-CH(Y)-, -O-CH(Y)-CH₂-, -S-(CH₂)q-, -S-CH₂-CH(Y)-, -S-CH(Y)-CH₂ oder -S-CH₂-CH(Y)-CH₂- steht, wobei Y ein Wasserstoffatom oder eine lineare C₁-C₃-Alkylgruppe bedeutet, m' und o' jeweils den Wert 2 oder 3 haben, n" und q jeweils den Wert 2, 3, 4 oder 5 haben, oder
(h) R die Gruppierung R"'-A"-B"- bedeutet, wobei R"' für eine unsubstituierte oder mit Halogen, linearem C₁-C₃-Alkyl oder linearem C₁-C₃-Alkoxy substituierte Pyridinyl-, Imidazolyl-, Pyrimidinyl-, Thiophenyl-, Furanyl-, Benzimidazolyl- oder Chinolinylgruppe steht, an die gegebenenfalls ein Phenylring ankondensiert sein kann, A" für eine Einfachbindung oder für die Gruppierung -CR^{1'}R^{2'} oder für ein - mit einer Phenyl-, Naphthyl-, Benzyl-, Pyridinyl-, Benzimidazolyl-, Thiophenyl- oder Furanylgruppe, die gegebenenfalls mit Halogen, linearem C₁-C₃-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem Cₗ-C₃-Alkyl oder linearem C₁-C₃-Alkoxy substituiert ist, oder eine Benzimidazolyl-, Pyridinyl-, Thiophenyl- oder Furanylgruppe, die gegebenenfalls mit Halogen, linearem C₁-C₃-Alkyl oder linearem C₁-C₃-Alkoxy substituiert ist, steht, B" für die Gruppierung -CH(Y)-S-(CH2)m-, -CH₂-S-CH₂-CH(Y)-CH₂-, -CH₂-S-CH(Y)-CH₂-, -(CH₂)ₙ,,-CH(Y)-, -CH₂-S-CH₂-CH(Y)-, -(CH₂)_{n"}-' -O(CH₂)_{n"}-' -S-CH₂-CH(Y)-, S-CH(Y)-CH₂-, -S-(CH₂)q- oder -S-CH₂-CH(Y)-CH₂- steht, wobei Y ein Wasserstoffatom oder eine lineare C₁-C₃-Alkylgruppe bedeutet, m' den Wert 2 oder 3 hat und n" und q den Wert 2, 3, 4 oder 5 haben,

R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet, sowie die physiologisch annehmbaren Salze davon.

In der allgemeinen Formel I hat der Substituent R verschiedene Bedeutungen gemäß den Ausführungsformen (a) bis (h) der vorliegenden Erfindung.

Bei allen Ausführungsformen steht R' für eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-₆-Alklygruppe, vorzugsweise C₁-C₄-Alkylgruppe. Im Falle der Substitution ist eine Ein- bis Dreifachsubstitution mit einem Halogenatom, zum Beispiel Chlor-, Brom- oder Jodatom, einer Ci-C₃-Alkoxygruppe und/oder einem Phenylrest bevorzugt. Besonders bevorzugt wird eine unsubstituierte C₁-C₄-Alkylgruppe, wie zum Beispiel die Methyl-, Ethyl-, n-Propyl-, Isopropyl-, n-Butyl- oder sec.-Butylgruppe. R' kann weiterhin eine Cycloalkylgruppe, vorzugsweise eine Cₛ-C₆-Cycloalkylgruppe, bedeuten. X bedeutet ein Wasserstoffatom oder eine Methylgruppe, während p den Wert 2 oder 3, vorzugsweise den Wert 3, hat.

Bei der Ausführungsform (a) bedeutet R die Gruppierung 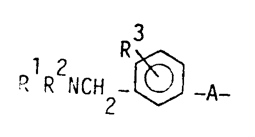 wobei R¹ und R², die gleich oder verschieden sein können, jeweils ein Wasserstoffatom, eine lineare C₁-C₆-Alkylgruppe, vorzugsweise eine lineare C₁-C₃-Alkylgruppe, zum Beispiel eine Methyl-, Ethyl- oder n-Propylgruppe, insbesondere eine Methylgruppe, oder eine Cs-C₆-Cycloalkylgruppe bedeuten. R¹ und R² können aber auch zusammen mit dem Stickstoffatom, an das sie gebunden sind, einen 5- bis 10gliedrigen stickstoffhaltigen heterocyclischen Ring bilden. Bevorzugte Beispiele für den so definierten 5- bis 10gliedrigen heterocyclischen Ring sind der Pyrrolidin-, Piperidin- und Homopiperidinring. R³ steht für ein Wasserstoffatom oder ein Halogenatom, beispielsweise ein Chlor-, Brom- oder Jodatom, das in ortho-, meta- oder para-Stellung, vorzugsweise in ortho-Stellung zu der (R¹ R²)NCH₂-Gruppierung gebunden ist. R³ kann weiterhin eine C₁-C₃-Alkoxygruppe, zum Beispiel eine Methoxy-, Ethoxy- oder Propoxygruppe, vorzugsweise eine Methoxygruppe, die ebenfalls in ortho-, meta- oder para-Stellung, vorzugsweise in para-Stellung zur (R¹R²)NCH₂-Gruppierung gebunden ist, sein. A bedeutet eine der folgenden Gruppierungen: 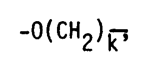 -O-CH₂CH(OH)CH₂-, -O-CH₂-CH(CHₐ)-CH₂-, -CH₂-O-CH₂-CH(OH)-CH₂- oder -O-CH₂-CH(OH)-CH(OH)-CH₂- . Darin hat k den Wert 3 oder 4, wobei der Wert 3 bevorzugt wird.

Bei der Ausführungsform (b) bedeutet R die Gruppierung 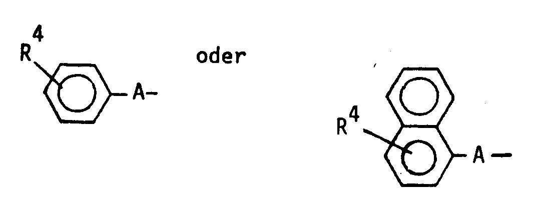 wobei R⁴ für ein Wasserstoffatom, ein vorzugsweise in para-Stellung zu A gebundenes Halogenatom, beispielsweise ein Chlor-, Brom- oder Jodatom, vorzugsweise ein Chloratom, eine C₁-C₃-Alkoxygruppe, beispielsweise eine Methoxy- oder Ethoxygruppe, eine C₁-C₃-Alkylgruppe, beispielsweise eine Methyl- oder Ethylgruppe, ganz besonders bevorzugt ein Wasserstoffatom, steht. A hat die im Zusammenhang mit der Ausführungsform (a) angegebene Definition, wobei die Gruppierung -O-(CH₂)₃- oder -O-CH₂CH(OH)CH₂- bevorzugt wird.

Bei der Ausführungsform (c) bedeutet R die Gruppierung 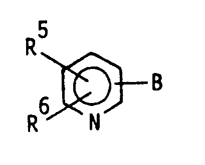 wobei R⁵ und R⁶, die gleich oder verschieden sein können, jeweils für ein Wasserstoffatom, ein Halogenatom, beispielsweise für ein Fluor-, Chlor- oder Bromatom, vorzugsweise für ein Bromatom, stehen. Das Halogenatom bzw. die Halogenatome ist bzw. sind vorzugsweise in 3- und/oder 5-Position des Pyridinrings gebunden. Wenn R⁶ ein Wasserstoffatom ist, dann steht R⁵ Vorzugsweise für ein Halogenatom, beispielsweise ein Fluor-, Chlor- oder Bromatom, insbesondere ein Bromatom. R⁵ und R⁶ können weiterhin jeweils eine C1-C3-Alkyl- oder C₁-C₃-Alkoxygruppe wie oben unter (a) definiert sein. Diese Gruppe bzw. Gruppen kann bzw. können vorzugsweise in 3- und/oder 5-Position des Pyridinrings gebunden sein. Methyl- oder Methoxygruppen werden für R⁵ und R⁶ bevorzugt. Steht R⁵ für ein Wasserstoffatom, dann bedeutet R⁶ vorzugsweise eine in 3- oder 5-Position des Pyridinrings gebundene Methyl- oder Methoxygruppe.

B, das in 2-, 3- oder 4-Position des Pyridinrings, vorzugsweise in 2- oder 3-Position, gebunden sein kann, steht für die Gruppierung 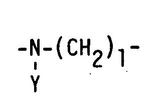 oder -(CH₂)ₘ-, wobei Y ein Wasserstoffatom oder eine lineare C₁-C₃-Alkylgruppe, vorzugsweise eine Methyl-, Ethyl-, n-Propyl- oder Isopropylgruppe, insbesondere eine Methylgruppe, bedeutet. 1 hat den Wert 2, 3 oder 4, vorzugsweise 2 oder 3, während m den Wert 3, 4 oder 5, vorzugsweise 4, hat.

Bei der Ausführungsform (d) bedeutet R die Gruppierung 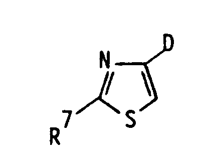 Darin kann R⁷ als Substituent die Gruppe (R¹ R²)N-CH₂- sein, in der R¹ und R², die gleich oder verschieden sein können, vorzugsweise eine lineare Ci-C₆-Alkylgruppe, besonders bevorzugt eine lineare C₁-C₄-Alkylgruppe, beispielsweise eine Methyl-, Ethyl- oder n-Propylgruppe, insbesondere eine Methylgruppe, sind. Weiterhin kann R⁷ eine der Gruppierungen (H₂ N)₂ C = N-, 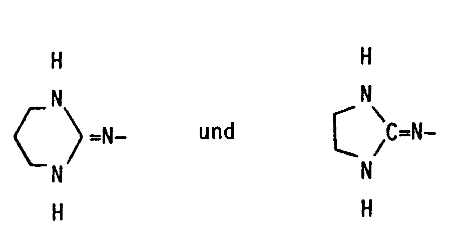 sein, wobei eine der beiden letztgenannten Gruppierungen ganz besonders bevorzugt wird. D bedeutet ein Verbindungsglied -CH₂-S-(CH₂)ₙ- oder -(CH₂)ₒ-, wobei n den Wert 2 oder 3, vorzugsweise 2, und o den Wert 2, 3 oder 4, vorzugsweise 3, hat.

Bei der Ausführungsform (e) bedeutet R die Gruppierung 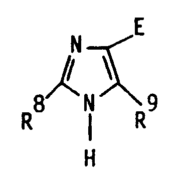 wobei R⁸ ein Wasserstoffatom oder eine gegebenenfalls durch ein Halogenatom, wie zum Beispiel ein Fluor-, Brom- oder Chloratom, vorzugsweise ein Chloratom, substituierte Benzylgruppe bedeutet, wobei die para-Stellung bevorzugt wird. R⁸ kann auch die Gruppierung (R¹ R²)N-CH₂- bedeuten, in der R¹ und R² die oben unter (a) genannten Bedeutungen haben, vorzugsweise aber jeweils für eine Methylgruppe stehen. R⁸ kann weiterhin auch eine Aminogruppe sein. Vorzugsweise ist R⁸ jedoch ein Wasserstoffatom. R⁹ steht für ein Wasserstoffatom, eine lineare C₁-C₃-Alkylgruppe, wie zum Beispiel eine Methyl- oder Ethylgruppe, vorzugsweise eine Methylgruppe, oder eine C₁-C₃-Alkylthiogruppe, ganz besonders bevorzugt eine Methylthiogruppe. Wenn R⁸ für ein Wasserstoffatom und R⁹ für eine Methylthiogruppe stehen, dann steht E vorzugsweise für die Gruppierung -CH₂-S-(CH₂)ₙ-, wobei n den Wert 2 oder 3, vorzugsweise 2, hat. E bedeutet weiterhin vorzugsweise die Gruppierung 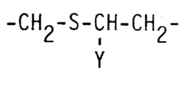 oder 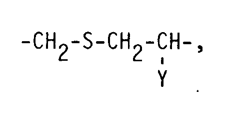 wobei Y für eine C1-C3-Alkylgruppe, zum Beispiel für eine Methyl- oder Ethylgruppe, vorzugsweise für eine Methylgruppe, steht.

Bei der Ausführungsform (f) steht R für die Gruppierung 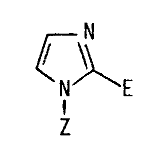 wobei Z ein Wasserstoffatom oder eine lineare C₁-C₃-Alkylgruppe, vorzugsweise eine Methylgruppe, bedeutet, E die gleiche Definition wie im Zusammenhang mit der Ausführungsform (e) angegeben besitzt, aber vorzugsweise die Gruppierung -CH₂-S-(CH₂)₂- bedeutet.

Bei der Ausführungsform (g) bedeutet R die Gruppierung R"-A'-B'-, wobei R" für eine unsubstituierte oder mit Halogen, linearem C₁-C₃-Alkyl oder linearem C₁-C₃-Alkoxy substituierte Phenylgruppe oder eine unsubstituierte oder mit Halogen, linearem C₁-C₃-Alkyl oder linearem C₁-C₃-Alkoxy substituierte Naphthylgruppe steht.

Wenn R" eine so substituierte Phenyl- oder Naphthylgruppe ist, dann kann R" durch die Gruppierung 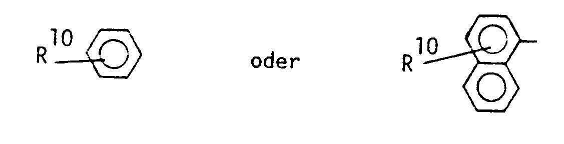 angegeben werden, worin R¹⁰ ein vorzugsweise in meta- oder para-Stellung zu A' gebundenes Halogenatom, beispielsweise ein Fluor-, Brom-, Chlor- oder Jodatom, vorzugsweise ein Fluor- oder Chloratom, ganz besonders bevorzugt ein Fluoratom, eine lineare C₁-C₃-Alkylgruppe, beispielsweise eine Methyl- oder Ethylgruppe, eine lineare C₁-C₃-Alkoxygruppe, beispielsweise eine Methoxygruppe oder eine Trifluormethylgruppe, bedeutet. A' steht für eine Einfachbindung, für die Gruppierung -CR^{1'}R^{2'} oder ein Stickstoffatom, das mit einer Phenyl-, Naphthyl-, Pyridinyl-, Furanyl- oder Thiophenylgruppe, die gegebenenfalls mit Halogen, linearem C₁-C₃-Alkyl oder linearem C₁-C₃-Alkoxy substituiert ist, oder einem Wasserstoffatom oder einer linearen C₁-C₃-Alkylgruppe substituiert ist. Im Falle der Substitution können die genannten Gruppen in meta- oder para-Position, vorzugsweise in para-Position, mit einem Halogenatom, beispielsweise einem Fluor-, Chlor-, Brom- oder Jodatom, vorzugsweise einem Fluoratom, oder mit einer linearen Cₗ-C₃-Alkylgruppe, beispielsweise mit einer Methyl-, Ethyl- oder Propylgruppe, vorzugsweise einer Methylgruppe, oder mit einer linearen C₁-C₃-Alkoxygruppe, beispielsweise einer Methoxy-, Ethoxy- oder Propoxygruppe, vorzugsweise einer Methoxygruppe, substituiert sein. R^{1'} bedeutet ein Wasserstoffatom oder eine Methylgruppe, während R für eine unsubstituierte oder mit Halogen, C₁-C₃-Alkyl oder C₁-C₃-Alkoxy substituierte Phenylgruppe oder eine unsubstituierte oder mit Halogen, C1-C3-Alkyl oder C₁-C₃-Alkoxy substituierte Pyridinyl-, Furanyl- oder Thiophenylgruppe steht. Im Falle der Substitution einer solchen Gruppe ist diese mit einem Halogenatom, beispielsweise einem Fluor-, Brom-, Chlor- oder Jodatom, vorzugsweise einem Fluor- oder Chloratom, einer linearen C₁-C₃-Alkylgruppe, beispielsweise einer Methyl- oder Ethylgruppe, oder einer linearen C₁-C₃-Alkoxygruppe, beispielsweise einer Methoxygruppe, substituiert. B' steht für eine der Gruppierungen -CH(Y)-S-(CH2)m-, -CH₂-S-CH₂-CH(Y)-CH₂-, -CH₂-S-CH(Y)-CH₂-, -CH₂-S-CH₂-CH(Y), -(CH₂)_{n"}-, -CH₂-CH(Y)-, -(CH₂)_{n"}-CH(Y)-, -O-(CH₂)₂-, -CH₂-O-(CH₂)ₒ-, -CH₂-O-CH₂-CH(Y)-CH₂-, -O-CH₂-CH-(Y)-, -O-CH(Y)-CH₂-, -S-(CH₂)q-, -S-CH₂-CH(Y)-, -S-CH(Y)-CH₂- oder -S-CH₂-CH(Y)-CH₂. In diesen Gruppen bedeutet Y ein Wasserstoffatom oder eine lineare C₁-C₃-Alkylgruppe, wie oben im Zusammenhang mit der Ausführungsform (c) definiert, vorzugsweise eine Methylgruppe. m' und o' haben den Wert 2 oder 3. n" und q haben den Wert 2, 3, 4 oder 5.

Bei der Ausführungsform (h) bedeutet R die Gruppierung R"'-A"-B"-, wobei R"' für eine unsubstituierte oder mit Halogen, linearem C1-C3-Alkyl oder linearem C₁-C₃-Alkoxy substituierte Pyridinyl-, Imidazolyl-, Pyrimidinyl-, Thiophenyl-, Furanyl-, Benzimidazolyl- oder Chinolinylgruppe steht. Im Falle der Substitution kann R"' durch die Gruppierung 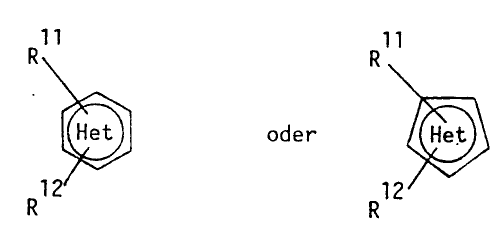 angegeben werden, worin R¹¹ und R¹² unabhängig voneinander jeweils ein Halogenatom, beispielsweise ein Fluor-, Brom-, Chlor- oder Jodatom, vorzugsweise ein Fluor- oder Chloratom, eine lineare C₁-C₃-Alkylgruppe, beispielsweise eine Methyl-, Ethyl- oder Propylgruppe, vorzugsweise eine Methylgruppe, oder eine lineare C₁-C₃-Alkoxygruppe, vorzugsweise eine Methoxygruppe, bedeuten. A" steht für eine Einfachbindung, für die Gruppierung -CR^{1'}R^{2'} oder ein Stickstoffatom, das mit einer Phenyl-, Naphthyl-, Benzyl-, Pyridinyl-, Benzimidazolyl-, Thiophenyl- oder Furanylgruppe, die gegebenenfalls mit Halogen, linearem Cᵢ-C₃-Alkyl oder linearem C₁-C₃-Alkoxy substituiert ist, oder einem Wasserstoffatom oder einer linearen Cᵢ-C₃-Alkylgruppe substituiert ist. Die Arylgruppe ist vorzugsweise eine Phenylgruppe. Die genannten Gruppen können beispielsweise in meta- oder para-Position, vorzugsweise in para-Position, mit einem Halogenatom, beispielsweise einem Fluor-, Chlor-, Brom- oder Jodatom, vorzugsweise einem Fluor- oder Chloratom, oder mit einer linearen C₁-C₃-Alkylgruppe, beispielsweise einer Methyl-, Ethyl- oder Propylgruppe, vorzugsweise einer Methylgruppe, oder mit einer linearen C₁-C₃-Alkoxygruppe, beispielsweise einer Methoxy-, Ethoxy-oder Propoxygruppe, vorzugsweise einer Methoxygruppe, substituiert sein.

R^{1'} bedeutet ein Wasserstoffatom oder eine Methylgruppe, während R für eine unsubstituierte oder mit Halogen, linearem C₁-C₃-Alkyl oder linearem C₁-C₃-Alkoxy substituierte Phenylgruppe oder eine unsubstituierte oder mit Halogen, linearem C1-C3-Alkyl oder linearem C₁-C₃-Alkoxy substituierte Benzimidazolyl-, Pyridinyl-, Thiophenyl- oder Furanylgruppe steht. Wenn die genannten Gruppen substituiert sind, dann sind sie vorzugsweise mit einem Halogenatom, beispielsweise einem Fluor-, Brom-, Chlor- oder Jodatom, vorzugsweise einem Fluor- oder Chloratom, einer linearen C₁-C₃-Alkylgruppe, beispielsweise einer Methyl-oder Ethylgruppe, oder einer linearen C₁-C₃-Alkoxygruppe, beispielsweise einer Methoxygruppe, substituiert. Wenn A" eine Einfachbindung ist, dann ist die Einfachbindung in Position 2, 3 oder 4 des heterocyclischen Restes angeordnet, d.h. sie verknüpft den Rest B, wie oben definiert, mit dem heterocyclischen Rest in Position 2, 3 oder 4 des heterocyclischen Restes. im Falle, daß der heterocyclische Ring ein Benzimidazolring ist, kommt als Verknüpfungsstelle nur die Position 2 des Benzimidazolrings in Betracht.

B" steht für eine der Gruppierungen -CH(Y)-S-(CH2)m, -CH₂-S-CH₂-CH(Y)-CH₂-, -CH₂-S-CH(Y)-CH₂-, -(CH₂)-CH(Y)-, -CH₂-S-CH₂-CH(Y)-, -(CH₂)_{n''}-,-O(CH₂)_{n''}-, -S-CH₂-CH(Y)-, S-(CH(Y)-CH₂-, -S-(CH₂)_{q}- oder -S-CH₂-CH(Y)-CH₂-, wobei Y ein Wasserstoffatom oder eine lineare C₁-C₃-Alkylgruppe bedeutet, m' den Wert 2 oder 3 hat und n" und q den Wert 2, 3, 4 oder 5 haben.

Bei einer bevorzugten Verbindungsgruppe der Ausführungsform (h) bedeutet R die Gruppierung R"'-A"-B"-, wobei R"' für die Gruppierung 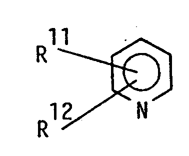 steht, wobei R¹¹ und R¹² unabhängig voneinander jeweils ein Halogenatom, beispielsweise ein Fluor-, Chlor-oder Bromatom, eine lineare C₁-C₃-Alkylgruppe, beispielsweise eine Methyl- oder Ethylgruppe, oder eine lineare C1-C3-Alkoxygruppe, beispielsweise eine Methoxy- oder Ethoxygruppe, bedeuten. A" steht vorzugsweise für die Gruppierung -CR^{1'}R^{2'}, wobei R^{1'} ein Wasserstoffatom oder eine Methylgruppe und R eine unsubstituierte oder ein- bis dreifach substituierte Phenylgruppe bedeuten. Im Falle der Substitution der Phenylgruppe kommen insbesondere 1 bis 3 Halogenatome, wie Fluor-, Chlor- oder Bromatome, vorzugsweise Fluor- oder Chloratome, eine bis drei lineare C₁-C₃-Alkylgruppen, vorzugsweise Methyl- oder Ethylgruppen, und eine bis drei lineare Alkoxygruppen, vorzugsweise Methoxy- oder Ethoxygruppen, in Betracht. Die Einfachsubstitution und insbesondere die Zweifachsubstitution werden bevorzugt. Im Falle der Einfachsubstitution wird die Substitution in 4-Position und im Falle der Zweifachsubstitution in 3- und 4-Position oder in 3- und 5-Position des Phenylrings bevorzugt. Als weitere Substituenten des Phenylrings kommen die Trifluormethyl- und die Hydroxygruppe in Betracht.

A" kann weiterhin ein Stickstoffatom, das mit einer Phenyl-, Pyridinyl-, Benzimidazolyl-, Thiophenyl-, Furanyl-, Benzyl- oder Methylgruppe oder mit einem Wasserstoffatom substituiert ist, sein. Die Substitution mit einer Phenylgruppe oder Benzylgruppe wird hierbei bevorzugt.

B" steht, wenn A" die Gruppierung -CR^{1'}R^{2'} ist, für die Gruppierung -(CH₂)ₙ-, -O(CH₂)₂- oder - SCH₂CH₂-, vorzugsweise -(CH₂)ₙ-, wobei n den Wert 2 oder 3 hat. B" steht, wenn A" ein mit einer Phenyl-, Pyridinyl-, Benzimidazolyl-, Thiophenyl-, Furanyl-, Benzyl- oder Methylgruppe oder einem Wasserstoffatom substituiertes Stickstoffatom ist, für die Gruppierung -(CH₂)ₙ-, wobei n den Wert 2 oder 3 hat.

Bei einer bevorzugten Verbindungsgruppe der Ausführungsform (h) bedeutet R die Gruppierung R"'-A"-B"-, wobei R"' für die Gruppierung 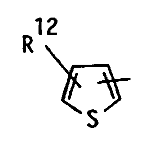 steht, worin R¹² ein Wasserstoffatom, ein Halogenatom, vorzugsweise ein Chloratom, eine lineare Cₗ-C₃-Alkylgruppe, vorzugsweise eine Methylgruppe, die Gruppierung (CH₃)₂-NCH₂- oder 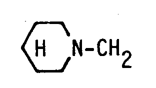 bedeutet. A" steht für die Gruppierung -CR^{1'}R^{2'}, wobei R^{1'} ein Wasserstoffatom oder eine Methylgruppe und R²' eine unsubstituierte oder eine ein- bis dreifach substituierte Phenylgruppe bedeuten. A" kann weiterhin für ein Stickstoffatom, das mit einer Phenyl-, Pyridinyl-, Benzimidazolyl-, Thiophenyl-, Furanyl-, Benzyl-oder Methylgruppe oder einem Wasserstoffatom substituiert ist, stehen. B" steht für eine der Gruppierungen -CH₂-S-CH₂-CH₂-, -CH₂-S-CH₂-CH(CH₃)-CH₂-,-CH₂-S-CH(CH₃)-CH₂- oder -CH₂-S-CH₂-CH(CH₃), vorzugsweise -CH₂-S-CH₂-CH₂-.

Bei einer weiteren bevorzugten Verbindungsgruppe der Ausführungsform(h) bedeutet R die Gruppierung R"'-A"-B"-, wobei R"' für die Gruppierung 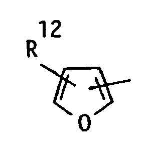 steht, worin R¹² ein Wasserstoffatom, ein Halogenatom, vorzugsweise ein Chloratom, eine lineare Cₗ-C₃-Alkylgruppe, vorzugsweise eine Methylgruppe, die Gruppierung (CH₃)₂-NCH₂- oder 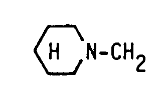 bedeutet. A" steht für eine Einfachbindung in Position 2. B" steht für eine der Gruppierungen -CH₂-S-CH₂-CH₂-, -CH₂-S-CH₂-CH(CH₃)-CH₂-, -CH₂-S-CH(CH₃)-CH₂- oder -CH₂-S-CH₂-CH(CH₃), vorzugsweise -CH₂-S-CH₂-CH₂-.

Bei einer weiteren bevorzugten Verbindungsgruppe der Ausführungsform (h) bedeutet R die Gruppierung R"'-A"-B"-, wobei R"' für die Gruppierung 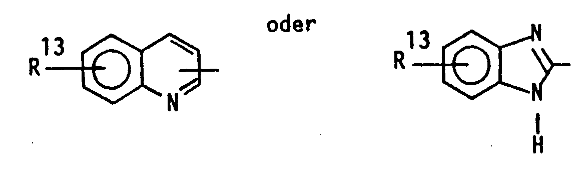 steht, worin R¹³ ein Wasserstoffatom, ein Halogenatom, vorzugsweise ein Chloratom, eine lineare Cₗ-C₃-Alkylgruppe, vorzugsweise eine Methylgruppe, die Gruppierung (CH₃)₂-NCH₂- oder 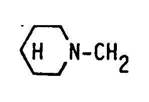 bedeutet. A" steht für eine Einfachbindung. B" steht für die Gruppierung -CH₂-S-CH₂-CH₂-, -CH₂-S-CH₂-CH(CH₃)-CH₂-, -CH₂-S-CH(CH₃)-CH₂- oder -CH₂-S-CH₂-CH(CH₃), vorzugsweise -CH₂-S-CH₂-CH₂-.

Die erfindungsgemäßen Verbindungen der allgemeinen Formel I können nach zwei verschiedenen Verfahrensvarianten hergestellt werden:
(1) durch Umsetzung einer Verbindung der allgemeinen Formel II 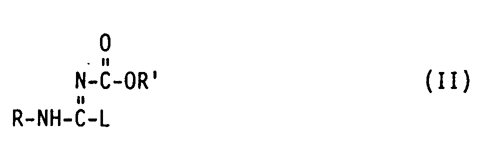 in der R und R' die oben angegebenen Bedeutungen haben und L für eine Austrittsgruppe, beispielsweise eine C₁-C₄-Alkylthio-, C₁-C₄-Alkoxy-, Arylthio- oder Aryloxygruppe, vorzugsweise eine Methylthio-oder Phenoxygruppe, steht, mit einer Verbindung der allgemeinen Formel III 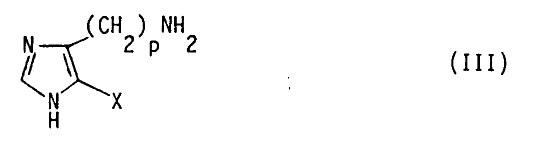 in der X und p wie oben definiert sind.
   Die Umsetzung erfolgt vorzugsweise in äquimolaren Mengen und in einem polaren Lösungsmittel, wie zum Beispiel Acetonitril, Dimethylsulfoxid, Dimethylformamid oder Pyridin, vorzugsweise in Acetonitril, bei Temperaturen von Raumtemperatur bis zur Rückflußtemperatur des verwendeten Lösungsmittels. Ist L eine Alkylthio- oder Arylthiogruppe, so ist es vorteilhaft, unter Säurekatalyse zu arbeiten, zum Beispiel unter Zusatz katalytischer Mengen p-Toluolsulfonsäure.
(2) oder durch Umsetzung einer Verbindung der allgemeinen Formel IV 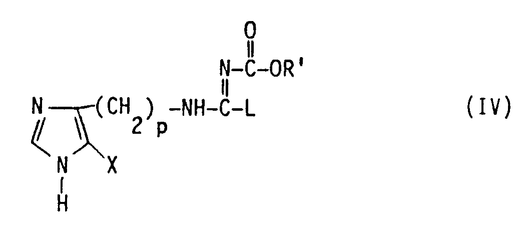 in der R', X und p wie oben definiert sind und L die gleiche Bedeutung wie in der Verfahrensvariante (1) hat, mit einer Verbindung der allgemeinen Formel V 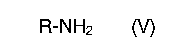 in der R die oben angegebene Bedeutung besitzt.
   Die verwendeten Mengen und Lösungsmittel sowie die Reaktionsbedingungen sind die gleichen wie oben im Zusammenhang mit der Verfahrensvariante (1) beschrieben.

Die in beiden Verfahrensvarianten (1) und (2) eingesetzten Ausgangsverbindungen der allgemeinen Formeln 11 und IV können in an sich bekannter Weise nach Literaturmethoden hergestellt werden. Verbindungen der allgemeinen Formeln II und IV, in denen L für eine Alkoxy- oder Aryloxygruppe steht, können zum Beispiel durch Umsetzung eines Amins der allgemeinen Formel III bzw. V, in denen X, p und R wie oben definiert sind, mit einer Verbindung der allgemeinen Formel VI, in der R' die oben angegebene Bedeutung besitzt und L für eine Alkoxy- bzw. Aryloxygruppe steht, hergestellt werden: 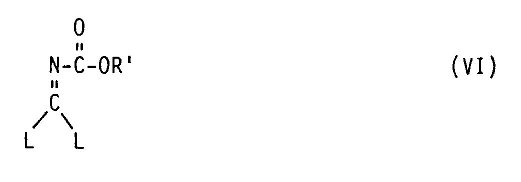 Verbindungen der allgemeinen Formeln II und IV, in denen L für eine Alkylthio- oder Arylthiogruppe steht, können zum Beispiel durch Acylierung einer Verbindung der allgemeinen Formeln VII bzw. VIII 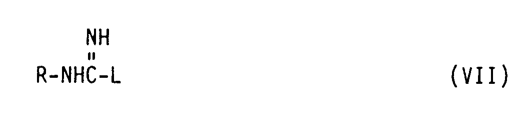 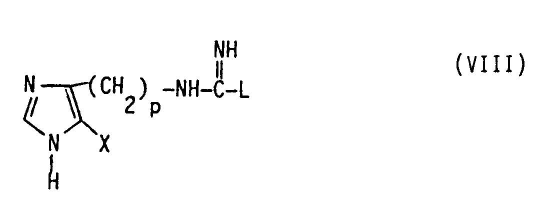 in denen R, X und p die oben angegebenen Bedeutungen besitzen und L für eine Alkylthio- oder Arylthiogruppe steht, mit einer Verbindung der allgemeinen Formel IX 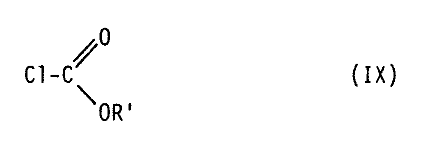 in der R' wie oben definiert ist, hergestellt werden. Man arbeitet dabei vorteilhafterweise in Gegenwart einer Base, wie zum Beispiel einem tertiären aliphatischen Amin, wie Triethylamin, oder einem heterocyclischen Amin, wie Pyridin.

Die nach den einzelnen Verfahrensvarianten erhaltenen Verbindungen werden in üblicher Weise isoliert und gereinigt, beispielsweise durch chromatische Arbeitsweisen, Umkristallisation etc.

Die bei den einzelnen Verfahrensvarianten erhaltenen Verbindungen können gegebenenfalls in ihr physiologisch annehmbares Salz umgewandelt werden.

Die Erfindung umfaßt neben den tautomeren und stereoisomeren Verbindungen und Hydraten der Substanzen der allgemeinen Formel I daher auch die physiologisch annehmbaren Salze dieser Verbindungen. Diese Salze können zum Beispiel mit Mineralsäuren, wie Chlor-, Brom-, Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc., gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutischen Trägern oder Verdünnungsmitteln hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/ oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Guanidincarbonsäureester der allgemeinen Formel I und ihre pharmakologisch annehmbaren Salze zeigen ausgeprägte kardiotone Wirkungen. Sie sind den bisher bekannten Verbindungen gleicher Wirkungsrichtung (vgl. DE 35 12 084, DE 35 28 214, DE 35 28 215 und EP O 199 845) überraschenderweise bei oraler Anwendung erheblich überlegen und eignen sich daher zur Behandlung und Vorbeugung von Erkrankungen des Herzens und des Kreislaufs. Sie zeigen eine ausgezeichnete Wirksamkeit in verschiedenartigen Tests, wie z.B. eine stark positiv inotrope Wirkung in vivo am Meerschweinchen.

### Hämodynamische Charakterisierung der positiv inotropen Wirkung am narkotisierten Meerschweinchen.

a) Methode
   Die Tiere werden mit Urethan (1.5 g/kg) narkotisiert. Zur volumen-kontrollierten Beatmung wird die Trachea kanüliert. Danach erfolgt die operative Freilegung beider Karotiden, über die rechte Karotis wird ein Tip-Katheter (3 F) eingeführt, der unter fortlaufender Druckregistrierung dann über die Aorta aszendens in den linken Ventrikel vorgeführt wird. Die erfolgreiche Passage der Aortenklappen wird hierbei durch die typische linksventrikuläre Druckkurve erkannt. Über die linke Karotis wird zur Thermodilution ein Thermistorfühler (3 F, F.Edwards) in den Aortenbogen vorgeschoben. Der Thermistorfühler hat gleichzeitig ein Lumen zur arteriellen Blutdruckregistrierung. Zur Applikation des Kälteinjektats (0.2 ml 0.9 % NaCI, 15 C) wird durch die rechte Vena jugularis ein Katheter vor den rechten Vorhof plaziert. Die Registrierung des EKGs erfolgt in der I. Ableitung. Das Duodenum wird mittels eines 1 cm langen Medianschnitts im oberen Abdominalbereich freigelegt; die Testsubstanzen werden über eine Nadel in das Duodenum injiziert. Alle Substanzen sind in Tylose (Injektionsvolumen 1 ml/kg) suspendiert, die Applikation erfolgt nach hämodynamischer Stabilisierung und unter ß-Blockade (Metoprolol 2 mg/kg i.m.).
   Alle Kreislaufparameter werden kontinuierlich auf einem Direktschreiber registriert. Die Kontraktilität (dp/dt) wird über die Volumendruckkurve berechnet.
b) Meßwerte

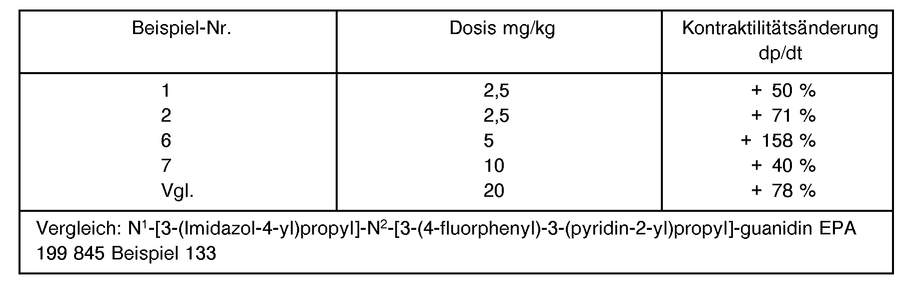

### Beispiel 1

### N¹-[3-(Imidazol-4-yl)propyl]-N²-[4-(pyridin-2-yl)butyl] -N³-methoxycarbonyl-guanidin

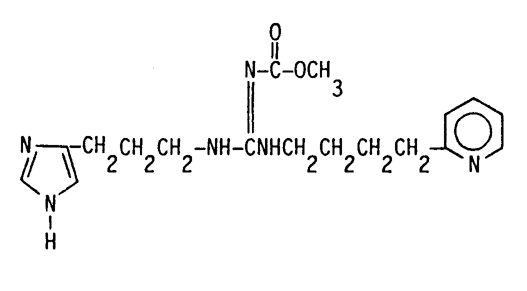

### a) N¹-[4-(Pyridin-2-yl)butyl]-N²-methoxycarbonyl-S-methylisothioharnstoff

Eine auf -15° C gekühlte Lösung von 3,0 g (8,5 mmol) N-[4-(Pyridin-2-yl)butyl] -S-ethyl-isothiuroniumjodid in 30 ml Dichlormethan wird vorsichtig mit 2,4 ml (17,1 mmol) Triethylamin versetzt. Unter weiterer Kühlung werden dann 0,66 ml (8,5 mmol) Chlorameisensäuremethylester in 20 ml Dichlormethan zugetropft, wobei die Innentemperatur unter -15 ° C gehalten wird. Nach einstündigem Nachrühren bei dieser Temperatur läßt man das Reaktionsgemisch sich auf Raumtemperatur erwärmen und rührt 20 Stunden weiter. Die Lösung wird dann zweimal mit 20 ml Wasser gewaschen. Die organische Phase wird mit Natriumsulfat getrocknet, filtriert und i. Vak. eingedampft. Das erhaltene Rohprodukt wird zur Reinigung an Kieselgel mit Dichlormethan/Aceton (80:20) chromatographiert. Die Hauptfraktion ergibt nach Eindampfen i. Vak. 2,17 g (90 %) eines grünlichen Öls.
C₁₃ H₁₉ N₃O₂ S (281,38)
Rf (CH₂C1₂/CH₃COCH₃ 90:10): 0,2

### b) N¹-[3-(Imidazol-4-yl)propyl]-N²-[4-(pyridin-2-yl)butyl]-N³-methoxycarbonyl-guanidin

1,00 g (3,5 mmol) N¹-[4-(Pyridin-2-yl)butyl]-N²-methoxycarbonyl-S-methyl-isothioharnstoff und 0,44 g (3,5 mmol) 3-(lmidazol-4-yl)propylamin werden in 30 ml Acetonitril 20 h unter Rückfluß gekocht. Das Lösungsmittel wird i. Vak. abgedampft und der Rückstand an Kieselgel mit Ethylacetat/Methanol (80:20) chromatographiert. Man erhält 0,43 g (34 %) der Titelverbindung als farbloses ÖI.
C₁₈ H₂₆ N₆ O₂ (358,45)
Rf (CH₃COOC₂Hs/CH₃OH 60:40): 0,4
¹H-NMR-Daten (CD₃OD, TMS als interner Standard)
   δ = 1,4 - 2,1 (m) 6 H,
      2,5 - 2,9 (m) 4 H,
      3,1 - 3,4 (m) 4 H,
      3,6 (s) 3 H,
      5,3 (breit) 3 H, austauschbar mit D₂0,
      6,8 (s) 1 H,
      7,1 - 7,8 (m) 3 H,
      7,6 (s) 1 H,
      8,4 (d) 1 H ppm.

### Beispiel 2

### N¹-[3-(Imidazol-4-yl)propyl]-N²-[4-(pyridin-2-yl)butyl]-N³-ethoxycarbonyl-guanidin

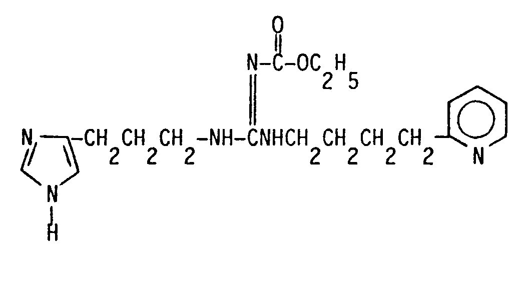

### a) N¹-[4-(Pyridin-2-yl)butyl]-N2-ethoxycarbonyl-S-methyl-isothioharnstoff

Hergestellt analog zu Beispiel 1 a) aus 7,0 g (20 mmol) N-[4-(Pyridin-2-yl) butyl]-S-methyl-isothiuroni- umjodid und 1,9 ml (20 mmol) Chlorameisensäureethylester. Chromatographische Reinigung des Rohprodukts an Kieselgel mit Dichlormethan/Aceton (9:1) als Laufmittel ergibt 4,94 g (84 %) eines gelblichen Öls.
C₁₄ H₂₁ N₃ O₂ S (295,41)
Rf (CH₂C1₂/CH₃COCH₃ 90:10): 0,3

### b) N¹-[3-(Imidazol-4-yl)propyl]-N²-[4-(pyridin-2-yl)butyl]-N³-ethoxycarbonyl-guanidin

1,75 g (5,9 mmol) N1-[4-(Pyridin-2-yl)butyl]-N2-ethoxycarbonyl-S-methyl-isothioharnstoff und 0,74 g (5,9 mmol) 3-(lmidazol-4-yl)propylamin werden unter Zusatz von 40 mg p-Toluolsulfonsäure in 30 ml Acetonitril 16 h gekocht. Das nach Abdampfen des Lösungsmittels i.Vak. erhaltene Öl wird mit Ethylacetat/Methanol (80:20) als Laufmittel an Kieselgel chromatographiert. Die Hauptfraktion ergibt beim Eindampfen i.Vak. ein farbloses Öl, das beim Verreiben mit Diethylether kristallisiert. Man erhält 0,81 g (37 %) farblose Kristalle vom Schmp. 92 - 94 C.
C₁₉H₂₈N₆O₂ (372,47)
Rf (CH₃COOC₂H₅/CH₃OH 60:40): 0,45
¹H-NMR-Daten (CD₃OD, TMS als interner Standard)
   δ = 1,25 (t) 3 H,
      1,5 - 2,0 (m) 6 H,
      2,65 (t) 2 H,
      2,8 (t) 2 H,
      3,1 - 3,4 (m) 4 H,
      4,05 (q) 2 H,
      4,9 (breit) 3 H, austauschbar mit D₂0,
      6,8 (s) 1 H,
      7,2 - 7,9 (m) 3 H,
      7,6 (s) 1 H,
      8,5 (d) 1 H ppm.

### Beispiel 3

### N¹[3-(Imidazol-4-yl)propyl]-N²-[3-[N-phenyl-N-(pyridin-2-yl)amino] propyl)-N³-methoxycarbonyl-guanidin

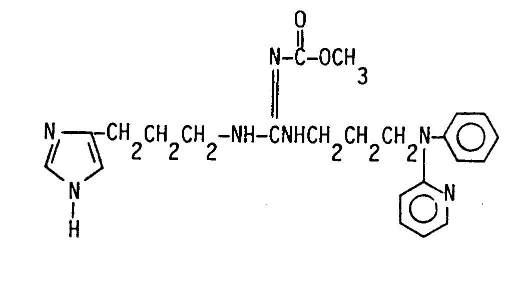

### a) N¹-[3-[N-Phenyl-N-(pyridin-2-yl)amino]propyl]-N²-methoxycarbonyl-S-methyl-isothioharnstoff

Aus 1,50 g (3,5 mmol) N-[3-[N-Phenyl-N-(pyridin-2-yl)amino]propyl]-S-methylisothiuroniumjodid und 0,27 ml (3,5 mmol) Chlorameisensäuremethylester erhält man analog zu Beispiel 1 a) 1,20 g (96 %) des Isothioharnstoffs als farbloses Öl.
C₁₈ H₂₂ N₄ O₂ S (358,46)
Rf (CH₂CI₂/CH₃0H 95:5): 0,6

### b) N¹-[3-(Imidazol-4-yl)propyl]-N²-[3-[N-phenyl-N-(pyridin-2-yl)amino] propyl]-N³-methoxycarbonyl-guanidin

1,00 g (2,8 mmol) N¹-[3-[N-Phenyl-N-(pyridin-2-yl)amino]propyl]-N²-methoxycarbonyl-S-methyl-isothioharnstoff und 0,35 g (2,8 mmol) 3-(lmidazol-4-yl)propylamin werden in 30 ml Acetonitril 16 h gekocht. Das Rohprodukt wird an Kieselgel mit Ethylacetat/Methanol (90:10) als Eluent chromatographisch gereinigt. Man erhält aus der Hauptfraktion nach Eindampfen i.Vak. 0,50 g (41 %) eines farblosen, amorphen Feststoffs.
C₂₃H₂₉H₇0₂ (435,53)
Rf (CH₃COOC₂H₅/CH₃OH 60:40): 0,55
¹H-NMR-Daten (CD₃OD, TMS als interner Standard)
   δ = 1,6 - 2,1 (m) 4 H,
      2,7 (t) 2 H,
      3,2 - 3,5 (m) 4 H,
      3,65 (s) 3 H,
      4,05 (t) 2 H,
      5,0 (breit) 3 H, austauschbar mit D₂0,
      6,3 - 6,8 (m) 2 H,
      6,85 (s) 1 H,
      7,2 - 7,6 (m) 6 H,
      7,6 (s) 1 H,
      8,2 (dd) 1 H ppm.

### Beispiel 4

### N¹-[3-(Imidazol-4-yl)propyl]-N²-[4-(pyridin-2-ylamino)butyl] -N³-ethoxycarbonyl-guanidin

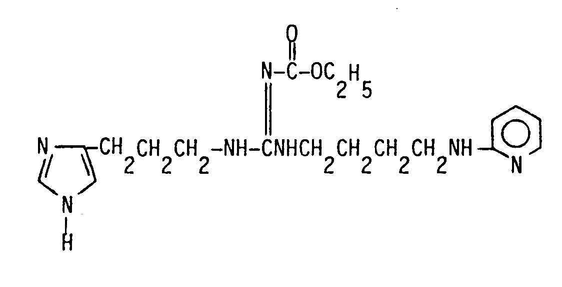

### a) N'-[4-(Pyridin-2-ylamino)butyl]-N²⁻ethoxycarbonyl-S-methyl -isothioharnstoff

Hergestellt analog zu Beispiel 1 a) aus 2,01 g (5,5 mmol) N-[4-(Pyridin-2-ylamino)butyl]-S-methyl- isothiuronium-jodid und 0,52 ml (5,5 mmol) Chlorameisensäureethylester. Man erhält 1,21 g (71 %) eines blaßgelben Öls, das im Kühlschrank erstarrt.
C₁₄H₂₂N₄O₂S (310,42)
Rf (CH₂Cl₂/CH₃OH 90:10): 0,6

### b) N¹-[3-(Imidazol-4-yl)propyl]-N²-[4-(pyridin-2-ylamino)butyl] -N³-ethoxycarbonyl-guanidin

1,21 g (3,9 mmol) N¹-[4-(Pyridin-2-ylamino)butyl]-N²-ethoxycarbonyl-S-methyl-isothioharnstoff, 0,48 g (3,9 mmol) 3-(lmidazol-4-yl)propylamin und 40 mg p-Toluolsulfonsäure werden in 30 ml Acetonitril 14 h gekocht. Die chromatographische Reinigung des Rohprodukts an Kieselgel mit Ethylacetat/Methanol (90:10→ 80:20) als Laufmittel liefert 0,81 g (53 %) eines beigen, amorphen Feststoffs.
C₁₉H₂₉N₇O₂ (387,49)
Rf (CH₃COOC₂H₅/CH₃OH 60:40): 0,5
¹H-NMR-Daten (CD₃OD, TMS als interner Standard)
   o = 1,25 (t) 3 H,
      1,6 - 2,1 (m) 6 H,
      2,7 (t) 2 H,
      3,1 - 3,5 (m) 6 H,
      4,15 (q) 2 H,
      5,0 (breit) 4 H, austauschbar mit D₂0
      6,5 - 6,7 (m) 2 H,
      6,95 (s) 1 H,
      7,4 - 7,6 (m) 1 H,
      7,8 (s) 1 H,
      8,0 (dd) 1 H ppm.

### Beispiel 5

### N¹-[3-(Imidazol-4-yl)propyl]-N²-[3-(4-fluorphenyl)-3-(pyridin-2-yl)propyl]-N³-methoxycarbonyl-guanidin

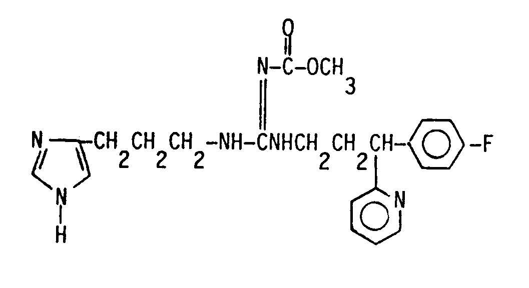

### a) N¹-[3-(4-Fluorphenyl)-3-(pyridin-2-yl)-propyl]-N²-methoxycarbonyl-S-methyl-isothioharnstoff

Analog zu Beispiel 1 a) erhält man aus 8,6 g (20 mmol) N-[3-(4-Fluorphenyl)-3-(pyridin-2-yl)propyl]-S-methyl-isothioronium-jodid und 1,55 ml (20 mmol) Chlorameisensäuremethylester 6,5 g (90 %) eines farblosen Öls.
C₁₈H₂₀FN₃O₂S (361,44)
Rf (CH₂C1₂/CH₃COCH₃ 90:10): 0,5

### b) N¹-[3-(Imidazol-4-yl)propyl]-N²-[3-(4-fluorphenyl)-3-(pyridin-2-yl) propyl]-N³-methoxycarbonyl-guanidin

In 30 ml Acetonitril werden 1,34 g (3,7 mmol) N¹-[3-(4-Fluorphenyl)-3-(pyridin-2-yl)-propyl]-N²- methoxycarbonyl-S-methyl-isoharnstoff und 0,46 g (3,7 mmol) 3-(lmidazol-4-yl)propylamin unter Zusatz von 30 mg p-Toluolsulfonsäure 14 h gekocht. Das Lösungsmittel wird i.Vak. abgedampft und der Rückstand an Kieselgel mit Ethylacetat/Methanol (90:10) chromatographiert. Die Hauptfraktion ergibt nach Eindampfen i.Vak. 0,91 g (56 %) eines farblosen, amorphen Feststoffs.
C₂₃H₂₇FN₆O₂ (438,51)
Rf (CH₃COOC₂H₅/CH₃OH 60:40): 0,45
¹H-NMR-Daten (CD₃OD, TMS als interner Standard)
   δ = 1,7 - 2,1 (quin) 2 H,
      2,2 - 2,8 (m) 4 H,
      3,1 - 3,5 (m) 4 H,
      3,7 (s) 3 H,
      4,25 (t) 1 H,
      5,3 (breit) 3 H, austauschbar mit D₂0,
      6,9 - 7,9 (m) 9 H
      8,6 (dd) 1 H ppm.

### Beispiel 6

### N¹-[3-(Imidazol-4-yl)propyl]-N²-[3-(4-fluorphenyl)-3-(pyridin-2-yl) propyl]-N³-ethoxycarbonyl-guanidin

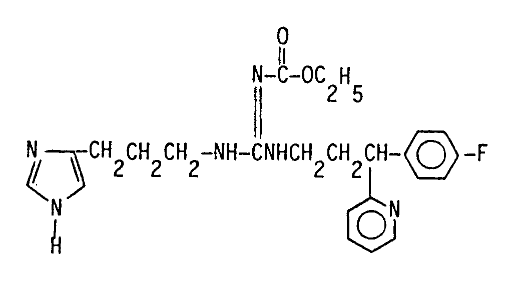

### a) N¹-[3-(4-Fluorphenyl)-3-(pyridin-2-yl)-propyl]-N²-ethoxycarbonyl -O-phenyl-isoharnstoff

1,40 g (4,9 mmol) N-(Ethoxycarbonyl)-imidokohlensäurediphenylester werden in 20 ml Tetrahydrofuran auf +5° C abgekühlt. Unter Kühlung werden 1,13 g (4,9 mmol) 3-(4-Fluorphenyl)-3-(pyridin-2-yl)-propylamin in 20 ml Tetrahydrofuran während 15 Minuten zugetropft. Nach vierstündigem Nachrühren bei Raumtemperatur wird die Lösung i.Vak. eingedampft. Das erhaltene farblose Öl wird ohne Reinigung weiter umgesetzt.
C₂₄H₂₄FN₃0₃ (421,47)
Rf (CH₂CI₂/CH₃0H 95:5): 0,7

### b) N¹-[3-(Imidazol-4-yl)propyl]-N²-[3-(4-fluorphenyl)-3-(pyridin-2-yl) -propyl)-N³-ethoxycarbonyl-guanidin

Zu einer Lösung von 2,06 g (4,9 mmol) rohem N¹-[3-(4-Fluorphenyl)-3-(pyridin-2-yl)-propyl]-N²- ethoxycarbonyl-O-phenyl-isoharnstoff in 40 ml Acetonitril werden 0,61 g (4,9 mmol) 3-(lmidazol-4-yl)-propylamin gegeben und das Reaktionsgemisch 7 h unter Rückfluß gekocht. Das Lösungsmittel wird i.Vak. abgedampft und der ölige Rückstand an Kieselgel mit Ethylacetat/Methanol (80:20) chromatographiert. Man erhält nach Eindampfen der Hauptfraktion i.Vak. 0,99 g (45 %) eines farblosen Öls.
C₂₄H₂₉FN₆O₂ (452,53)
Rf(CH₃COOC₂H₅/CH₃OH 60:40): 0,6
¹H-NMR-Daten (CD₃OD, TMS als interner Standard)
   δ = 1,2 (t) 3 H,
      1,7 - 2,1 (quin) 2 H,
      2,2 - 2,8 (m) 4 H,
      3,1 - 3,5 (m) 4 H,
      4,1 (q) 2 H,
      4,2 (t) 1 H,
      4,9 (breit) 3 H, austauschbar mit D₂0,
      6,8 - 7,9 (m) 9 H,
      8,55 (dd) 1 H ppm.

### Beispiel 7

### N¹-[3-(Imidazol-4-yl)propyl]-N²-[3-(4-fluorphenyl)-3-(pyridin-2-yl) propyl]-N³-butoxycarbonyl-guanidin

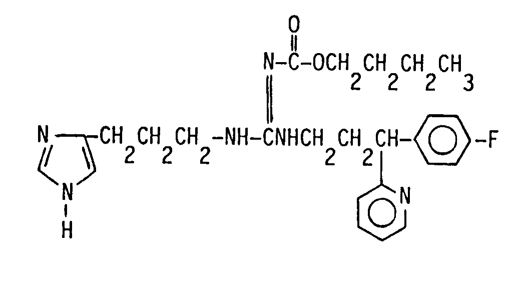

### a) N¹-[3-(4-Fluorphenyl)-3-(pyridin-2-yl)propyl]-N²-butoxycarbonyl-O-phenyl-isoharnstoff

Hergestellt analog zu Beispiel 6 a) aus 3,13 g (10 mmol) N-(Butoxycarbonyl) -imidokohlensäurediphenylester und 2,30 g (10 mmol) 3-(4-Fluorphenyl)-3-(pyridin-2-yl)-propylamin in Tetrahydrofuran. Das Produkt wird nicht isoliert, sondern in Lösung weiter umgesetzt.
C₂₆H₂₈FN₃0₃ (449,52)
Rf(CH₂Cl₂/CH₃OH 95:5): 0,85

### b) N¹-[3-(Imidazol-4-yl)propyl]-N²-[3-(4-fluorphenyl)-3-(pyridin-2-yl) propyl]-N³-butoxycarbonyl-guanidin

1,25 g (10 mmol) 3-(lmidazol-4-yl)propylamin werden zu der Lösung von 4,49 g (10 mmol) rohem N¹⁻[3-(4-Fluorphenyl)-3-(pyridin-2-yl)-propyl] -N²-butoxycarbonyl-O-phenyl-isoharnstoff in 60 ml Tetrahydrofuran gegeben und die Mischung 8 h unter Rückfluß gekocht. Das nach Abdampfen des Lösungsmittels i.Vak. erhaltene Öl wird an Kieselgel mit Ethylacetat/Methanol (80:20) als Eluent chromatographiert. Man erhält 1,40 g (29 %) eines farblosen, amorphen Feststoffs aus der Hauptfraktion.
C₂₆H₃₃FN₆O₂ (480,59)
Rf(CH₃COOC₂H₅/CH₃OH 60:40): 0,65
¹H-NMR-Daten (CDCl₃, TMS als interner Standard)
   o = 0,95 (t) 3 H,
      1,1 - 2,9 (m) 10 H,
      3,2 - 3,8 (m) 4 H,
      4,0 - 4,3 (2 t) 3 H,
      6,9 - 7,8 (m) 9 H,
      8,7 (d) 1 H,
      9,3 (breit) 1 H, austauschbar mit D₂0,
      10,6 (breit) 1 H, austauschbar mit D₂0 ppm.

### Beispiel 8

### N¹-[3-(Imidazol-4-yl)propyl]-N²-[3-(4-fluorphenyl)-3-(pyridin-2-yl) propyl]-N³-phenoycarbonyl-guanidin

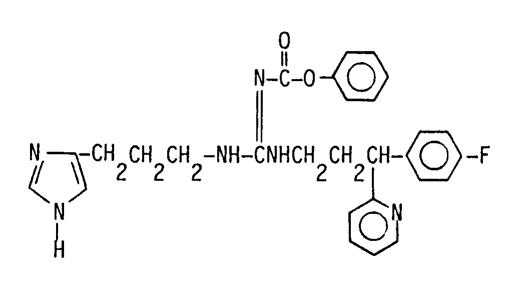

### a) N¹-[3-(4-Fluorphenyl)-3-(pyridin-2-yl)-propyl]-N²-phenoxycarbonyl-O-phenyl-isoharnstoff

Analog zu Beispiel 6 a) werden 3,33 g (10 mmol) N-(Phenoxycarbonyl)-imidokohlensäure-diphenylester und 2,30 g (10 mmol) 3-(4-Fluorphenyl)-3-(pyridin -2-yl)-propylamin in 60 ml Acetonitril umgesetzt. Nach fünfstündigem Rühren bei Raumtemperatur wird die Verbindung ohne Isolierung in Lösung weiter umgesetzt.
C₂₈H₂₄FN₃0₃ (469,51)

### b) N¹-[3-(Imidazol-4-yl)propyl]-N²-[3-(4-fluorphenyl)-3-(pyridin-2-yl) propyl]-N³-phenoxycarbonyl-guanidin

Die nach Beispiel 8 a) erhaltene Lösung von 42,69 g (10 mmol) N¹-[3-(4-Fluorphenyl)-3-(pyridin-2-yl)-propyl]-N²⁻phenoxycarbonyl-O-phenyl-isoharnstoff in Acetonitril wird mit 1,25 g (10 mmol) 3-(lmidazol -4-yl)-propylamin versetzt und 6 h gekocht. Das Rohprodukt wird an Kieselgel mit Ethylacetat/Methanol (80:20) chromatographiert. Aus der Hauptfraktion werden 3,65 g (73 %) eines farblosen, amorphen Feststoffs erhalten.
C₂₈H₂9FN₆O₂ (500,57)
Rf(CH₃COOC₂H₅/CH₃OH 80:20): 0,6
¹H-NMR-Daten (d₆-DMSO, TMS als interner Standard)
   o = 1,7 (quin) 2 H,
      2,1 - 2,8 (m) 4 H,
      2,8 - 3,5 (m) 4 H,
      4,3 (t) 1 H,
      6,7 - 7,9 (m) 15 H, 1 H austauschbar mit D₂0,
      8,7 (dd) 1 H,
      9,5 (t) 1 H, austauschbar mit D₂0 ppm.

### Beispiel 9

### N¹-[3-(Imidazol-4-yl)propyl]-N²-[3-(4-fluorphenyl)-3-(pyridin-2-yl) propyl]-N³-benzyloxycarbonyl-guanidin

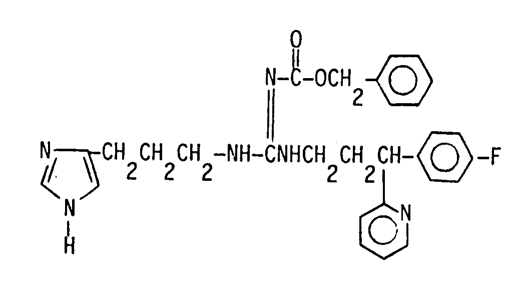

### a) N¹-[3-(4-Fluorphenyl)-3-(pyridin-2-yl)-propyl]-N²-benzyloxycarbonyl-S-methyl-isothioharnstoff

Erhalten analog zu Beispiel 1 a) als hellgelbes Öl aus 2,16 g (5 mmol) N-[3-(4-Fluorphenyl)-3-(pyridin-2-yl)-propyl]-S-methyl-isothiuroniumjodid und 0,71 ml (5 mmol) Chlorameisensäurebenzylester nach Chromatographie an Kieselgel mit Dichlormethan/Aceton (95:5) als Laufmittel.
Ausbeute: 1,55 g (71 %)
C₂₄H₂₄FN₃O₂S (437,54)
Rf(CH₂CI₂/CH₃COCH₃ 95:5): 0,7

### b) N¹-[3-(Imidazol-4-yl)propyl]-N²-[3-(4-fluorphenyl)-3-(pyridin-2-yl) propyl]-N³-benzyloxycarbonyl-guanidin

1,23 g (2,8 mmol) N¹-[3-(4-Fluorphenyl)-3-(pyridin-2-yl)-propyl]-N²-benzyloxycarbonyl-S-methyl-isothioharnstoff und 0,35 g (2,8 mmol) 3-(lmidazol-4-yl)propylamin werden in 25 ml Acetonitril 24 h gekocht. Chromatographische Reinigung des nach Abdampfen des Lösungsmittels i.Vak. erhaltenen Rohprodukts an Kieselgel mit Ethylacetat/Methanol (80:20) als Eluenten ergibt 0,58 g (40 %) eines farblosen, amorphen Feststoffs.
C₂₉ H₃₁ FN₆ O₂ (514,60)
Rf(CH₃COOC₂H₅/CH₃OH 80:20): 0,5
¹H-NMR-Daten (CDCl₃, TMS als interner Standard)
   o = 1,7 - 2,9 (m) 6 H,
      3,1 - 3,7 (m) 4 H,
      4,1 - 4,3 (m) 1 H,
      5,2 (s) 2 H,
      6,85 (s) 1 H,
      6,9 - 7,8 (m) 14 H, 1 H austauschbar mit D₂0,
      8,7 (dd) 1 H,
      9,3 (breit) 1 H, austauschbar mit D₂0 ppm.

### Beispiel 10

### N¹-[3-(Imidazol-4-yl)propyl]-N²-[2-[N-(4-chlorbenzyl)-N-(pyridin-2-yl) amino]ethyl]-N³-ethoxycarbonyl-guanidin

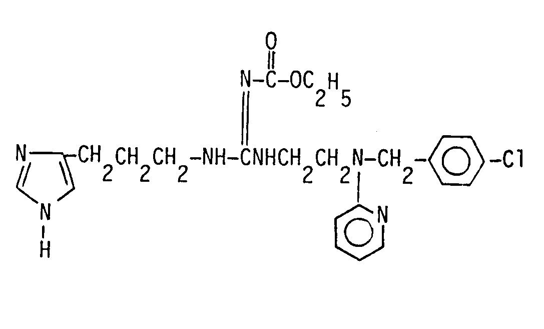

### a) N¹-[2-[N-(4-Chlorbenzyl)-N-(pyridin-2-yl)amino]ethyl]N²-ethoxycarbonyl-S-methyl-isothioharnstoff

Hergestellt analog zu Beispiel 1 a) aus 1,50 g (3,2 mmol) N-[2-[N-(4-Chlorbenzyl)-N-(pyridin-2-yl)amino]-ethyl]-S-methyl-isothiuronium-jodid und 0,31 ml (3,2 mmol) Chlorameisensäureethylester. 1,20 g (91 %) farbloses Öl.
C₁₉H₂₃ClN₄O₂S (406,93)
Rf(CH₂C1₂/CH₃COCH₃ 99:1): 0,4

### b) N¹-[3-(Imidazol-4-yl)propyl]-N²-[2-[2-[N-(4-chlorbenzyl)-N-(pyridin-2-yl)-amino]ethyl]-N³-ethoxycarbonyl- guanidin

1,02 g (2,5 mmol) N¹-[2-[N-(4-Chlorbenzyl)-N-(pyridin-2-yl)-amino]ethyl] -N²-ethoxycarbonyl-S-methyl-isothioharnstoff und 0,31 g (2,5 mmol) 3-(lmidazol-4-yl)propylamin werden in 20 ml Acetonitril 24 h gekocht. Das Rohprodukt wird durch Säulenchromatographie an Kieselgel mit Ethylacetat/Methanol (80:20) als Eluent gereinigt. Aus der Hauptfraktion erhält man nach Eindampfen i. Vak. ein farbloses Öl, das mit 5 ml Ethylacetat/Methanol (80:20) kristallisiert wird. Es werden 0,42 g (35 %) farblose Kristalle vom Schmp. 94 - 96° C erhalten.
C₂₄H₃₀CIN₇O₂ (484,00)
Rf(CH₃COOC₂H₅/CH₃0H/NH₃ konz. 80:15:5): 0,55
¹H-NMR-Daten (CD₃OD, TMS als interner Standard)
   δ = 1,25 (t) 3 H,
      1,9 (quin) 2 H,
      2,7 (t) 2 H,
      3,2 - 3,9 (m) 6 H,
      4,1 (q) 2 H,
      4,8 (s) 2 H,
      4,9 (breit) 3 H, austauschbar mit D₂0,
      6,6 - 6,9 (m) 3 H,
      7,2 - 7,7 (m) 6 H,
      8,2 (dd) 1 H ppm.

### Beispiel 11

### N¹-[3-(Imidazol-4-yl)propyl-N²-[2-[[(5-methyl-imidazol-4-yl)methyl] thio]ethyl]-N³-ethoxycarbonyl-guanidin

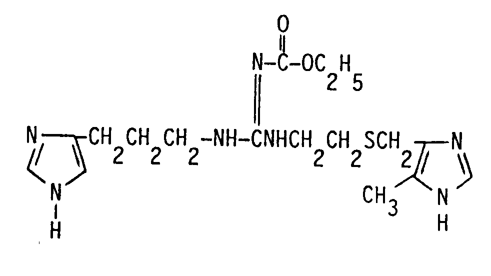

### a) N¹-[2-[[(1-Ethoxycarbonyl-5-methyl-imidazol-4-yl)methyl]thio]ethyl] -N²-ethoxycarbonyl-S-methyl-isothioharnstoff

7,8 g (20,9 mmol) N-[2-[[(5-Methyl-imidazol-4-yl)methyl]thio]ethyl]-S-methyl-isothiuronium-jodid werden analog zu Beispiel 1 a) mit 4,0 ml (41,8 mmol) Chlorameisensäureethylester und 8,7 ml (62,7 mmol) Triethylamin umgesetzt. Das nach Extraktion und Eindampfen der organischen Phase erhaltene Rohprodukt wird an Kieselgel mit Dichlormethan/Aceton (90:10) chromatographiert und ergibt nach Eindampfen der Hauptfraktion ein farbloses Öl, das spontan kristallisiert. Man erhält 4,5 g (55 %) farblose Kristalle vom Schmp. 75 - 76 C.
C₁₅H₂₄N₄O₄S₂ (388,51)
Rf(CH₂C1₂/CH₃COCH₃ 90:10): 0,4

### b) N¹-[3-(Imidazol-4-yl)propyl]-N²-[2-[[(5-methyl-imidazol-4-yl)methyl] thio]ethyl]-N³-ethoxycarbonyl-guanidin

4,0 g (10,3 mmol) N¹-[2-[[(1-Ethoxycarbonyl-5-methyl-imidazol-4-yl)methyl] thio]ethyl]-N²⁻ ethoxycarbonyl-S-methyl-isothioharnstoff und 1,3 g (10,3 mmol) 3-(lmidazol-4-yl)propylamin werden mit 0,1 g p-Toluolsulfonsäure in 100 ml Acetonitril 16 h gekocht. Das Lösungsmittel wird i.Vak. abgedampft und der Rückstand an Kieselgel mit Ethylacetat/Methanol (60:40) als Laufmittel chromatographiert. Aus der polaren Hauptfraktion erhält man nach Eindampfen i.Vak. 1,1 g (27 %) eines farblosen, amorphen Feststoffs.
C₁₇H₂₇N₇O₂S (393,51)
Rf(CH₃COOC₂H₅/CH₃OH 60:40): 0,5
¹H-NMR-Daten (CD₃OD, TMS als interner Standard)
   o = 1,2 (t) 3 H,
      1,9 (quin) 2 H,
      2,2 (s) 3 H,
      2,7 (t) 4 H,
      3,2 - 3,6 (m) 4 H,
      3,8 (s) 2 H,
      4,1 (q) 2 H,
      5,2 (breit) 4 H, austauschbar mit D₂0,
      6,9 (s) 1 H,
      7,6 (s) 1 H,
      7,7 (s) 1 H ppm.

### Beispiel 12

### N¹-[3-(3,4-Dichlorphenyl)-3-(pyridin-2-yl)propyl]-N²-ethoxycarbonyl-N³-[3-(1H-imidazol-4-yl)propyl]guanidin

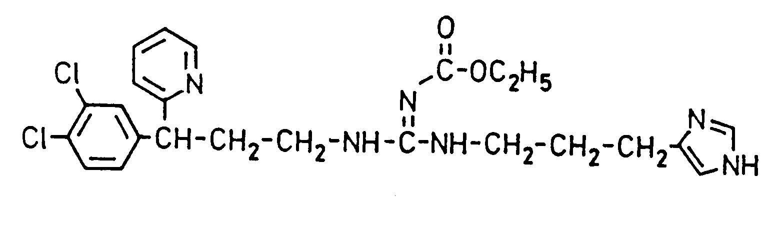

1,41 g (5 mmol) 3-(3,4-Dichlorphenyl)-3-(pyridin-2-yl)propylamin und 1,43 g (5 mmol) N-Ethoxycarbonyldiphenylimidocarbonat werden in 20 ml Acetonitril 20 min bei Raumtemperatur gerührt. Nach Zusatz von 0,65 g (5,2 mmol) 3-(1H-Imidazol-4-yl)propylamin wird 10 h unter Rückfluß erhitzt, anschließend der Reaktionsansatz i. Vak. eingedampft, der Rückstand in 5prozentiger Salzsäure aufgenommen und mit Ether extrahiert. Nach Alkalisieren mit Ammoniak wird mit Methylenchlorid extrahiert, die organische Phase mit Wasser gewaschen, über Natriumsulfat getrocknet und i.Vak. eingedampft. Das Reaktionsprodukt wird durch Chromatographie an Kieselgel 60 PF₂₅₄ gipshaltig isoliert (Fließmittel: Chloroform/Methanol, 99,5/0,5, Ammoniakatmosphäre). Nach Eindampfen der Eluate wird der zunächst erhaltene amorphe Feststoff (1,4 g, 56 %) aus Ethylacetat/Ether kristallisiert. Schmp. 73-75 ° C.

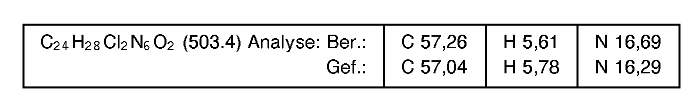

MS (FAB-Methode): m/z (rel. Int [%]) = 503 ([M + H] , 9), 264 (73), 109 (100), 95 (31).
¹H-NMR-Daten: (d₆-DMSO, TMS als interner Standard)
   δ = 1,13 (t) 3H,
      1,75 (m) 2H,
      2,25 (m) 1 H,
      2,43 (m) 1 H,
      2,5 (m, teilweise verdeckt) 2H,
      3,10 (m) 2H,
      3,16 (m) 2H,
      3,88 (q) 2H,
      4,20 (t) 1 H,
      6,6 - 7,2 (breit) 2H, 1 H austauschbar mit D₂0,
      7,23 (m) 1 H,
      7,35 - 7,8 (m) 6H,
      8,54 (d) 1 H,
      8,9 (breit) 1 H, austauschbar mit D₂0,
      11.8 (breit) 1 H, austauschbar mit D₂0, ppm.

### Beispiel 13

N¹-[3-(3,4-Difluorphenyl)-3-(pyridin-2-yl)propyl]-N²-ethoxycarbonyl-N³-[3-(1H-imidazol-4-yl)propyl]-guanidin

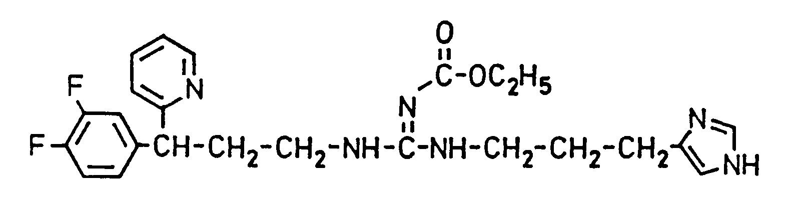

Die Herstellung erfolgt analog Beispiel 12, ausgehend von 1,24 g (5 mmol) 3-(3,4-Difluorphenyl)-3-(pyridin-2-yl)propylamin. Ausbeute: 1,2 g (51 %) farblose Kristalle vom Schmp. 119-120 °C (Ethylacetat/Ether).

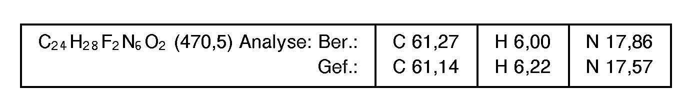

MS (FAB-Methode): m/z (rel. Int [%]) = 471 ([M + H] , 8), 425 (4), 323 (100), 204 (19), 109 (69), 95 (15), 81 (25).
¹H-NMR-Daten: (d₆-DMSO, TMS als interner Standard)
   δ = 1,13 (t) 3H,
      1,75 (m) 2H,
      2,24 (m) 1 H,
      2,44 (m) 1 H,
      2,5 (m, teilweise verdeckt) 2H,
      3,08 (m) 2H,
      3,17 (m) 2H,
      3,88 (q) 2H,
      4,18 (t) 1 H,
      6,6 - 7,2 (breit) 2H, 1 H austauschbar mit D₂0,
      7,2 - 7,6 (m) 6H,
      7,71 (m) 1 H,
      8,54 (d) 1 H,
      8,9 (breit) 1 H, austauschbar mit D₂0,
      11.8 (breit) 1 H, austauschbar mit D₂0, ppm.

### Beispiel 14

### N¹-[3-(3,5-Difluorphenyl)-3-(pyridin-2-yl)propyl]-N²-ethoxycarbonyl-N³-[3-(1H-imidazol-4-yl)propyl]-guanidin

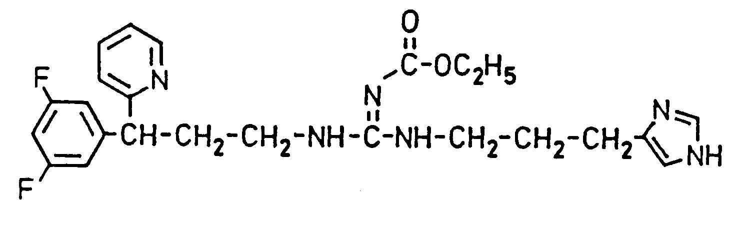

Die Herstellung erfolgt analog Beispiel 12 ausgehend von 1,24 g (5 mmol) 3-(3,5-Difluorphenyl)-3-(pyridin-2-yl)propylamin. Ausbeute: 1,3 g (55 %) farblose Kristalle vom Schmp. 104-105 °C (Ethylacetat/Ether).

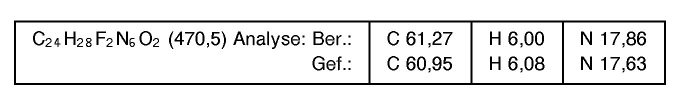

MS (FAB-Methode): m/z (rel. Int. [%]) = 471 ([M+H]⁺, 9), 232 (100), 204 (17), 172 (11), 109 (66), 95 (15).
¹H-NMR-Daten: (d₆-DMSO, TMS als interner Standard)
   δ = 1,13 (t) 3H,
      1,76 (m) 2H,
      2,2 - 2,6 (m) 4H,
      3,10 (m) 2H,
      3,16 (m) 2H,
      3,91 (q) 2H,
      4,21 (t) 1 H,
      6,77 (breit) 1 H,
      6,9 (breit) 1 H, austauschbar mit D₂0,
      7,0 - 7,45 (m) 5H,
      7,50 (s) 1 H,
      7,71 (m) 1 H,
      8,55 (d) 1 H,
      8,9 (breit) 1 H, austauschbar mit D₂0,
      11.85 (breit) 1 H, austauschbar mit D₂0, ppm.

### Beispiel 15

### N¹-[3-(4-Fluorphenyl)-3-(pyridin-3-yl)propyl]-N²-ethoxycarbonyl-N³-[3-(1H-imidazol-4-yl)propyl]-guanidin

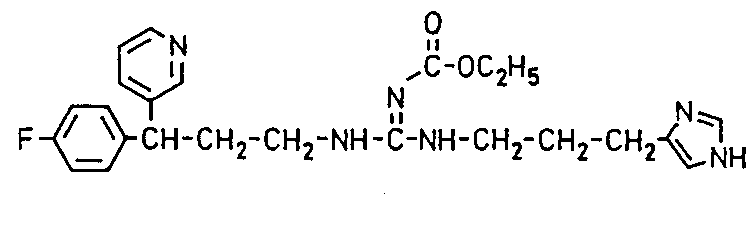

Die Herstellung erfolgt analog Beispiel 12 ausgehend von 1,15 g (5 mmol) 3-(4-Fluorphenyl)-3-(pyridin-3-yl)propylamin. Ausbeute: 1,15 g (47 %) farblose Kristalle vom Schmp. 104-105 ° C (Ethylacetat/Ether).

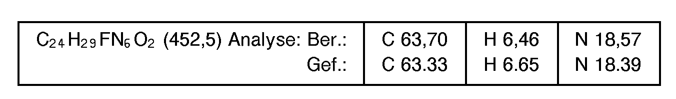

¹H-NMR-Daten: (d₆-DMSO, TMS als interner Standard)
   δ = 1,13 (t) 3H,
      1,75 (m) 2H,
      2,29 (m) 2H,
      2,5 (m, teilweise verdeckt) 2H,
      3,09 (m) 2H,
      3,16 (m) 2H,
      3,88 (q) 2H,
      4,08 (t) 1 H,
      6,77 (breit) 1 H,
      7,0 (sehr breit) 1 H, austauschbar mit D₂0,
      7,12 (dd) 2H,
      7,30 (dd) 1 H,
      7,40 (dd) 2H,
      7,50 (s) 1 H,
      7,77 (d) 1 H,
      8,40 (d) 1 H,
      8,56 (s) 1 H,
      8,95 (breit) 1 H, austauschbar mit D₂0,
      18,4 (breit) 1 H, austauschbar mit D₂0, ppm.

### Beispiel 16

### N¹-[(1,1-Dimethylethyl)oxycarbonyl]-N²-[3-(4-fluorphenyl)-3-(pyridin-2-yl)propyl]-N³-[3-(1H-imidazol-4-yl)-propyl]guanidin

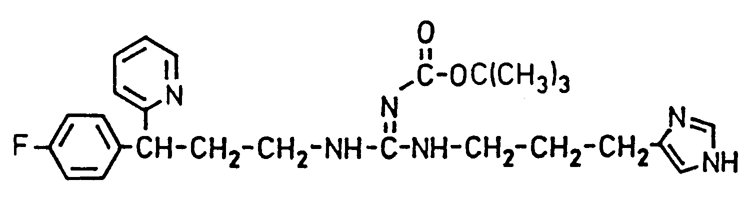

Die Herstellung erfolgt analog Beispiel 12 aus 1,15 g (5 mmol) 3-(4-Fluorphenyl)-3-(pyridin-2-yl)-propylamin, 1,57 g (5 mmol) N-tert-Butoxycarbonyldiphenylimidocarbonat und 0,65 g (5,2 mmol) 3-(1H-Imidazol-4-yl)propylamin. Ausbeute: 1,1 g (46 %) farblose Kristalle vom Schmp. 116-117 °C (Ethylacetat/Ether).

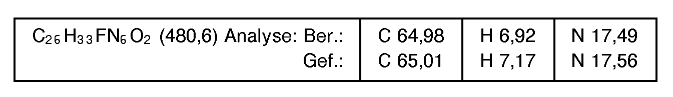

MS (FAB-Methode): m/z (rel. Int. [%]) = 481 ([M+H]⁺, 6), 381 (39), 214 (100), 186 (29), 151 (12), 109 (90,) 100 (45), 95 (22), 82 (31), 81 (39), 57 (88).
¹H-NMR-Daten: (d₆-DMSO, TMS als interner Standard)
   δ = 1,37 (s) 9H,
      1,74 (m) 2H,
      2,21 (m) 1 H,
      2,35 - 2,6 (m) 3H,
      3,06 (m) 2H,
      3,17 (m) 2H,
      4,17 (t) 1 H,
      6,76 (s) 1 H,
      6,9 (breit) 1 H, austauschbar mit D₂0,
      7,10 (dd) 2H,
      7,20 (dd) 1 H,
      7,32 (d) 1 H,
      7,40 (dd) 2H
      7,49 (s) 1 H,
      7,69 (dd) 1 H,
      8,53 (d) 1 H,
      8,9 (breit) 1 H, austauschbar mit D₂0,
      11,9 (breit) 1 H, austauschbar mit D₂0, ppm.

### Beispiel 17

### N¹-Ethoxycarbonyl-N²-[3-(1H-imidazol-4-yl)propyl]-N³-[2-phenyl-2-(2-pyridyl)ethyl]guanidin

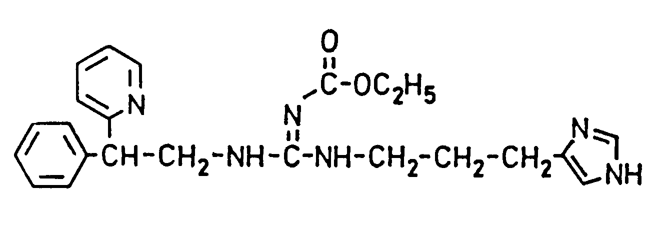

Die Herstellung erfolgt analog Beispiel 12 ausgehend von 0,99 g (5 mmol) 2-Phenyl-2-(2-pyridyl)-ethylamin. Ausbeute: 0,6 g (27 %) farblose Kristalle vom Schmp. 145 ° C (Ethylacetat/Ethanol)
C₂₃H₂₈N₆O₂ (420,5)
MS (FAB-Methode): m/z (rel. Int. [%]) = 421 ([M + H] , 13), 394 (6), 375 (8), 182 (100), 180 (38), 169 (25), 168 (21), 162 (29), 109 (65), 95 (10), 81 (25).
¹H-NMR-Daten: (d₆-DMSO, TMS als interner Standard)
   δ = 1,11 (m) 3H,
      1,67 (m) 2H,
      2,45 (m) 2H,
      3,0 - 4,05 (breit) 6H,
      4,45 (breit) 1 H,
      6,73 (s) 1 H,
      7,1 (breit) 1 H, austauschbar mit D₂0,
      7,15 - 7,45 (m) 7H,
      7,50 (s) 1 H,
      7,70 (m) 1 H,
      8,55 (m) 1 H,
      8,90 (breit) 1 H, austauschbar mit D₂0,
      13,43 (breit) 1 H, austauschbar mit D₂0, ppm.

### Beispiel 18

### N¹-Ethoxycarbonyl-N²-[3-(4-fluorphenyl)-3-phenylpropyl]-N³-[3-(1H-imidazol-4-yl)propyl]-guanidin

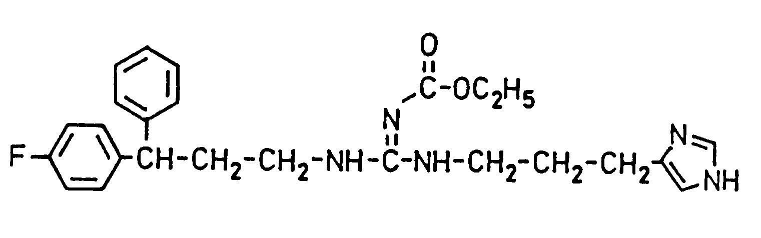

Die Herstellung erfolgt analog Beispiel 12 ausgehend von 1,15 g (5 mmol) 3-(4-Fluorphenyl)-3-phenylpropylamin. Ausbeute: 1,1 g (49 %) farblose Kristalle vom Schmp. 131 ° C (Ethylacetat/Ether).

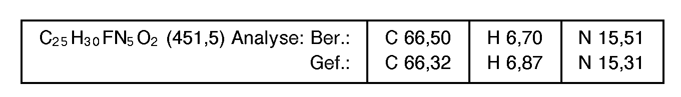

MS (FAB-Methode): m/z (rel. Int. [%]) = 452 ([M+H]⁺, 6), 406 (4), 185 (20), 109 (100), 95(18), 91 (20).
¹H-NMR-Daten: (d₆-DMSO, TMS als interner Standard)
   δ = 1,13 (t) 3H,
      1,75 (m) 2H,
      2,2 - 2,35 (m) 2H,
      2,5 (m, teilweise verdeckt) 2H,
      3,08 (m) 2H,
      3,17 (m) 2H,
      3,89 (q) 2H,
      4,01 (t) 1 H,
      6,6 - 7,1 (breit) 2H, 1 H austauschbar mit D₂0,
      7,1 - 7,4 (m) 9H,
      7,49 (s) 1 H,
      8,9 (breit) 1 H, austauschbar mit D₂0,
      11,8 (breit) 1 H, austauschbar mit D₂0, ppm.

### Beispiel 19

### N¹-(3,3-Diphenylpropyl)-N²-ethoxycarbonyl-N³-[3-(1H-imidazol-4-yl)propyl]-guanidin

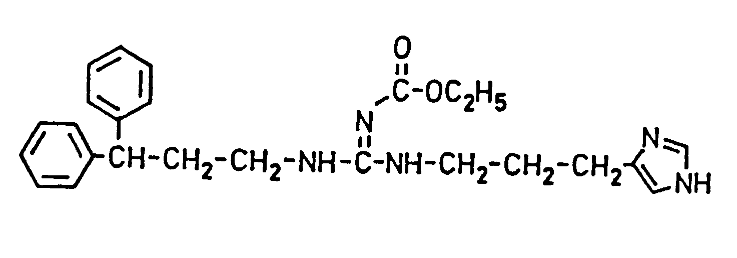

Die Herstellung erfolgt analog Beispiel 1 ausgehend von 2,06 g (5 mmol) N-(3,3-Diphenylpropyl)-S-methyl-isothiuronium-iodid. Die Lösung des zunächst analog Beispiel 1 a) hergestellten N¹-(3,3-Diphenylpro- pyl)-N²⁻ethoxycarbonyl-S-methyl-isothioharnstoffes in Methylenchlorid wurde mit Wasser gewaschen, über Natriumsulfat getrocknet, i. Vak. eingedampft und der Rückstand in 20 ml Acetonitril aufgenommen. Nach Zusatz von 0,65 g (5,2 mmol) 3-(1H-Imidazol-4-yl)propylamin wurde 20 h unter Rückfluß erhitzt, i. Vak. eingedampft und das Produkt durch Chromatographie an Kieselgel 60 PF₂₅₄ gipshaltig isoliert (Fließmittel: Chloroform/Ethanol, 98+2, Ammoniakatmosphäre). Der nach Eindampfen der Eluate erhaltene amorphe Feststoff (0,9 g, 42 %) kristallisiert aus Ethylacetat/Ether in farblosen Kristallen vom Schmp. 125-126 °C.

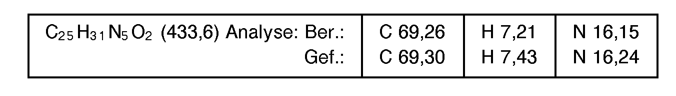

¹H-NMR-Daten: (d₆-DMSO, TMS als interner Standard)
   δ = 1,13 (t) 3H,
      1,75 (m) 2H,
      2,28 (m) 2H,
      2,5 (m, teilweise verdeckt) 2H,
      3,09 (m) 2H,
      3,17 (m) 2H,
      3,89 (q) 2H,
      3,99 (t) 1 H,
      6,76 (breit) 1 H,
      7,0 (breit) 1 H, austauschbar mit D₂0,
      7,1 - 7,35 (m) 10H,
      7,49 (s) 1 H,
      8,95 (breit) 1 H, austauschbar mit D₂0,
      11,9 (breit) 1 H, austauschbar mit D₂0, ppm.

### Beispiel 20

### N¹-Ethoxycarbonyl-N²-[3-(1H-imidazol-4-yl)propyl)-N³-{2-[(pyridin-2-yl)methylthio]ethyl}guanidin

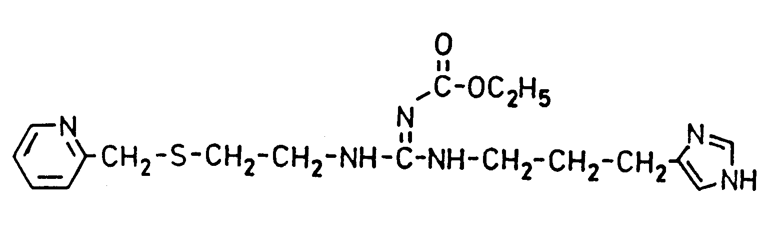

Die Herstellung erfolgt analog Beispiel 19 ausgehend von 1,85 g (5 mmol) N-{2-[(Pyridin-2-yl)-methylthio]ethyll-S-methyl-isothiuronium-iodid. Ausbeute: 1,3 g (54 %) farblose Kristalle vom Schmp. 60-61 °C (Ethylacetat/Ether).

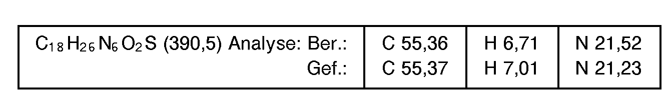

¹H-NMR-Daten: (d₆-DMSO, TMS als interner Standard)
   δ = 1,14 (t) 3H,
      1,77 (m) 2H,
      2,51 (m) 2H,
      2,62 (m) 2H,
      3,19 (m) 2H,
      3,38 (m) 2H,
      3,84 (s) 2H,
      3,93 (q) 2H,
      6,77 (s) 1 H,
      7,2 (sehr breit) 1 H, austauschbar mit D₂0,
      7,26 (dd) 1 H,
      7,44 (d) 1 H,
      7,53 (s) 1 H,
      7,75 (dd) 1 H,
      8,48 (d) 1 H,
      9,0 (breit) 1 H, austauschbar mit D₂0,
      11,8 (breit) 1 H, austauschbar mit D₂0, ppm.

### Beispiel 21

### N¹-Ethoxycarbonyl-N²-[3-(1H-imidazol-4-yl)propyl)-N³-{2[(pyridin-3-yl)methylthio]ethyl}guanidin

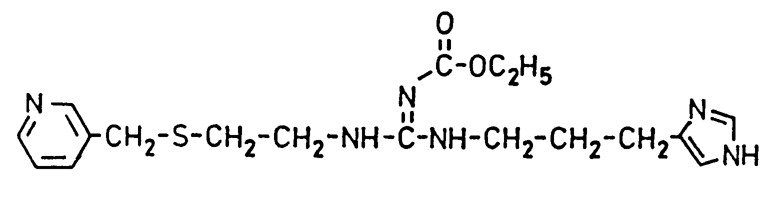

Die Herstellung erfolgt analog Beispiel 19 ausgehend von 1,85 g (5 mmol) N-{2-[(Pyridin-3-yl)-methylthio]ethyl}-S-methyl-isothiuronium-iodid. Ausbeute: 1,2 g (50 %) farblose Kristalle vom Schmp. 90-91 °C (Ethylacetat/Ether).

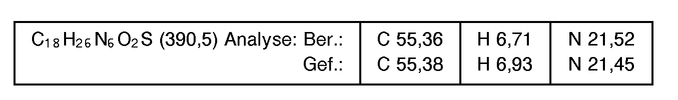

¹H-NMR-Daten: (d₆-DMSO, TMS als interner Standard)
   δ = 1,15 (t) 3H,
      1,78 (m) 2H,
      2,5 - 2,65 (m) 4H,
      3,17 (m) 2H,
      3,38 (m) 2H,
      3,80 (s) 2H,
      3,93 (q) 2H,
      6,78 (breit) 1 H;
      7,2 (sehr breit) 1 H, austauschbar mit D₂0,
      7,34 (dd) 1 H,
      7,53 (s) 1 H,
      7,79 (d) 1 H,
      8,45 (d) 1 H,
      8,54 (s) 1 H,
      7,95 (breit) 1 H, austauschbar mit D₂0,
      11,85 (breit) 1H, austauschbar mit D₂0, ppm.

### Beispiel 22

### N¹-Ethoxycarbonyl-N²⁻[3-(1H-imidazol-4-yl)propyl]-N³-[2-(phenylmethylthio) ethyl]guanidin

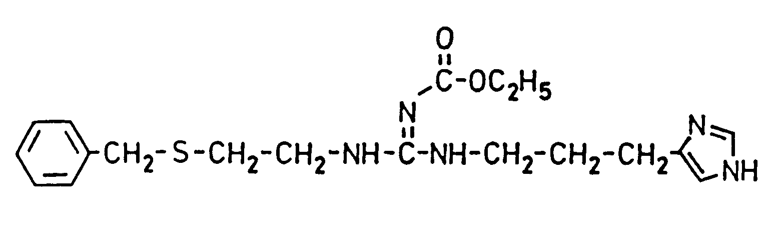

Die Herstellung erfolgt analog Beispiel 12 ausgehend von 0,84 g (5 mmol) 2-(Phenylmethylthio)-ethylamin. Ausbeute: 1,0 g (51 %) farblose Kristalle vom Schmp. 91-92 ° C (Ethylacetat/Ether).

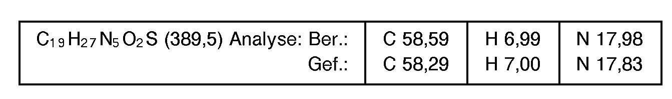

MS (FAB-Methode): m/z (rel. Int. [%]) = 390 ([M+H]⁺, 23), 344 (11), 172 (9), 151 (10), 109 (47), 95 (11), 91 (100).
¹H-NMR-Daten: (d₆-DMSO, TMS als interner Standard)
   δ = 1,15 (t) 3H,
      1,76 (m) 2H,
      2,5 - 2,65 (m, teilweise verdeckt) 4H,
      3,18 (m) 2H,
      3,37 (m) 2H,
      3,76 (s) 2H,
      3,93 (q) 2H,
      6,77 (s) 1 H,
      7,0 (breit) 1 H, austauschbar mit D₂0,
      7,2 - 7,4 (m) 5H,
      7,53 (s) 1 H,
      9,0 (breit) 1 H, austauschbar mit D₂0,
      11,85 (breit) 1H, austauschbar mit D₂0, ppm.

### Beispiel 23

### N¹-{2-[(2-Diaminomethylenaminothiazol-4-yl)methylthio]ethyl}-N²-ethoxycarbonyl-N³-[3-(1H-imidazol-4-yl)-propyl]guanidin

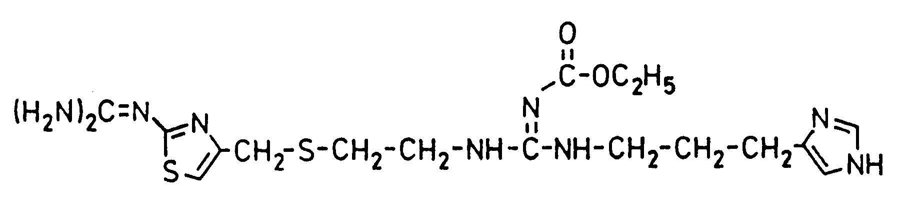

Die Herstellung erfolgt analog Beispiel 12 ausgehend von 1,16 g (5 mmol) 2-[(2-Diaminomethylenaminothiazol-4-yl)methylthio]ethylamin. Ausbeute: 1,2 g (53 %) farblose Kristalle, die nach Umkristallisation aus Ethylacetat/Ethanol bei 110-114 ° C sintern.

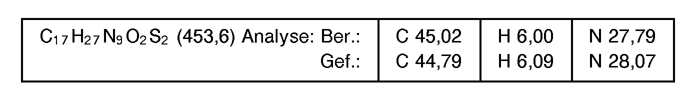

MS (FAB-Methode): m/z (rel. Int. [%]) = 454 ([M+H]⁺, 10), 408 (8), 211 (18), 155 (100), 113 (29), 109 (71), 95 (22), 93 (14), 82 (24), 81 (29).
¹H-NMR-Daten: (d₆-DMSO, TMS als interner Standard)
   δ = 1,14 (t) 3H,
      1,77 (m) 2H,
      2,45 - 2,65 (m) 4H,
      3,18 (m) 2H,
      3,38 (m) 2H,
      3,61 (s) 2H,
      3,93 (q) 2H,
      6,56 (s) 1 H,
      6,77 (s) 1 H,
      6,84 (breit) 4H, austauschbar mit D₂0,
      7,75 (breit) 1 H, austauschbar mit D₂0,
      7,52 (s) 1 H,
      8,95 (breit) 1 H, austauschbar mit D₂0,
      11,85 (breit) 1 H, austauschbar mit D₂0, ppm.

## Patentansprüche (Patentansprüche für folgende(n) Vertragsstaat(en) : AT, BE, CH, DE, FR, GB, IT, LI, LU, NL, SE)

1. Guanidincarbonsäureester der allgemeinen Formel I 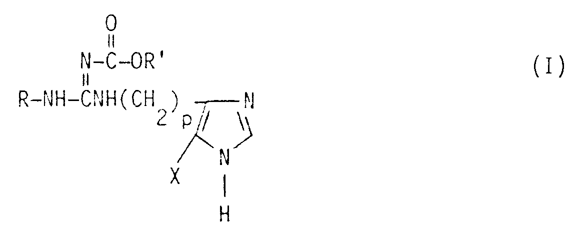 in der
(a) R die Gruppierung 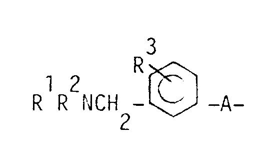 bedeutet, wobei R¹ und R², die gleich oder verschieden sein können, jeweils für Wasserstoff, lineares C₁-C₆-Alkyl oder C₅-C₆-Cycloalkyl stehen oder R¹ und R² zusammen mit dem Stickstoffatom, an das sie gebunden sind, einen 5- bis 10gliedrigen stickstoffhaltigen alicyclischen, heterocyclischen Ring bilden, R³ für ein Wasserstoffatom, ein Halogenatom oder eine C₁-C₃-Alkoxygruppe steht, A die Gruppierung -0-(CH₂)ₖ-, -O-CH₂CH(OH)CH₂-, -O-CH₂CH(CH₃)-CH₂-,-CH₂-O-CH₂-CH-(OH)-CH₂-oder -O-CH₂-CH(OH)-CH(OH)-CH₂ bedeutet, worin k den Wert 3 oder 4 hat, oder
(b) R die Gruppierung 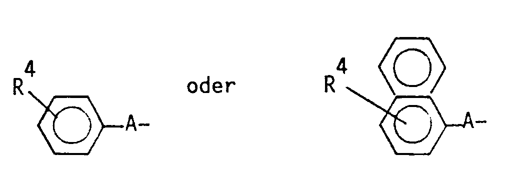 bedeutet, wobei R⁴ für ein Wasserstoffatom, ein vorzugsweise in para-Stellung zu A gebundenes Halogenatom, eine C₁-C₃-Alkoxy- oder C₁-C₃-Alkylgruppe steht und A die oben unter (a) genannte Bedeutung besitzt, oder
(c) R die Gruppierung 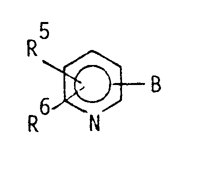 bedeutet, wobei R⁵ und R⁶, die gleich oder verschieden sein können, jeweils für ein Wasserstoffatom, ein Halogenatom oder eine lineare C₁-C₃-Alkyl- oder eine lineare C₁-C₃-Alkoxygruppe stehen, B in Position 2, 3 oder 4 des Pyridinrings gebunden sein kann und die Gruppierung 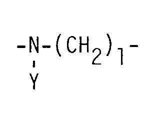 oder -(CH₂)ₘ- bedeutet, wobei I den Wert 2, 3 oder 4 und m den Wert 3, 4 oder 5 haben und Y für ein Wasserstoffatom oder für eine lineare C₁-C₃-Alkylgruppe steht, oder
(d) R die Gruppierung 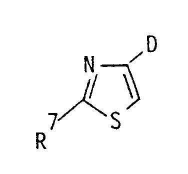 bedeutet, wobei R⁷ für die Gruppe (R¹R²)N-CH₂-, (H₂N)₂C = N-, 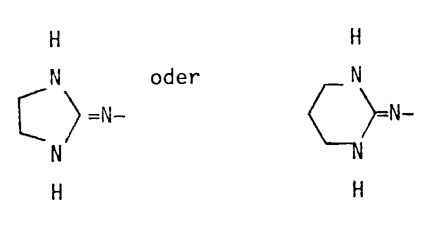 steht, wobei R¹ und R² die oben unter (a) angegebene Bedeutung besitzen, D für die Gruppierung-CH₂-S-(CH₂)ₙ- oder -(CH₂)ₒ- steht, wobei n den Wert 2 oder 3 und o den Wert 2, 3 oder 4 haben, oder
(e) R die Gruppierung 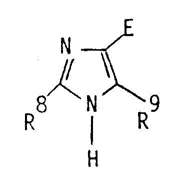 bedeutet, wobei R⁸ für ein Wasserstoffatom, eine gegebenenfalls durch ein Halogenatom substituierte Benzylgruppe, die Gruppierung (R¹R²)N-CH₂- oder eine Aminogruppe steht, wobei R¹ und R² die oben unter (a) genannten Bedeutungen haben, R⁹ ein Wasserstoffatom, eine lineare C₁-C₃-Alkyl-oder C₁-C₃-Alkylthiogruppe bedeutet, E für die Gruppierung 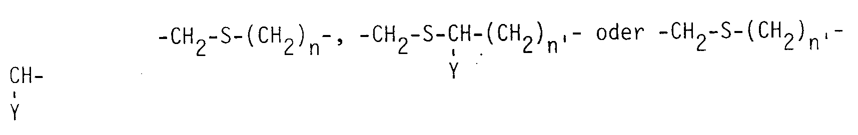 steht, wobei n den Wert 2 oder 3 und n' den Wert 1, 2 oder 3 hat und Y die oben unter (c) angegebene Bedeutung besitzt, oder
(f) R die Gruppierung 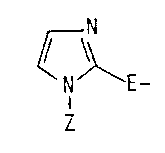 bedeutet, wobei Z ein Wasserstoffatom oder eine lineare C₁-C₃-Alkylgruppe bedeutet und E die oben unter (e) genannte Bedeutung besitzt, oder
(g) R die Gruppierung R"-A'-B'- bedeutet, wobei R" für eine unsubstituierte oder mit Halogen, linearem C₁-C₃-Alkyl oder linearem C₁-C₃-Alkoxy substituierte Phenylgruppe oder eine unsubstituierte oder mit Halogen, linearem C₁-C₃-Alkyl oder linearem C₁-C₃-Alkoxy substituierte Naphthylgruppe steht, A' für eine Einfachbindung oder für die Gruppierung -CR^{1'}R^{2'} oder für ein - mit einer Phenyl-, Naphthyl-, Benzyl-, Pyridinyl-, Furanyl- oder Thiophenylgruppe, die gegebenenfalls mit Halogen, linearem C₁-C₃-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R^{2'} für eine Phenylgruppe, die gegebenenfalls mit Halogen, linearem C₁-C₃-Alkyl oder linearem C₁-C₃-Alkoxy substituiert ist, oder eine Pyridinyl-, Furanyl- oder Thiophenylgruppe, die gegebenenfalls mit Halogen, linearem C₁-C₃-Alkyl oder linearem C₁-C₃-Alkoxy substituiert ist, steht, B' für die Gruppierung -CH(Y)-S-(CH2)m-, -CH₂-S-CH₂-CH(Y)-CH₂-, -CH₂-S-CH(Y)-CH₂-, -CH₂-S-CH₂-CH(Y)-, -(CH₂)_{n"}-, -(CH2)-CH(Y)-, -(CH₂)_{n"}-CH(Y), -O-(CH₂)₂-, -CH₂-O-(CH₂)_{o'}-, -CH₂-O-CH₂-CH(Y)-CH₂-, -O-CH₂-CH(Y)-, -O-CH(Y)-CH₂-, -S-(CH₂)q-, -S-CH₂-CH(Y)-, -S-CH(Y)-CH₂ oder -S-CH₂-CH(Y)-CH₂- steht, wobei Y ein Wasserstoffatom oder eine lineare C₁-C₃-Alkylgruppe bedeutet, m' und o' jeweils den Wert 2 oder 3 haben, n" und q jeweils den Wert 2, 3, 4 oder 5 haben, oder
(h) R die Gruppierung R"'-A"-B"- bedeutet, wobei R"' für eine unsubstituierte oder mit Halogen, linearem C₁-C₃-Alkyl oder linearem C₁-C₃-Alkoxy substituierte Pyridinyl-, Imidazolyl-, Pyrimidinyl-, Thiophenyl-, Furanyl-, Benzimidazolyl- oder Chinolinylgruppe steht, an die gegebenenfalls ein Phenylring ankondensiert sein kann, A" für eine Einfachbindung oder für die Gruppierung -CR^{1'}R^{2'} oder für ein - mit einer Phenyl-, Naphthyl-, Benzyl-, Pyridinyl-, Benzimidazolyl-, Thiophenyl- oder Furanylgruppe, die gegebenenfalls mit Halogen, linearem C₁-C₃-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem C1-C3-Alkyl oder linearem C₁-C₃-Alkoxy substituiert ist, oder eine Benzimidazolyl-, Pyridinyl-, Thiophenyl- oder Furanylgruppe, die gegebenenfalls mit Halogen, linearem C₁-C₃-Alkyl oder linearem C₁-C₃-Alkoxy substituiert ist, steht, B" für die Gruppierung -CH(Y)-S-(CH₂)_{m'}-, -CH₂-S-CH₂-CH(Y)-CH₂-, -CH₂-S-CH(Y)-CH₂-, -(CH₂)_{n"}-CH(Y)-, -CH₂-S-CH₂-CH(Y)-, -(CH₂)-n -O(CH₂)_{n"}-, -S-CH2-CH(Y)-, S-CH(Y)-CH₂-, -S-(CH₂)q- oder -S-CH₂-CH(Y)-CH₂- steht, wobei Y ein Wasserstoffatom oder eine lineare C₁-C₃-Alkylgruppe bedeutet, m' den Wert 2 oder 3 hat und n" und q den Wert 2, 3, 4 oder 5 haben,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet, sowie die physiologisch annehmbaren Salze davon.

2. Guanidincarbonsäureester nach Anspruch 1, dadurch gekennzeichnet, daß R für die Gruppierung 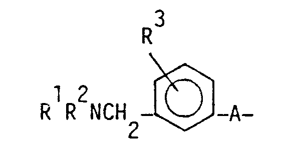 steht, R¹ und R² zusammen mit dem Stickstoffatom einen Pyrrolidin- oder Piperidinring bilden, R³ für ein Wasserstoffatom steht, A die Gruppierung -O(CH₂)ₖ-, OCH₂CH(OH)CH₂- oder OCH₂CH(CH₃)CH₂- bedeutet, R' eine lineare oder verzweigte, gegebenenfalls ein- oder mehrfach mit einem Halogenatom, einer C₁-C₃-Alkoxygruppe und/oder einem Phenyl- oder Naphthylrest substituierte Ci-C6-Alkyl- oder Cycloalkylgruppe bedeutet, k und p den Wert 3 haben und X für ein Wasserstoffatom steht.

3. Guanidincarbonsäureester nach Anspruch 1, dadurch gekennzeichnet, daß R für die Gruppierung 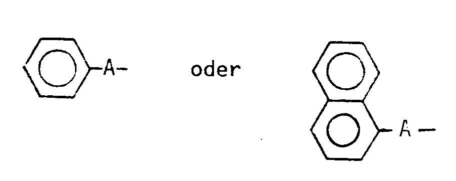 steht, wobei A die Gruppierung -0-(CH₂)ₖ-, -O-CH₂CH(OH)CH₂-, -O-CH₂CH(CH₃)-CH₂-, -CH₂-O-CH₂-CH(OH)-CH₂- oder -O-CH₂-CH(OH)-CH(OH)-CH₂ bedeutet,
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

4. Guanidincarbonsäureester nach Anspruch 1, dadurch gekennzeichnet, daß R für die Gruppierung 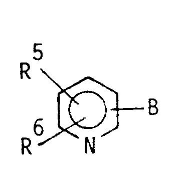 steht, wobei R⁵ für ein in 5-Stellung des Pyridinrings gebundenes Halogenatom oder Wasserstoffatom steht, R⁶ für ein in 3-Stellung des Pyridinrings gebundenes Wasserstoffatom, eine Methyl- oder eine Methoxygruppe steht, B in Position 2, 3 oder 4 des Pyridinrings gebunden sein kann und die Gruppierung 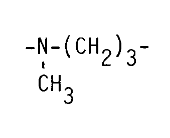 oder -(CH₂)₃-₄- oder -NH-(CH₂), bedeutet, wobei I den Wert 2, 3 oder 4 hat,
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

5. Guanidincarbonsäureester nach Anspruch 1, dadurch gekennzeichnet, daß R für die Gruppierung 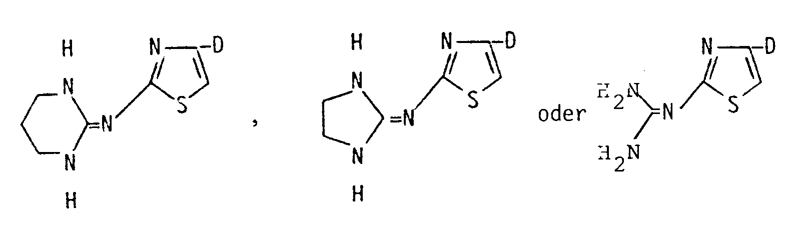 steht, D die Gruppierung -CH₂-S-(CH₂)₂- oder -(CH₂)₃- oder -(CH₂)₄-bedeutet und
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C1-C6-Alkyl- oder Cycloalkylgruppe bedeutet, p den Wert 2 oder 3 hat und X ein Wasserstoffatom oder eine Methylgruppe bedeutet.

6. Guanidincarbonsäureester nach Anspruch 1, dadurch gekennzeichnet, daß R für die Gruppierung 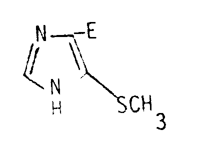 steht, wobei E die Gruppierung -CH₂-S-(CH₂)₂-, 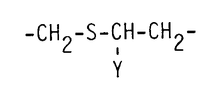 oder 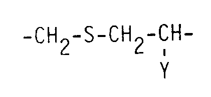 bedeutet, Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

7. Guanidincarbonsäureester nach Anspruch 1, dadurch gekennzeichnet, daß R für die Gruppierung 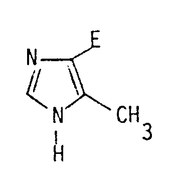 steht, E die Gruppierung -CH₂-S-(CH₂)₂-, 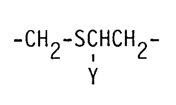 oder 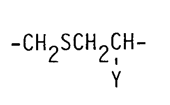 bedeutet, wobei Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

8. Guanidincarbonsäureester nach Anspruch 1, dadurch gekennzeichnet, daß R für die Gruppierung 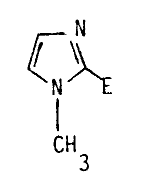 steht, F für die Gruppierung 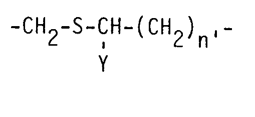 oder 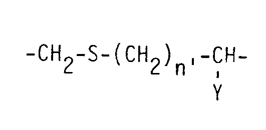 steht, wobei n' den Wert 1, 2 oder 3 hat und Y für ein Wasserstoffatom oder für eine lineare Cₗ-C₃-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

9. Guanidincarbonsäureester nach Anspruch 1, dadurch gekennzeichnet, daß R die Gruppierung R"-A'-B'- bedeutet, wobei R" für eine unsubstituierte Phenylgruppe steht, A' eine Einfachbindung bedeutet, B' für die Gruppierung -CH₂-S-(CH₂)_{m'}- oder -CH₂-S-CH₂-CH(Y)-CH₂-steht, wobei m' den Wert 2 oder 3 hat und Y für ein Wasserstoffatom oder eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

10. Guanidincarbonsäureester nach Anspruch 1, dadurch gekennzeichnet, daß R die Gruppierung R"-A'-B'-bedeutet, wobei R" für eine unsubstituierte oder mit Halogen, linearem C₁-C₃-Alkyl oder linearem C₁-C₃-Alkoxy substituierte Phenylgruppe steht, A' ein mit einer Phenyl-, Naphthyl-, Pyridinyl-, Furanyl oder Thiophenylgruppe substituiertes Stickstoffatom bedeutet, B' für die Gruppierung -(CH₂)_{n"}- steht, wobei n" den Wert 2 oder 3 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

11. Guanidincarbonsäureester nach Anspruch 1, dadurch gekennzeichnet, daß R die Gruppierung R"-A'-B'- bedeutet, wobei R" für eine unsubstituierte oder mit Halogen, linearem C₁-C₃-Alkyl oder linearem C₁-C₃-Alkoxy substituierte Phenylgruppe steht, A' für die Gruppierung -CR^{1'}R^{2'}- steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Pyridinyl-, Furanyl oder Thiophenylgruppe, die gegebenenfalls mit Halogen, linearem C1-C3-Alkyl oder linearem Cₗ-C₃-Alkoxy substituiert ist, steht, B' die Gruppierung -(CH₂)_{n"}- bedeutet, wobei n" den Wert 2, 3 oder 4 hat, R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

12. Guanidincarbonsäureester nach Anspruch 1, dadurch gekennzeichnet, daß R die Gruppierung R"'-A"-B"- bedeutet, wobei R"' für einen unsubstituierten oder mit Halogen, linearem C₁-C₃-Alkyl oder linearem C₁-C₃-Alkoxy substituierten Pyridinring steht, A" für eine Einfachbindung in Position 2, 3 oder 4 des Pyridinrings und B" für die Gruppierung -CH2-S-(CH2)m'- oder -CH₂-S-CH₂-CH(Y)-CH₂-steht, wobei m' den Wert 2 oder 3 hat und Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

13. Guanidincarbonsäureester nach Anspruch 1, dadurch gekennzeichnet, daß R die Gruppierung R"'-A"-B"- bedeutet, wobei R"' für einen unsubstituierten oder mit Halogen, linearem C₁-C₃-Alkyl oder linearem C₁-C₃-Alkoxy substituierten Thiophenring steht, A" eine Einfachbindung bedeutet, B" für die Gruppierung -CH₂-S-(CH₂)_{m'}-oder -CH₂-S-CH₂-CH(Y)-CH₂- steht, wobei m' den Wert 2 oder 3 hat und Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

14. Guanidincarbonsäureester nach Anspruch 1, dadurch gekennzeichnet, daß R die Gruppierung R"'-A"-B"- bedeutet, wobei R"' für einen unsubstituierten oder mit Halogen, linearem C₁-C₃-Alkyl oder linearem C₁-C₃-Alkoxy substituierten Pyridinring steht, A" die Gruppierung -CR^{1'R2'} bedeutet, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Pyridinyl-, Furanyl, Thiophenyl- oder Benzimidazolylgruppe, die gegebenenfalls mit Halogen, linearem C₁-C₃-Alkyl oder linearem C₁-C₃-Alkoxy substituiert ist, steht, B" für die Gruppierung -(CH₂)_{n"} - steht, wobei n" den Wert 2, 3, 4 oder 5 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

15. Guanidincarbonsäureester nach Anspurch 1, dadurch gekennzeichnet, daß R die Gruppierung R"'-A"-B" bedeutet, wobei R"' für einen unsubstituierten oder mit Halogen, linearem C₁-C₃-Alkyl oder linearem C₁-C₃-Alkoxy substituierten Pyridinring steht, A" für eine Einfachbindung in Position 2, 3 oder 4 des Pyridinrings steht, B" für die Gruppierung -(CH₂)_{n"}- steht, wobei n" den Wert 2, 3, 4 oder 5 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

16. Guanidincarbonsäureester nach Anspruch 1, dadurch gekennzeichnet, daß R die Gruppierung R"'-A"-B"- bedeutet, wobei R"' für einen unsubstituierten oder mit Halogen, linearem C₁-C₃-Alkyl oder linearem C₁-C₃-Alkoxy substituierten Pyridinring steht, A" für eine Einfachbindung oder für die Gruppierung -CR^{1'}R^{2'} oder für ein - mit einer Phenyl-, Naphthyl-, Benzyl-, Pyridinyl-, Benzimidazolyl-, Thiophenyl- oder Furanylgruppe, die gegebenenfalls mit Halogen, linearem C1-C3-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Benzimidazolyl-, Pyridinyl-, Thiophenyl- oder Furanylgruppe, die gegebenenfalls mit Halogen, linearem C₁-C₃-Alkyl oder linearem C₁-C₃-Alkoxy substituiert ist, steht, B" für die Gruppierung -(CH₂)_{n"}- steht, wobei n" den Wert 2, 3, 4 oder 5 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

17. Guanidincarbonsäureester nach Anspruch 1, dadurch gekennzeichnet, daß R die Gruppierung R"'-A"-B"- bedeutet, wobei R"' für die Gruppierung 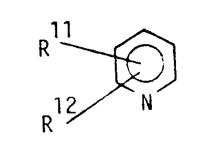 steht, wobei R¹¹ und R¹² unabhängig voneinander jeweils ein Halogenatom, eine lineare Cₗ-C₃-Alkylgruppe oder eine lineare C₁-C₃-Alkoxygruppe bedeuten, A" für die Gruppierung -CR^{1'}R^{2'} steht, wobei R^{1'} ein Wasserstoffatom oder eine Methylgruppe und R eine unsubstituierte oder eine ein- bis dreifach mit Halogen, linearem C₁-C₃-Alkyl oder linearem C₁-C₃-Alkoxy substituierte Phenylgruppe bedeuten, oder A" für ein Stickstoffatom , das mit einer Phenyl-, Naphthyl-, Pyridinyl-, Furanyl-, Thiophenyl- oder Benzimidazolylgruppe oder einem Wasserstoffatom substituiert ist, steht, B", wenn A" die Gruppierung -CR^{1'}R^{2'} ist, für die Gruppierung -(CH₂)ₙ-, -O(CH₂)₂- oder -SCH₂CH₂-steht und B", wenn A" ein mit einer Phenyl-, Naphthyl-, Pyridinyl-, Furanyl-, Thiophenyl- oder Benzimidazolylgruppe oder einem Wasserstoffatom substituiertes Stickstoffatom ist, für die Gruppierung -(CH₂)ₙ- steht, wobei n den Wert 2 oder 3 hat, und
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

18. Guanidincarbonsäureester nach Anspruch 1, dadurch gekennzeichnet, daß R die Gruppierung R"'-A"-B"- bedeutet, wobei R"' für die Gruppierung 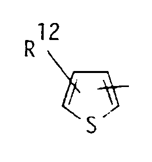 steht, worin R¹² ein Wasserstoffatom, ein Halogenatom, eine lineare C₁-C₃-Alkylgruppe, die Gruppierung (CH₃)₂-NCH₂- oder 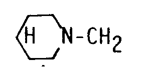 bedeutet, A" für die Gruppierung -CR^{1'}R^{2'} steht, wobei R^{1'} ein Wasserstoffatom oder eine Methylgruppe und R eine unsubstituierte oder eine ein- bis dreifach mit Halogen, linearem C₁-C₃-Alkyl oder linearem C₁-C₃-Alkoxy substituierte Phenylgruppe bedeuten, oder A" für ein Stickstoffatom, das mit einer Phenyl-, Naphthyl-, Pyridinyl-, Furanyl-, Thiophenyl- oder Benzimidazolylgruppe oder einem Wasserstoffatom substituiert ist, steht, B" für die Gruppierung -CH₂-S-CH₂-CH₂-, -CH₂-S-CH₂-CH(CH₃)-CH₂-, -CH₂-S-CH(CH₃)-CH₂- oder -CH₂-S-CH₂-CH(CH₃)-steht, und
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

19. Guanidincarbonsäureester nach Anspruch 1, dadurch gekennzeichnet, daß R die Gruppierung R"'-A"-B"- bedeutet, wobei R"' für die Gruppierung 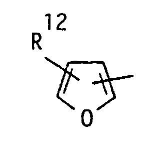 steht, worin R¹² ein Wasserstoffatom, ein Halogenatom, eine lineare C₁-C₃-Alkylgruppe, die Gruppierung (CH₃)₂-NCH₂- oder 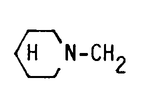 bedeutet, A" eine Einfachbindung in Position 2 bedeutet, B" für die Gruppierung -CH₂S-CH₂-CH₂-, -CH₂-S-CH₂-CH(CH₃)-CH₂-, -CH₂-S-CH(CH₃)-CH₂- oder -CH₂-S-CH₂-CH(CH₃) steht, und
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C1-C6-Alkyl- oder Cycloalkylgruppe bedeutet, p den Wert 2 oder 3 hat und X ein Wasserstoffatom oder eine Methylgruppe bedeutet.

20. Guanidincarbonsäureester nach Anspruch 1, dadurch gekennzeichnet, daß R die Gruppierung R"'-A"-B"- bedeutet, wobei R"' die Gruppierung 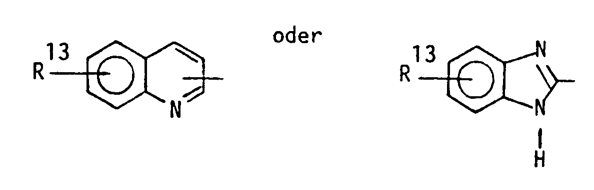 bedeutet, worin R¹³ ein Wasserstoffatom, ein Halogenatom, eine lineare C₁-C₃-Alkylgruppe, die Gruppierung (CH₃)₂-NCH₂- oder 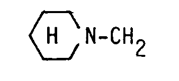 bedeutet, A" eine Einfachbindung bedeutet, B" für die Gruppierung -CH₂-S-CH₂-CH₂-, -CH₂-S-CH₂-CH(CH₃)-CH₂-, -CH₂-S-CH(CH₃)-CH₂- oder -CH₂-S-CH₂-CH(CH₃) steht, und
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

21. N¹-[3-(Imidazol-4-yl)propyl]-N²-[4-(pyridin-2-yl)butyl]-N³-methoxycarbonylguanidin und die physiologisch annehmbaren Salze.

22. N¹-[3-(Imidazol-4-yl)propyl]-N²-[4-(pyridin-2-yl)butyl]-N³-ethoxycarbonylguanidin und die physiologisch annehmbaren Salze.

23. N¹-[3-(Imidazol-4-yl)propyl]-N²-[3-(4-fluorphenyl)-3-(pyridin-2-yl)propyl]-N³-methoxycarbonylguanidin und die physiologisch annehmbaren Salze.

24. N¹-[3-(Imidazol-4-yl)propyl]-N²-[3-(4-fluorphenyl)-3-(pyridin-2-yl)propyl]-N³-ethoxycarbonylguanidin und die physiologisch annehmbaren Salze.

25. N¹-[3-(Imidazol-4-yl)propyl]-N²-[3-(4-fluorphenyl)-3-(pyridin-2-yl)propyl]-N³-butoxycarbonylguanidin und die physiologisch annehmbaren Salze.

26. Verfahren zur Herstellung von Guanidinderivaten der allgemeinen Formel I 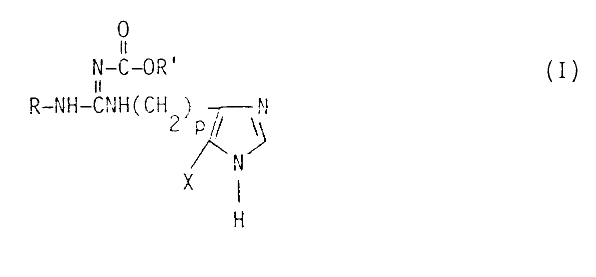 in der
(a) R die Gruppierung 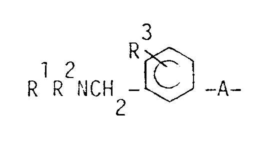 bedeutet, wobei R¹ und R², die gleich oder verschieden sein können, jeweils für Wasserstoff, lineares C₁-C₆-Alkyl oder C₅-C₆-Cycloalkyl stehen oder R¹ und R² zusammen mit dem Stickstoffatom, an das sie gebunden sind, einen 5- bis 10gliedrigen stickstoffhaltigen alicyclischen, heterocyclischen Ring bilden, R³ für ein Wasserstoffatom, ein Halogenatom oder eine C₁-C₃-Alkoxygruppe steht, A die Gruppierung -0-(CH₂)ₖ-, -O-CH₂CH(OH)CH₂-, -O-CH₂CH(CH₃)-CH₂-,-CH₂-O-CH₂-CH-(OH)-CH₂- oder -O-CH₂-CH(OH)-CH(OH)-CH₂ bedeutet, worin k den Wert 3 oder 4 hat, oder
(b) R die Gruppierung 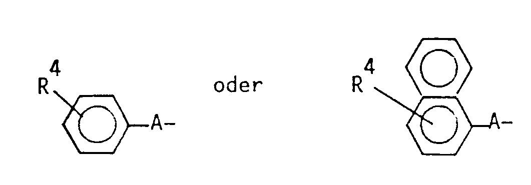 bedeutet, wobei R⁴ für ein Wasserstoffatom, ein vorzugsweise in para-Stellung zu A gebundenes Halogenatom, eine C₁-C₃-Alkoxy- oder C₁-C₃-Alkylgruppe steht und A die oben unter (a) genannte Bedeutung besitzt, oder
(c) R die Gruppierung 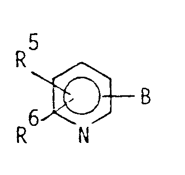 bedeutet, wobei R⁵ und R⁶, die gleich oder verschieden sein können, jeweils für ein Wasserstoffatom, ein Halogenatom oder eine lineare C₁-C₃-Alkyl- oder eine lineare C₁-C₃-Alkoxygruppe stehen, B in Position 2, 3 oder 4 des Pyridinrings gebunden sein kann und die Gruppierung 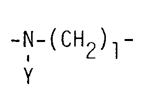 oder -(CH₂)ₘ- bedeutet, wobei I den Wert 2, 3 oder 4 und m den Wert 3, 4 oder 5 haben und Y für ein Wasserstoffatom oder für eine lineare C₁-C₃-Alkylgruppe steht, oder
(d) R die Gruppierung 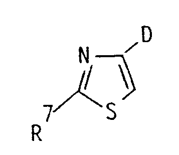 bedeutet, wobei R⁷ für die Gruppe (R¹R²)N-CH₂-, (H₂N)₂ C = N-, 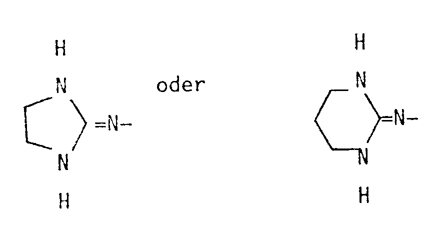 steht, wobei R¹ und R² die oben unter (a) angegebene Bedeutung besitzen, D für die Gruppierung -CH₂-S-(CH₂)ₙ- oder -(CH₂)ₒ- steht, wobei n den Wert 2 oder 3 und o den Wert 2, 3 oder 4 haben, oder
(e) R die Gruppierung 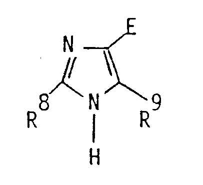 bedeutet, wobei R⁸ für ein Wasserstoffatom, eine gegebenenfalls durch ein Halogenatom substituierte Benzylgruppe, die Gruppierung (R¹R²)N-CH₂- oder eine Aminogruppe steht, wobei R¹ und R² die oben unter (a) genannten Bedeutungen haben, R⁹ ein Wasserstoffatom, eine lineare C₁-C₃-Alkyl-oder C₁-C₃-Alkylthiogruppe bedeutet, E für die Gruppierung 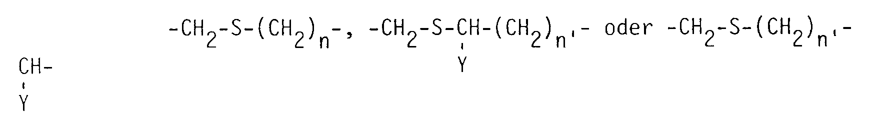 steht, wobei n den Wert 2 oder 3 und n' den Wert 1, 2 oder 3 hat und Y die oben unter (c) angegebene Bedeutung besitzt, oder
(f) R die Gruppierung 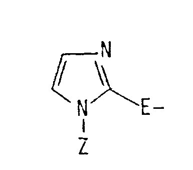 bedeutet, wobei Z ein Wasserstoffatom oder eine lineare C₁-C₃-Alkylgruppe bedeutet und E die oben unter (e) genannte Bedeutung besitzt, oder
(g) R die Gruppierung R"-A'-B'- bedeutet, wobei R" für eine unsubstituierte oder mit Halogen, linearem C₁-C₃-Alkyl oder linearem C₁-C₃-Alkoxy substituierte Phenylgruppe oder eine unsubstituierte oder mit Halogen, linearem C₁-C₃-Alkyl oder linearem C₁-C₃-Alkoxy substituierte Naphthylgruppe steht, A' für eine Einfachbindung oder für die Gruppierung -CR^{1'}R^{2'} oder für ein - mit einer Phenyl-, Naphthyl-, Benzyl-, Pyridinyl-, Furanyl- oder Thiophenylgruppe, die gegebenenfalls mit Halogen, linearem C₁-C₃-Alkyl oder linearem C₁-C₃-Alkoxy substituiert ist, oder linearen Ci-C₃-Alkylgruppe substituiertes - Stickstoffatom steht, wobei R^{1'} ein Wasserstoffatom oder eine Methylgruppe bedeutet, R^{2'} für eine Phenylgruppe, die gegebenenfalls mit Halogen, linearem C₁-C₃-Alkyl oder linearem C₁-C₃-Alkoxy substituiert ist, oder eine Pyridinyl-, Furanyl- oder Thiophenylgruppe, die gegebenenfalls mit Halogen, linearem C₁-C₃-Alkyl oder linearem C₁-C₃-Alkoxy substituiert ist, steht, B' für die Gruppierung -CH(Y)-S-(CH2)m-, -CH₂-S-CH₂-CH(Y)-CH₂-, -CH₂-S-CH(Y)-CH₂-, -CH₂-S-CH₂-CH(Y)-, -(CH₂)_{n"}- -(CH2)-CH(Y)-, -(CH₂)_{n"}-CH(Y)-, -O-(CH₂)₂-, -CH₂-O-(CH₂)_{o'}-, -CH₂-O-CH₂-CH(Y)-CH₂-, -O-CH₂-CH(Y)-, -O-CH(Y)-CH₂-, -S-(CH₂)q-, -S-CH₂-CH(Y)-, -S-CH(Y)-CH₂ oder -S-CH₂-CH(Y)-CH₂- steht, wobei Y ein Wasserstoffatom oder eine lineare C₁-C₃-Alkylgruppe bedeutet, m' und o' jeweils den Wert 2 oder 3 haben, n" und q jeweils den Wert 2, 3, 4 oder 5 haben, oder
(h) R die Gruppierung R"'-A"-B"- bedeutet, wobei R"' für eine unsubstituierte oder mit Halogen, linearem C₁-C₃-Alkyl oder linearem C₁-C₃-Alkoxy substituierte Pyridinyl-, Imidazolyl-, Pyrimidinyl-, Thiophenyl-, Furanyl-, Benzimidazolyl- oder Chinolinylgruppe steht, an die gegebenenfalls ein Phenylring ankondensiert sein kann, A" für eine Einfachbindung oder für die Gruppierung -CR^{1'}R^{2'} oder für ein - mit einer Phenyl-, Naphthyl-, Benzyl-, Pyridinyl-, Benzimidazolyl-, Thiophenyl- oder Furanylgruppe, die gegebenenfalls mit Halogen, linearem C₁-C₃-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Benzimidazolyl-, Pyridinyl-, Thiophenyl- oder Furanylgruppe, die gegebenenfalls mit Halogen, linearem C₁-C₃-Alkyl oder linearem C₁-C₃-Alkoxy substituiert ist, steht, B" für die Gruppierung -CH(Y)-S-(CH2)_{m'}-, -CH₂-S-CH₂-CH(Y)-CH₂-, -CH₂-S-(H(Y)-CH₂-, -(CH2)_{n"}-CH(Y)-, -CH₂-S-CH₂-CH(Y)-, -(CH₂)-n -O(CH₂)_{n"}-, -S-CH2-CH(Y)-, S-CH(Y)-CH₂-, -S-(CH₂)q- oder -S-CH₂-CH(Y)-CH₂- steht, wobei Y ein Wasserstoffatom oder eine lineare C₁-C₃-Alkylgruppe bedeutet, m' den Wert 2 oder 3 hat und n" und q den Wert 2, 3, 4 oder 5 haben,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte Cᵢ-C₆-Alkyl- oder Cycloalkylgruppe bedeutet, p den Wert 2 oder 3 hat und X ein Wasserstoffatom oder eine Methylgruppe bedeutet, sowie der physiologisch annehmbaren Salze davon,
dadurch gekennzeichnet, daß man
(1) eine Verbindung der allgemeinen Formel II 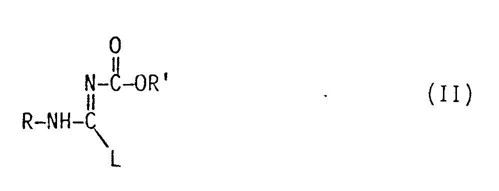 in der R und R' die oben angegebenen Bedeutungen besitzen und L für eine Austrittsgruppe steht, mit einer Verbindung der allgemeinen Formel III 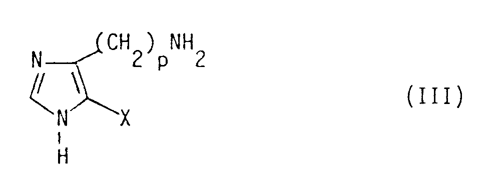 in der X und p wie oben definiert sind, zu einer Verbindung der allgemeinen Formel I umsetzt oder
(2) eine Verbindung der allgemeinen Formel IV 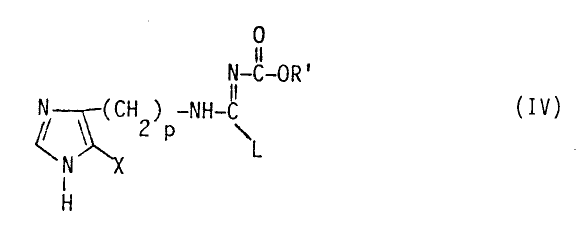 in der R', X und p die oben angegebenen Bedeutungen besitzen und L für eine Austrittsgruppe steht, mit einer Verbindung der allgemeinen Formel V
R-NH₂ (V)
in der R die oben angegebene Bedeutung besitzt, zu einer Verbindung der allgemeinen Formel I umsetzt
und daß man gegebenenfalls die nach (1) oder (2) erhaltenen Verbindungen in an sich bekannter Weise in ihr physiologisch annehmbares Salz umwandelt.

27. Arzneimittel, dadurch gekennzeichnet, daß es eine Verbindungen nach den Ansprüchen 1 bis 25 und mindestens einen inerten, pharmazeutisch annehmbaren Träger oder ein inertes, pharmazeutisch annehmbares Verdünnungsmittel enthält.

## Patentansprüche (Patentansprüche für folgende(n) Vertragsstaat(en) : ES, GR)

1. Verfahren zur Herstellung von Guanidinderivaten der allgemeinen Formel I 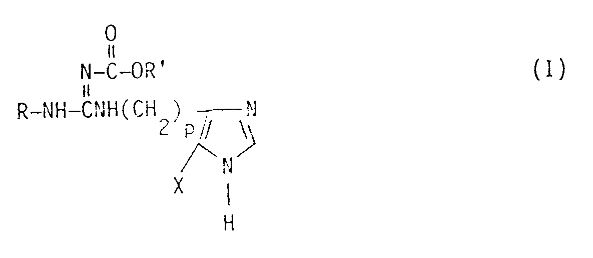 in der
(a) R die Gruppierung 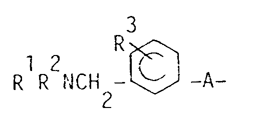 bedeutet, wobei R¹ und R², die gleich oder verschieden sein können, jeweils für Wasserstoff, lineares C₁-C₆-Alkyl oder C₅-C₆-Cycloalkyl stehen oder R¹ und R² zusammen mit dem Stickstoffatom, an das sie gebunden sind, einen 5- bis 10gliedrigen stickstoffhaltigen alicyclischen, heterocyclischen Ring bilden, R³ für ein Wasserstoffatom, ein Halogenatom oder eine C₁-C₃-Alkoxygruppe steht, A die Gruppierung -0-(CH₂)ₖ-, -O-CH₂CH(OH)CH₂-, -O-CH₂CH(CH₃)-CH₂-,-CH₂-O-CH₂-CH-(OH)-CH₂- oder -O-CH₂-CH(OH)-CH(OH)-CH₂ bedeutet, worin k den Wert 3 oder 4 hat, oder
(b) R die Gruppierung 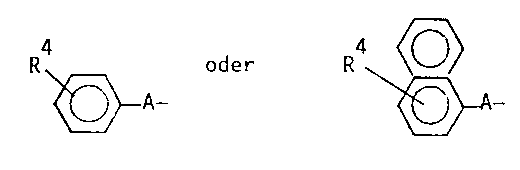 bedeutet, wobei R⁴ für ein Wasserstoffatom, ein vorzugsweise in para-Stellung zu A gebundenes Halogenatom, eine C₁-C₃-Alkoxy- oder C₁-C₃-Alkylgruppe steht und A die oben unter (a) genannte Bedeutung besitzt, oder
(c) R die Gruppierung 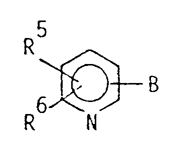 bedeutet, wobei R⁵ und R⁶, die gleich oder verschieden sein können, jeweils für ein Wasserstoffatom, ein Halogenatom oder eine lineare C₁-C₃-Alkyl- oder eine lineare C₁-C₃-Alkoxygruppe stehen, B in Position 2, 3 oder 4 des Pyridinrings gebunden sein kann und die Gruppierung 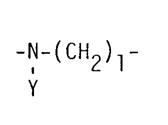 oder -(CH₂)ₘ- bedeutet, wobei I den Wert 2, 3 oder 4 und m den Wert 3, 4 oder 5 haben und Y für ein Wasserstoffatom oder für eine lineare C₁-C₃-Alkylgruppe steht, oder
(d) R die Gruppierung 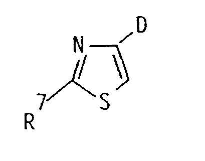 bedeutet, wobei R⁷ für die Gruppe (R¹R²)N-CH₂-, (H₂N)₂C = N-, 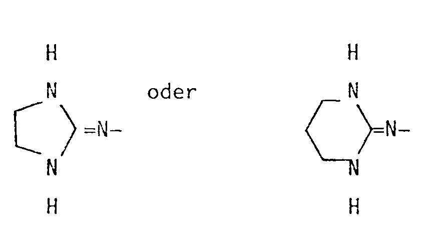 steht, wobei R¹ und R² die oben unter (a) angegebene Bedeutung besitzen, D für die Gruppierung -CH₂-S-(CH₂)ₙ- oder -(CH₂)ₒ- steht, wobei n den Wert 2 oder 3 und o den Wert 2, 3 oder 4 haben, oder
(e) R die Gruppierung 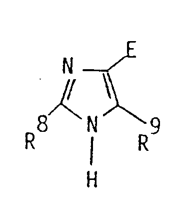 bedeutet, wobei R⁸ für ein Wasserstoffatom, eine gegebenenfalls durch ein Halogenatom substituierte Benzylgruppe, die Gruppierung (R¹R²)N-CH₂- oder eine Aminogruppe steht, wobei R¹ und R² die oben unter (a) genannten Bedeutungen haben, R⁹ ein Wasserstoffatom, eine lineare C₁-C₃-Alkyl-oder C₁-C₃-Alkylthiogruppe bedeutet, E für die Gruppierung 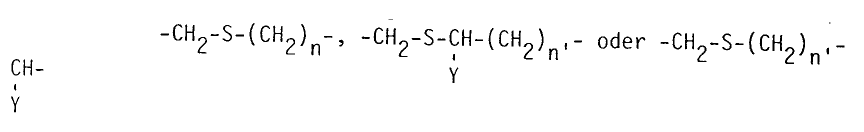 steht, wobei n den Wert 2 oder 3 und n' den Wert 1, 2 oder 3 hat und Y die oben unter (c) angegebene Bedeutung besitzt, oder
(f) R die Gruppierung 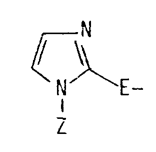 bedeutet, wobei Z ein Wasserstoffatom oder eine lineare C₁-C₃-Alkylgruppe bedeutet und E die oben unter (e) genannte Bedeutung besitzt, oder
(g) R die Gruppierung R"-A'-B'- bedeutet, wobei R" für eine unsubstituierte oder mit Halogen, linearem C₁-C₃-Alkyl oder linearem C₁-C₃-Alkoxy substituierte Phenylgruppe oder eine unsubstituierte oder mit Halogen, linearem C₁-C₃-Alkyl oder linearem C₁-C₃-Alkoxy substituierte Naphthylgruppe steht, A' für eine Einfachbindung oder für die Gruppierung -CR^{1'}R^{2'} oder für ein - mit einer Phenyl-, Naphthyl-, Benzyl-, Pyridinyl-, Furanyl- oder Thiophenylgruppe, die gegebenenfalls mit Halogen, linearem C1-C3-Alkyl oder linearem C₁-C₃-Alkoxy substituiert ist, oder linearen Ci-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Pyridinyl-, Furanyl- oder Thiophenylgruppe, die gegebenenfalls mit Halogen, linearem C₁-C₃-Alkyl oder linearem C₁-C₃-Alkoxy substituiert ist, steht, B' für die Gruppierung -CH(Y)-S-(CH2)m-, -CH₂-S-CH₂-CH(Y)-CH₂-, -CH₂-S-CH(Y)-CH₂-, -CH₂-S-CH₂-CH(Y)-, -(CH₂)_{n"}- -(CH2)-CH(Y)-, -(CH2)_{n"}-CH(Y), -O-(CH₂)₂-, -CH₂-O-(CH₂)_{o'}-, -CH₂-O-CH₂-CH(Y)-CH₂-, -O-CH₂-CH(Y)-, -O-CH(Y)-CH₂-, -S-(CH₂)q-, -S-CH₂-CH(Y)-, -S-CH(Y)-CH₂ oder -S-CH₂-CH(Y)-CH₂- steht, wobei Y ein Wasserstoffatom oder eine lineare C₁-C₃-Alkylgruppe bedeutet, m' und o' jeweils den Wert 2 oder 3 haben, n" und q jeweils den Wert 2, 3, 4 oder 5 haben, oder
(h) R die Gruppierung R"'-A"-B"- bedeutet, wobei R"' für eine unsubstituierte oder mit Halogen, linearem C₁-C₃-Alkyl oder linearem C₁-C₃-Alkoxy substituierte Pyridinyl-, Imidazolyl-, Pyrimidinyl-, Thiophenyl-, Furanyl-, Benzimidazolyl- oder Chinolinylgruppe steht, an die gegebenenfalls ein Phenylring ankondensiert sein kann, A" für eine Einfachbindung oder für die Gruppierung -CR^{1'}R^{2'} oder für ein - mit einer Phenyl-, Naphthyl-, Benzyl-, Pyridinyl-, Benzimidazolyl-, Thiophenyl- oder Furanylgruppe, die gegebenenfalls mit Halogen, linearem C₁-C₃-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Benzimidazolyl-, Pyridinyl-, Thiophenyl- oder Furanylgruppe, die gegebenenfalls mit Halogen, linearem C₁-C₃-Alkyl oder linearem C₁-C₃-Alkoxy substituiert ist, steht, B" für die Gruppierung -CH(Y)-S-(CH2)m-, -CH₂-S-CH₂-CH(Y)-CH₂-, -CH₂-S-CH(Y)-CH₂-, -(CH2)_{n"}-CH(Y)-, -CH₂-S-CH₂-CH(Y)-, -(CH₂)-n -O(CH₂)_{n"}-, -S-CH2-CH(Y)-, S-CH(Y)-CH₂-, -S-(CH₂)q- oder -S-CH₂-CH(Y)-CH₂- steht, wobei Y ein Wasserstoffatom oder eine lineare C₁-C₃-Alkylgruppe bedeutet, m' den Wert 2 oder 3 hat und n" und q den Wert 2, 3, 4 oder 5 haben,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet, sowie der physiologisch annehmbaren Salze davon,
dadurch gekennzeichnet, daß man
(1) eine Verbindung der allgemeinen Formel II 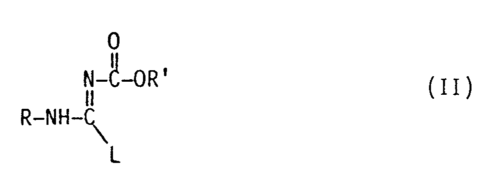 in der R und R' die oben angegebenen Bedeutungen besitzen und L für eine Austrittsgruppe steht, mit einer Verbindung der allgemeinen Formel III 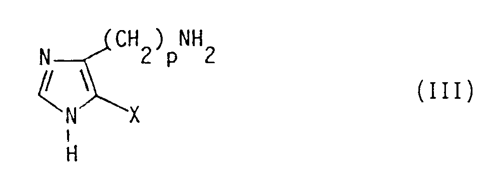 in der X und p wie oben definiert sind, zu einer Verbindung der allgemeinen Formel I umsetzt oder
(2) eine Verbindung der allgemeinen Formel IV 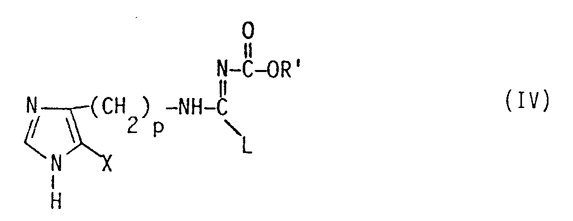 in der R', X und p die oben angegebenen Bedeutungen besitzen und L für eine Austrittsgruppe steht, mit einer Verbindung der allgemeinen Formel V 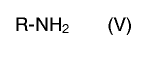 in der R die oben angegebene Bedeutung besitzt, zu einer Verbindung der allgemeinen Formel I umsetzt
und daß man gegebenenfalls die nach (1) oder (2) erhaltenen Verbindungen in an sich bekannter Weise in ihr physiologisch annehmbares Salz umwandelt.

2. Verfahren nach Anspruch 1 zur Herstellung von Verbindungen, bei denen R für die Gruppierung 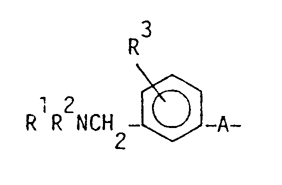 steht, R¹ und R² zusammen mit dem Stickstoffatom einen Pyrrolidin- oder Piperidinring bilden, R³ für ein Wasserstoffatom steht, A die Gruppierung -O(CH₂)ₖ-, OCH₂CH(OH)CH₂- oder OCH₂CH(CH₃)CH₂- bedeutet, R' eine lineare oder verzweigte, gegebenenfalls ein- oder mehrfach mit einem Halogenatom, einer C₁-C₃-Alkoxygruppe und/oder einem Phenyl- oder Naphthylrest substituierte Ci-C6-Alkyl- oder Cycloalkylgruppe bedeutet, k und p den Wert 3 haben und X für ein Wasserstoffatom steht.

3. Verfahren nach Anspruch 1 zur Herstellung von Verbindungen, bei denen R für die Gruppierung 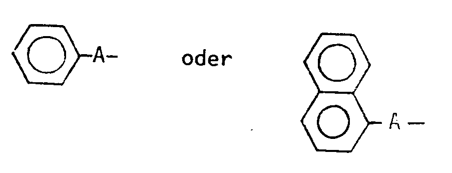 steht, wobei A die Gruppierung -0-(CH₂)ₖ-, -O-CH₂CH(OH)CH₂-, -O-CH₂CH(CH₃)-CH₂-, -CH₂-O-CH₂-CH(OH)CH₂- oder -O-CH₂-CH(OH)-CH(OH)-CH₂ bedeutet,
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

4. Verfahren nach Anspruch 1 zur Herstellung von Verbindungen, bei denen R für die Gruppierung 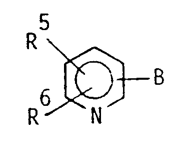 steht, wobei R⁵ für ein in 5-Stellung des Pyridinrings gebundenes Halogenatom oder Wasserstoffatom steht, R⁶ für ein in 3-Stellung des Pyridinrings gebundenes Wasserstoffatom, eine Methyl- oder eine Methoxygruppe steht, B in Position 2, 3 oder 4 des Pyridinrings gebunden sein kann und die Gruppierung 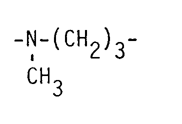 oder -(CH₂)₃-₄- oder -NH-(CH₂), bedeutet, wobei I den Wert 2, 3 oder 4 hat,
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

5. Verfahren nach Anspruch 1 zur Herstellung von Verbindungen, bei denen R für die Gruppierung 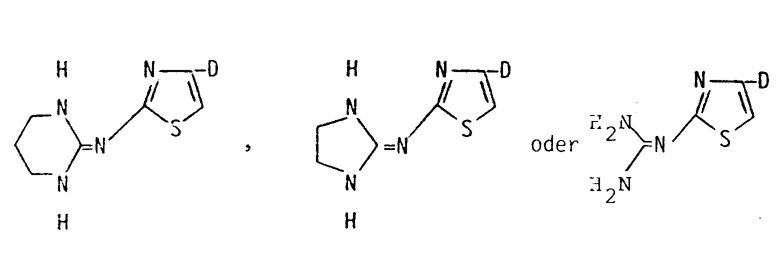 steht, D die Gruppierung -CH₂-S-(CH₂)₂- oder -(CH₂)₃- oder -(CH₂)₄-bedeutet und
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

6. Verfahren nach Anspruch 1 zur Herstellung von Verbindungen, bei denen R für die Gruppierung 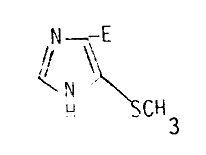 steht, wobei E die Gruppierung -CH₂-S-(CH₂)₂-, 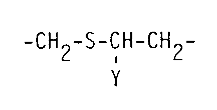 oder 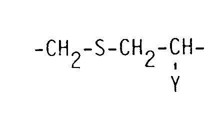 bedeutet, Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl oder Cycloalkylgruppe bedeutet, p den Wert 2 oder 3 hat und X ein Wasserstoffatom oder eine Methylgruppe bedeutet.

7. Verfahren nach Anspruch 1 zur Herstellung von Verbindungen, bei denen R für die Gruppierung 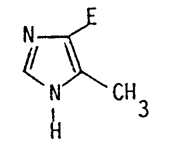 steht, E die Gruppierung -CH₂-S-(CH₂)₂-, 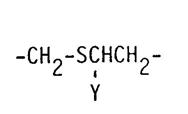 oder 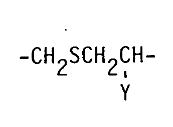 bedeutet, wobei Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

8. Verfahren nach Anspruch 1 zur Herstellung von Verbindungen, bei denen R für die Gruppierung 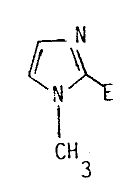 steht, E für die Gruppierung 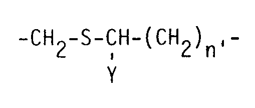 oder 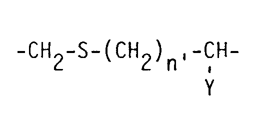 steht, wobei n' den Wert 1, 2 oder 3 hat und Y für ein Wasserstoffatom oder für eine lineare Cₗ-C₃-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

9. Verfahren nach Anspruch 1 zur Herstellung von Verbindungen, bei denen R die Gruppierung R"-A'-B'- bedeutet, wobei R" für eine unsubstituierte Phenylgruppe steht, A' eine Einfachbindung bedeutet, B' für die Gruppierung -CH₂-S-(CH₂)_{m'}- oder -CH₂-S-CH₂-CH(Y)-CH₂-steht, wobei m' den Wert 2 oder 3 hat und Y für ein Wasserstoffatom oder eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

10. Verfahren nach Anspruch 1 zur Herstellung von Verbindungen, bei denen R die Gruppierung R"-A'-B'- bedeutet, wobei R" für eine unsubstituierte oder mit Halogen, linearem C1-C3-Alkyl oder linearem Ci-C₃-Alkoxy substituierte Phenylgruppe steht, A' ein mit einer Phenyl-, Naphthyl-, Pyridinyl-, Furanyl oder Thiophenylgruppe substituiertes Stickstoffatom bedeutet, B' für die Gruppierung -(CH₂)_{n"} - steht, wobei n" den Wert 2 oder 3 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

11. Verfahren nach Anspruch 1 zur Herstellung von Verbindungen, bei denen R die Gruppierung R"-A'-B'- bedeutet, wobei R" für eine unsubstituierte oder mit Halogen, linearem C₁-C₃-Alkyl oder linearem Ci-C₃-Alkoxy substituierte Phenylgruppe steht, A' für die Gruppierung -CR^{1'}R^{2'}- steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Pyridinyl-, Furanyl oder Thiophenylgruppe, die gegebenenfalls mit Halogen, linearem C₁-C₃-Alkyl oder linearem Cₗ-C₃-Alkoxy substituiert ist, steht, B' die Gruppierung -(CH₂)_{n"}- bedeutet, wobei n" den Wert 2, 3 oder 4 hat, R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

12. Verfahren nach Anspruch 1 zur Herstellung von Verbindungen, bei denen R die Gruppierung R"'-A"-B"- bedeutet, wobei R"' für einen unsubstituierten oder mit Halogen, linearem C₁-C₃-Alkyl oder linearem C₁-C₃-Alkoxy substituierten Pyridinring steht, A" für eine Einfachbindung in Position 2, 3 oder 4 des Pyridinrings und B" für die Gruppierung -CH₂-S-(CH₂)_{m'}- oder -CH₂-S-CH₂-CH(Y)-CH₂- steht, wobei m' den Wert 2 oder 3 hat und Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

13. Verfahren nach Anspruch 1 zur Herstellung von Verbindungen, bei denen R die Gruppierung R"'-A"-B"- bedeutet, wobei R"' für einen unsubstituierten oder mit Halogen, linearem C₁-C₃-Alkyl oder linearem C₁-C₃-Alkoxy substituierten Thiophenring steht, A" eine Einfachbindung bedeutet, B" für die Gruppierung -CH₂-S-(CH₂)_{m'}- oder -CH₂-S-CH₂-CH(Y)-CH₂- steht, wobei m' den Wert 2 oder 3 hat und Y für ein Wasserstoffatom oder für eine lineare C1-C3-Alkylgruppe steht,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

14. Verfahren nach Anspruch 1 zur Herstellung von Verbindungen, bei denen R die Gruppierung R"'-A"-B"- bedeutet, wobei R"' für einen unsubstituierten oder mit Halogen, linearem C₁-C₃-Alkyl oder linearem C₁-C₃-Alkoxy substituierten Pyridinring steht, A" die Gruppierung -CR^{1'}R^{2'} bedeutet, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Pyridinyl-, Furanyl, Thiophenyl- oder Benzimidazolylgruppe, die gegebenenfalls mit Halogen, linearem C₁-C₃-Alkyl oder linearem C₁-C₃-Alkoxy substituiert ist, steht, B" für die Gruppierung -(CH₂)_{n"}- steht, wobei n" den Wert 2, 3, 4 oder 5 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

15. Verfahren nach Anspruch 1 zur Herstellung von Verbindungen, bei denen R die Gruppierung R"'-A"-B" bedeutet, wobei R"' für einen unsubstituierten oder mit Halogen, linearem C₁-C₃-Alkyl oder linearem C₁-C₃-Alkoxy substituierten Pyridinring steht, A" für eine Einfachbindung in Position 2, 3 oder 4 des Pyridinrings steht, B" für die Gruppierung -(CH₂)_{n"}- steht, wobei n" den Wert 2, 3, 4 oder 5 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

16. Verfahren nach Anspruch 1 zur Herstellung von Verbindungen, bei denen R die Gruppierung R"'-A"-B"- bedeutet, wobei R"' für einen unsubstituierten oder mit Halogen, linearem C₁-C₃-Alkyl oder linearem C₁-C₃-Alkoxy substituierten Pyridinring steht, A" für eine Einfachbindung oder für die Gruppierung -CR^{1'}R^{2'} oder für ein - mit einer Phenyl-, Naphthyl-, Benzyl-, Pyridinyl-, Benzimidazolyl-, Thiophenyl- oder Furanylgruppe, die gegebenenfalls mit Halogen, linearem C₁-C₃-Alkyl oder linearem C₁-C₃-Alkoxy substituiert ist, oder linearen C₁-C₃-Alkylgruppe substituiertes - Stickstoffatom steht, wobei R^{1'} ein Wasserstoffatom oder eine Methylgruppe bedeutet, R für eine Phenylgruppe, die gegebenenfalls mit Halogen, linearem C₁-C₃-Alkyl oder linearem C₁-C₃-Alkoxy substituiert ist, oder eine Benzimidazolyl-, Pyridinyl-, Thiophenyl- oder Furanylgruppe, die gegebenenfalls mit Halogen, linearem C₁-C₃-Alkyl oder linearem C₁-C₃-Alkoxy substituiert ist, steht, B" für die Gruppierung -(CH₂)_{n"}- steht, wobei n" den Wert 2, 3, 4 oder 5 hat,
R' eine lineare oder verzweigte, gegebenenfalls ein- oder mehrfach mit einem Halogenatom, einer C₁-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

17. Verfahren nach Anspruch 1 zur Herstellung von Verbindungen, bei denen R die Gruppierung R"'-A"-B"- bedeutet, wobei R"' für die Gruppierung 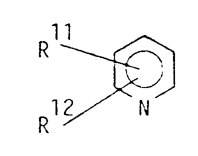 steht, wobei R¹¹ und R¹² unabhängig voneinander jeweils ein Halogenatom, eine lineare Cₗ-C₃-Alkylgruppe oder eine lineare C₁-C₃-Alkoxygruppe bedeuten, A" für die Gruppierung -CR^{1'}R^{2'} steht, wobei R^{1'} ein Wasserstoffatom oder eine Methylgruppe und R eine unsubstituierte oder eine ein- bis dreifach mit Halogen, linearem C₁-C₃-Alkyl oder linearem C₁-C₃-Alkoxy substituierte Phenylgruppe bedeuten, oder A" für ein Stickstoffatom , das mit einer Phenyl-, Naphthyl-, Pyridinyl-, Furanyl-, Thiophenyl- oder Benzimidazolylgruppe oder einem Wasserstoffatom substituiert ist, steht, B", wenn A" die Gruppierung -CR^{1'}R^{2'} ist, für die Gruppierung -(CH₂)ₙ-, -O(CH₂)₂- oder -SCH₂CH₂-steht und B", wenn A" ein mit einer Phenyl-, Naphthyl-, Pyridinyl-, Furanyl-, Thiophenyl- oder Benzimidazolylgruppe oder einem Wasserstoffatom substituiertes Stickstoffatom ist, für die Gruppierung -(CH₂)ₙ- steht, wobei n den Wert 2 oder 3 hat, und
R' eine lineare oder verzweigte, gegebenenfalls ein- oder mehrfach mit einem Halogenatom einer Ci - C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

18. Verfahren nach Anspruch 1 zur Herstellung von Verbindungen, bei denen R die Gruppierung R"'-A"-B"- bedeutet, wobei R"' für die Gruppierung 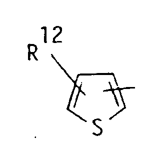 steht, worin R¹² ein Wasserstoffatom, ein Halogenatom, eine lineare C₁-C₃-Alkylgruppe, die Gruppierung (CH₃)₂-NCH₂- oder 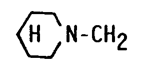 bedeutet, A" für die Gruppierung -CR^{1'}R^{2'} steht, wobei R^{1'} ein Wasserstoffatom oder eine Methylgruppe und R eine unsubstituierte oder eine ein- bis dreifach mit Halogen, linearem C₁-C₃-Alkyl oder linearem C₁-C₃-Alkoxy substituierte Phenylgruppe bedeuten, oder A" für ein Stickstoffatom, das mit einer Phenyl-, Naphthyl-, Pyridinyl-, Fura nyl-, Thiophenyl- oder Benzimidazolylgruppe oder einem Wasserstoffatom substituiert ist, steht, B" für die Gruppierung -CH₂-S-CH₂-CH₂-, -CH₂-S-CH₂-CH(CH₃)-CH₂-, -CH₂-S-CH(CH₃)-CH₂- oder -CH₂-S-CH₂-CH(CH₃)-steht, und
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

19. Verfahren nach Anspruch 1 zur Herstellung von Verbindungen, bei denen R die Gruppierung R"'-A"-B"- bedeutet, wobei R"' für die Gruppierung 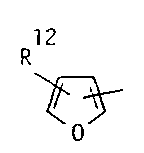 steht, worin R¹² ein Wasserstoffatom, ein Halogenatom, eine lineare C₁-C₃-Alkylgruppe, die Gruppierung (CH₃)₂-NCH₂- oder 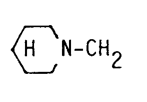 bedeutet, A" eine Einfachbindung in Position 2 bedeutet, B" für die Gruppierung -CH₂-S-CH₂-CH₂-, -CH₂-S-CH₂-CH(CH₃)-CH₂-, -CH₂-S-CH(CH₃)-CH₂- oder -CH₂-S-CH₂-CH(CH₃) steht, und
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

20. Verfahren nach Anspruch 1 zur Herstellung von Verbindungen, bei denen R die Gruppierung R"'-A"-B"- bedeutet, wobei R"' die Gruppierung 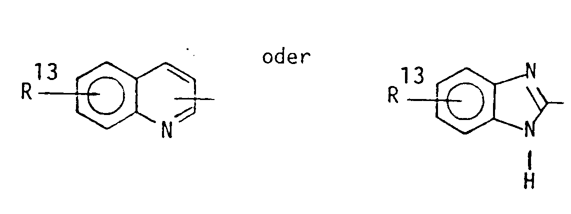 bedeutet, worin R¹³ ein Wasserstoffatom, ein Halogenatom, eine lineare C₁-C₃-Alkylgruppe, die Gruppierung (CH₃)₂-NCH₂- oder 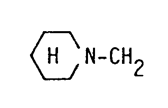 bedeutet, A" eine Einfachbindung bedeutet, B" für die Gruppierung -CH₂-S-CH₂-CH₂-, -CH₂-S-CH₂-CH(CH₃)-CH₂-, -CH₂-S-CH(CH₃)-CH₂ oder -CH₂-S-CH₂-CH(CH₃) steht, und
R' eine lineare oder verzweigte, gegebenenfalls ein- oder mehrfach mit einem Halogenatom, einer Ci-C₃-Alkoxygruppe und/oder einem Phenyl- oder Naphthylrest substituierte C₁-C₆-Alkyl- oder Cycloalkylgruppe bedeutet, p den Wert 2 oder 3 hat und X ein Wasserstoffatom oder eine Methylgruppe bedeutet.

21. Verfahren nach Anspruch 1 zur Herstellung von N¹-[3-(Imidazol-4-yl)propyl]-N²-[4-(pyridin-2-yl)butyl]-N³- methoxycarbonylguanidin und den physiologisch annehmbaren Salzen.

22. Verfahren nach Anspruch 1 zur Herstellung von N¹-[3-(Imidazol-4-yl)propyl]-N²-[4-(pyridin-2-yl)butyl]-N³- ethoxycarbonylguanidin und den physiologisch annehmbaren Salzen.

23. Verfahren nach Anspruch 1 zur Herstellung von N¹-[3-(Imidazol-4-yl)propyl]-N²-[3-(4-fluorphenyl)-3-(pyridin-2-yl)propyl]-N³⁻methoxycarbonylguanidin und den physiologisch annehmbaren Salzen.

24. Verfahren nach Anspruch 1 zur Herstellung von Nⁱ⁻[3-(lmidazol-4-yl )propyl]-N²⁻[3-(4-fluorphenyl)-3-(pyridin-2-yl)propyl]-N³⁻ethoxycarbonylguanidin und den physiologisch annehmbaren Salzen.

25. Verfahren nach Anspruch 1 zur Herstellung von N¹-[3-(Imidazol-4-yl)propyl]-N²-[3-(4-fluorphenyl)-3-(pyridin-2-yl)propyl]-N³⁻butoxycarbonylguanidin und den physiologisch annehmbaren Salzen.

## Claims (Claims for the following Contracting State(s) : AT, BE, CH, DE, FR, GB, IT, LI, LU, NL, SE)

1. Guanidine carboxylic acid esters corresponding to general formula I 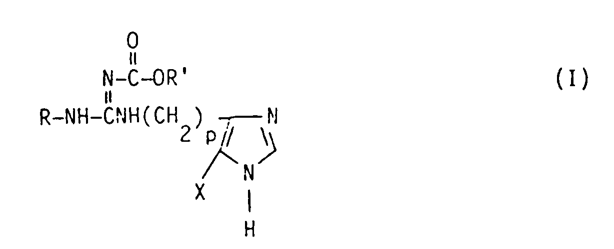 in which
(a) R represents the group 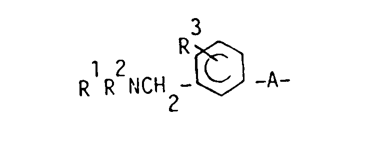 where R¹ and R² may be the same or different and represent hydrogen, linear C₁₋₆ alkyl or C₅₋₆ cycloalkyl or R¹ and R² together with the nitrogen atoms to which they are attached form a 5- to 10- membered, nitrogen-containing, alicyclic heterocyclic ring, R³ is a hydrogen atom, a halogen atom or a C₁₋₃ alkoxy group, A represents the group -O-(CH₂)ₖ-, -O-CH₂CH(OH)CH₂-, -0-CH2CH(CH3)-CH2-, -CH₂-O-CH₂-CH(OH)-CH₂- or -0-CH₂-CH(OH)-CH(OH)-CH₂, k having the value 3 or 4, or
(b) R represents the group 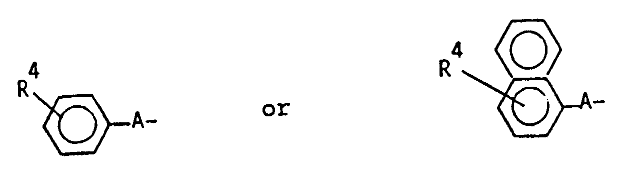 where R⁴ is a hydrogen atom, a halogen atom attached to A preferably in the para position, a C₁ -₃ alkoxy group or a C₁ -₃ alkyl group and A is as defined above under (a), or
(c) R represents the group 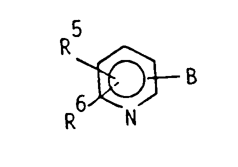 where R⁵ and R⁶ may be the same or different and represent a hydrogen atom, a halogen atom or a linear C₁ -₃ alkyl group or a linear C₁ -₃ alkoxy group, B may be attached in position 2, 3 or 4 of the pyridine ring and represents the group 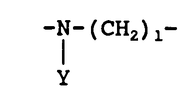 or -(CH2)m-, I having the value 2, 3 or 4 and m having the value 3, 4 or 5 and Y being a hydrogen atom or a linear C₁ -₃ alkyl group or
(d) R represents the group 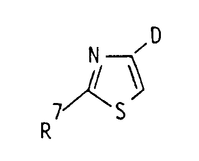 where R⁷ represents the group (R¹R²)N-CH₂-, (H₂N)₂ C = N-, 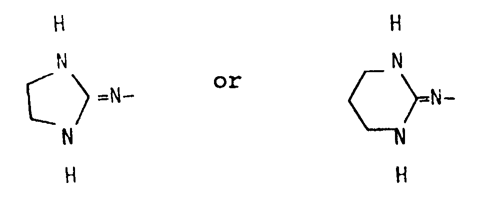 R¹ and R² being as defined above in (a), D represents the group -CH₂-S-(CH₂)ₙ- or -(CH₂)ₒ-, n having the value 2 or 3 and o having the value 2, 3 or 4, or
(e) R represents the group 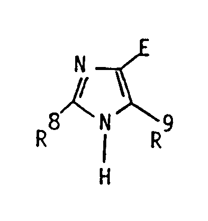 in which R⁸ is a hydrogen atom, an optionally halogen-substituted benzyl group, the group (R¹R²)N-CH₂- or an amino group, R¹ and R² being as defined above in (a), R⁹ is a hydrogen atom, a linear C₁₋₃ alkyl or C₁₋₃ alkylthio group, E represents the group -CH₂-S-(CH₂)ₙ-, 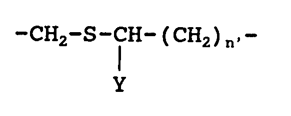 or 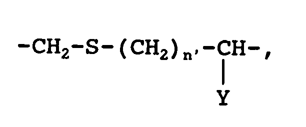 n having the value 2 or 3 and n' having the value 1, 2 or 3 and Y being as defined above in (c), or
(f) R represents the group 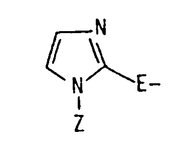 where Z is a hydrogen atom or a linear C₁ -₃ alkyl group and E is as defined above in (e), or
(g) R represents the group R"-A'-B'-, where R" is an unsubstituted or halogen-, linear C₁ -₃ alkyl- or linear C₁ -₃ alkoxy-substituted phenyl group or an unsubstituted or halogen-, linear C₁ -₃ alkyl- or linear C₁₋₃ alkoxy-substituted naphthyl group, A' is a single bond or represents the group -CR^{1'}R^{2'} or a nitrogen atom substituted by a phenyl, naphthyl, benzyl, pyridinyl, furanyl or thiophenyl group - optionally substituted by halogen, linear C₁ -₃ alkyl or linear C₁ -₃ alkoxy - or by a linear C₁ -₃ alkyl group, R^{1'} being a hydrogen atom or a methyl group, R^{2'} being a phenyl group optionally substituted by halogen, linear C₁ -₃ alkyl or linear C₁ -₃ alkoxy or a pyridinyl, furanyl or thiophenyl group optionally substituted by halogen, linear C₁ -₃ alkyl or linear C₁ -₃ alkoxy, B' represents the group -CH(Y)-S-(CH2)m-, -CH₂-S-CH₂-CH(Y)-CH₂-, -CH₂-S-CH(Y)-CH₂-, -CH₂-S-CH₂-CH(Y)-, -(CH₂)ₙ"-, - (CH₂)-CH(Y)-, -(CH2)ₙ"-CH(Y)-, -O-(CH₂)₂-, -CH₂-O-(CH₂)_{o'}-, -CH₂-O-CH₂-CH(Y)-CH₂-, -O-CH₂-CH(Y)-, -0-CH(Y)-CH₂-, -S-(CH₂)q-, -S-CH₂-CH(Y)-, -S-CH(Y)-CH₂ or -S-CH₂-CH(Y)-CH₂-, Y being a hydrogen atom or a linear C₁ -₃ alkyl group, m' and o' having the value 2 or 3, n" and q each having the value 2, 3, 4 or 5, or
(h) R represents the group R"'-A"-B"-, where R"' is an unsubstituted or halogen-, linear C₁ -₃ alkyl-or linear C₁₋₃ alkoxy-substituted pyridinyl, imidazolyl, pyrimidinyl, thiophenyl, furanyl, benzimidazolyl or quinolyl group, to which a phenyl ring may optionally be fused, A" is a single bond or represents the group -CR^{1'}R^{2'} or a nitrogen atom substituted by a phenyl, naphthyl, benzyl, pyridinyl, benzimidazolyl, thiophenyl or furanyl group - optionally substituted by halogen, linear C₁ -₃ alkyl or linear C₁₋₃ alkoxy - or by a linear C₁₋₃ alkyl group, R^{1'} being a hydrogen atom or a methyl group, R being a phenyl group optionally substituted by halogen, linear C₁ -₃ alkyl or linear C₁ -₃ alkoxy or a benzimidazolyl, pyridinyl, thiophenyl or furanyl group optionally substituted by halogen, linear C₁ -₃ alkyl or linear C₁₋₃ alkoxy, B" represents the group -CH(Y)-S-(CH2)m-, -CH₂-S-CH₂-CH(Y)-CH₂-, -CH₂-S-CH(Y)-CH₂-, -(CH₂)_{n"} CH(Y)-, -CH₂-S-CH₂-CH(Y)-, -(CH₂)ₙ"- -, -O(CH₂)_{n"}-, -S-CH2-CH(Y)-, -S-CH(Y)-CH₂-, -S-(CH₂)q-, or -S-CH₂-CH(Y)-CH₂-, Y representing a hydrogen atom or a linear C₁-₃ alkyl group, m' having the value 2 or 3 and n" and q having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group,
and physiologically acceptable salts thereof.

2. Guanidine carboxylic acid esters as claimed in claim 1, characterized in that R represents the group 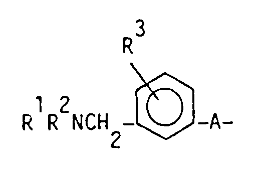 where R¹ and R² together with the nitrogen atom form a pyrrolidine or piperidine ring, R³ represents a hydrogen atom, A is the group -O(CH₂)ₖ, OCH₂CH(OH)CH₂- or OCH₂CH(CH₃)-CH₂-,
R' is a linear or branched C₁ -₆ alkyl or cycloalkyl group optionally substituted one or times by a halogen atom, a C₁ -₃ alkoxy group and/or a phenyl or naphthyl radical, k and p having the value 3 and X is a hydrogen atom.

3. Guanidine carboxylic acid esters as claimed in claim 1, characterized in that R represents the group 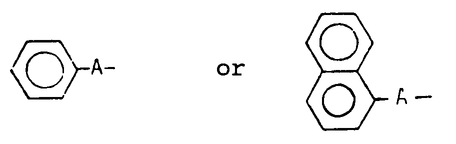 where A is the group -0-(CH₂)ₖ-, -O-CH₂CH(OH)CH₂-, -0-CH₂CH(CH₃)-CH₂-, -CH₂-0-CH₂-CH(OH)-CH₂- or -0-CH₂-CH(OH)-CH(OH)-CH₂,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

4. Guanidine carboxylic acid esters as claimed in claim 1, characterized in that R represents the group 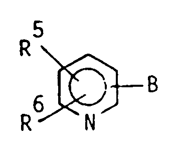 where R⁵ is a halogen atom or hydrogen atom attached in the 5 position of the pyridine ring, R⁶ is a hydrogen atom attached in the 3 position of the pyridine ring, a methyl group or a methoxy group, B may be attached in position 2, 3 or 4 of the pyridine ring and represents the group 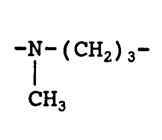 or -(CH₂)₃-₄- or -NH-(CH₂)₁, I having the value 2, 3 or 4, R' is a linear or branched C₁-₆ alkyl or cycloalkyl group optionally substituted one or more times by a halogen atom, a Cᵢ -₃ alkoxy group and/or a phenyl or naphthyl radical, p has the value 2 or 3 and X is a hydrogen atom or a methyl group.

5. Guanidine carboxylic acid esters as claimed in claim 1, characterized in that R represents the group 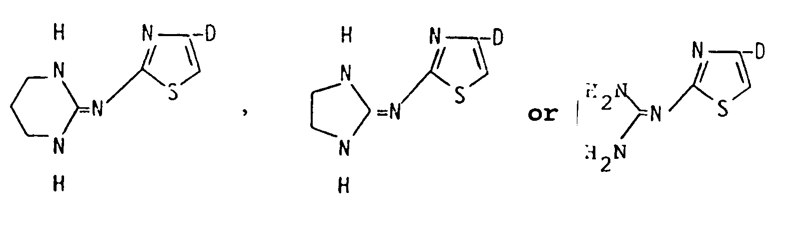 where D is the group -CH₂-S-(CH₂)₂- or -(CH₂)₃- or -(CH₂)₄-and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

6. Guanidine carboxylic acid esters as claimed in claim 1, characterized in that R represents the group 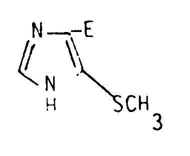 where E is the group -CH₂-S-(CH₂)₂-, 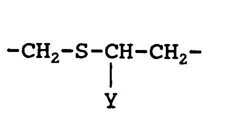 or 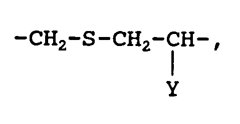 Y representing a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

7. Guanidine carboxylic acid esters as claimed in claim 1, characterized in that R represents the group 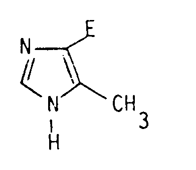 where E is the group -CH₂-S-(CH₂)₂-, 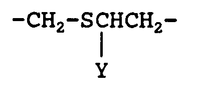 or 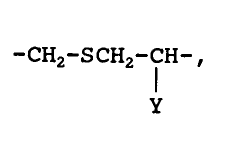 Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

8. Guanidine carboxylic acid esters as claimed in claim 1, characterized in that R represents the group 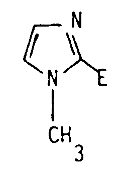 where E is the group 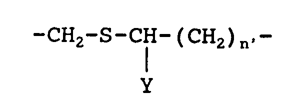 or 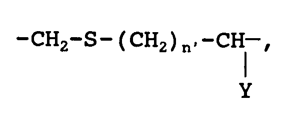 n' having the value 1, 2 or 3 and Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

9. Guanidine carboxylic acid esters as claimed in claim 1, characterized in that R represents the group R"-A'-B'-, where R" is an unsubstituted phenyl group, A' is a single bond, B'is the group -CH₂-S-(CH₂)-ₘ- or -CH₂-S-CH₂-CH(Y)-CH₂-, m' having the value 2 or 3 and Y representing a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

10. Guanidine carboxylic acid esters as claimed in claim 1, characterized in that R represents the group R"-A'-B'-, where R" is an unsubstituted or halogen-, linear C₁ -₃ alkyl-or linear C₁ -₃ alkoxy-substituted phenyl group, A' is a nitrogen atom substituted by a phenyl, naphthyl, pyridinyl, furanyl or thiophenyl group, B' represents the group - (CH₂)_{n"}-, n" having the value 2 or 3,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

11. Guanidine carboxylic acid esters as claimed in claim 1, characterized in that R represents the group R"-A'-B'-, where R" is an unsubstituted or halogen-, linear C₁ -₃ alkyl-or linear C₁ -₃ alkoxy-substituted phenyl group, A' represents the group -CR^{1'}R^{2'}-, R^{1'} being a hydrogen atom or a methyl group, R being a phenyl group optionally substituted by halogen, linear Cᵢ -₃ alkyl or linear Ci -₃ alkoxy or a pyridinyl, furanyl or thiophenyl group optionally substituted by halogen, linear C₁ -₃ alkyl or linear C₁ -₃ alkoxy, B' represents the group -(CH₂)_{n"}-, n" having the value 2, 3 or 4,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

12. Guanidine carboxylic acid esters as claimed in claim 1, characterized in that R represents the group R"'-A"-B"-where R"' is an unsubstituted or halogen-, linear C₁ -₃ alkyl-or linear C₁ -₃ alkoxy-substituted pyridine ring, A" is a single bond in position 2, 3 or 4 of the pyridine ring and B" is the group -CH₂-S-(CH₂)ₙ,- or -CH₂-S-CH₂-CH(Y)-CH₂-, m' having the value 2 or 3 and Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

13. Guanidine carboxylic acid esters as claimed in claim 1, characterized in that R represents the group R"'-A"-B"-where R"' is an unsubstituted or halogen-, linear C₁ -₃ alkyl-or linear C₁ -₃ alkoxy-substituted thiophene ring, A" is a single bond, B" is the group -CH₂-S-(CH₂)_{m'}- or -CH₂-S-CH₂-CH(Y)-CH₂-, m' having the value 2 or 3 and Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

14. Guanidine carboxylic acid esters as claimed in claim 1, characterized in that R represents the group R"'-A"-B"-where R"' is an unsubstituted or halogen-, linear C₁ -₃ alkyl-or linear C₁ -₃ alkoxy-substituted pyridine ring, A" is the group CR^{1'}R^{2'}, R^{1'} being a hydrogen atom or methyl group and R being a phenyl group optionally substituted by halogen, linear Cᵢ -₃ alkyl or linear C₁₋₃ alkoxy or a pyridinyl, furanyl, thiophenyl or benzimidazolyl group optionally substituted by halogen, linear C₁ -₃ alkyl or linear Ci -₃ alkoxy, B" is the group -(CH₂)ₙ"-, n" having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

15. Guanidine carboxylic acid esters as claimed in claim 1, characterized in that R represents the group R"'-A"-B", where R"' is an unsubstituted or halogen-, linear C₁₋₃ alkyl-or linear C₁₋ alkoxy-substituted pyridine ring, A" is single bond in position 2, 3 or 4 of the pyridine ring and B" is the group -(CH₂)ₙ"-, n" having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

16. Guanidine carboxylic acid esters as claimed in claim 1, characterized in that R represents the group R"'-A"-B"-where R"' is an unsubstituted or halogen-, linear C₁ -₃ alkyl-or linear C₁ -₃ alkoxy-substituted pyridine ring, A" is single bond or the group -CR^{1'}R^{2'} or a nitrogen atom substituted by a phenyl, naphthyl, benzyl, pyridinyl, benzimidazolyl, thiophenyl or furanyl group - optionally substituted by halogen, linear C1-3 alkyl or linear C₁₋₃ alkoxy - or by a linear C₁₋₃ alkyl group, R^{1'} being a nitrogen atom or a methyl group and R being a phenyl group optionally substituted by halogen, linear C₁₋₃ alkyl or linear Cᵢ -₃ alkoxy or a benzimidazolyl, pyridinyl, thiophenyl or furanyl group optionally substituted by halogen, linear C₁₋₃ alkyl or linear C₁₋₃ alkoxy, B" is the group -(CH₂)ₙ"-, n" having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

17. Guanidine carboxylic acid esters as claimed in claim 1, characterized in that R represents the group R"'-A"-B"-where R"' is the group 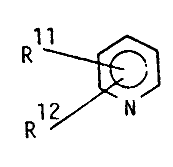 R¹¹ and R¹² independently of one another representing a halogen atom, a linear C₁₋₃ alkyl group or a linear C₁₋₃ alkoxy group, A" is the group CR^{1'}R^{2'}, R^{1'} being a hydrogen alkoxy group, A" is the group CR^{1'}R^{2'}, R^{1'} being a hydrogen atom or a methyl group and R being an unsubstituted phenyl group or a phenyl group substituted once to three times by halogen, linear C₁ -₃ alkyl or linear C₁ -₃ alkoxy, or A" is a nitrogen atom substituted by a phenyl, naphthyl, pyridinyl, furanyl, thiophenyl or benzimidazolyl group or a hydrogen atom, B" represents the group -(CH₂)ₙ-, -O(CH₂)₂- or -SCH₂CH₂-whereA" is the group -CR^{1'}R^{2'} and the group -(CH₂)ₙ- (n = 2 or 3) where A" is a nitrogen atom substituted by a phenyl, naphthyl, pyridinyl, furanyl, thiophenyl or benzimidazolyl group or by a hydrogen atom and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

18. Guanidine carboxylic acid esters as claimed in claim 1, characterized in that R represents the group R"'-A"-B"-where R"' is the group 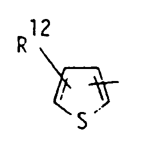 R¹² being a hydrogen atom, a halogen atom, a linear C₁₋₃ alkyl group, the group (CH₃)₂-NCH₂- or 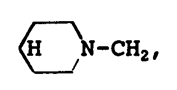 A" is the group -CR^{1'}R^{2'}, R^{1'} being a hydrogen atom or a methyl group and R being an unsubstituted phenyl group or a phenyl group substituted once to three times by halogen, linear C₁ -₃ alkyl or linear C₁ -₃ alkoxy, or A" is a nitrogen atom substituted by a phenyl, naphthyl, pyridinyl, furanyl, thiophenyl or benzimidazolyl group or by a hydrogen atom, B" is the group -CH₂-S-CH₂-CH₂-, -CH₂-S-CH₂-CH(CH₃)-CH₂-, -CH₂-S-CH(CH₃)-CH₂- or -CH₂-S-CH₂-CH(CH₃)- and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

19. Guanidine carboxylic acid esters as claimed in claim 1, characterized in that R represents the group R"'-A"-B"-where R'" is the group 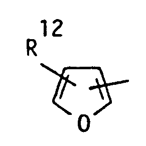 R₁₂ being a hydrogen atom, a halogen atom, a linear a linear C₁₋₃ alkyl group, the group (CH₃)₂-NCH₂- or 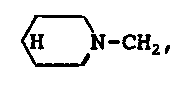 A" is a single bond in position 2, B" is the group -CH₂-S-CH₂-CH₂-, -CH₂-S-CH₂-CH(CH₃) -CH₂-, -CH₂-S-CH(CH₃)-CH₂- or -CH₂-S-CH₂-CH(CH₃)- and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

20. Guanidine carboxylic acid esters as claimed in claim 1, characterized in that R represents the group R"'-A"'B"-where R"' is the group 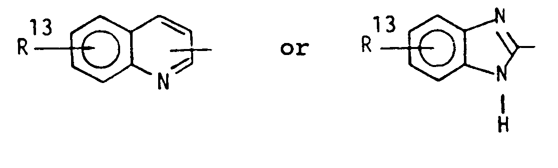 R¹³ being a hydrogen atom, a halogen atom, a linear C₁₋₃ alkyl group, the group (CH₃)₂-NCH₂- or 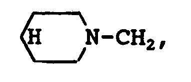 A" is a single bond, B" is the group -CH₂-S-CH₂-CH₂-, -CH₂-S-CH₂-CH(CH₃)-CH₂-,-CH₂-S-CH(CH₃)-CH₂- or -CH₂-S-CH₂-CH(CH₃) and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

21. N'-[3-(imidazol-4-yl)propyl]-N²-[4-pyridin-2-yl)butyl]-N³-methoxycarbonyl guanidine and physiologically acceptable salts thereof.

22. N'-[3-(imidazol-4-yl)propyl]-N²-[4-pyridin-2-yl)butyl]-N³-ethoxycarbonyl guanidine and physiologically acceptable salts thereof.

23. N'-[3-(imidazol-4-yl)propyl]-N²-[3-(4-fluorophenyl)-3-(pyridin-2-yl)propyl]-N³-methoxycarbonyl guanidine and physiologically acceptable salts thereof.

24. N'-[3-(imidazol-4-y)propyl]-N²-[3-(4-fluorophenyl)-3-(pyridin-2-yl)propyl]-N³-ethoxycarbonyl guanidine and physiologically acceptable salts thereof.

25. N'-[3-(imidazol-4-yl)propyl]-N2-[3-(4-fluorophenyl)-3-(pyridin-2-yl)propyl]-N3-butoxycarbonyl guanidine and physiologically acceptable salts thereof.

26. A process for the production of guanidine derivatives corresponding to general formula I 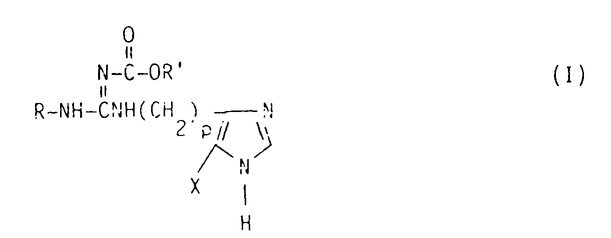 in which
(a) R represents the group 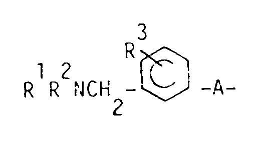 where R¹ and R² may be the same or different and represent hydrogen, linear C₁₋₆ alkyl or Cs-6 cycloalkyl or R¹ and R² together with the nitrogen atoms to which they are attached form a 5- to 10- membered, nitrogen-containing, alicyclic heterocyclic ring, R³ is a hydrogen atom, a halogen atom or a C₁₋₃ alkoxy group, A represents the group -O-(CH₂)ₖ-, -O-CH₂CH(OH)CH₂-, -0-CH2CH(CH3)-CH2-, -CH₂-O-CH₂-CH(OH)-CH₂- or -0-CH₂-CH(OH)-CH(OH)-CH₂, k having the value 3 or 4, or
(b) R represents the group 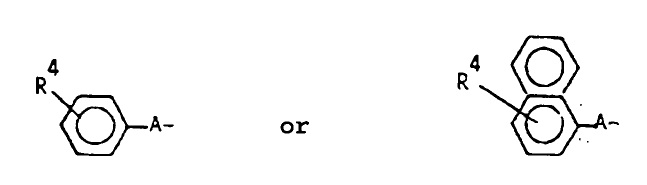 where R⁴ is a hydrogen atom, a halogen atom attached to A preferably in the para position, a C₁ -₃ alkoxy group or a C₁ -₃ alkyl group and A is as defined above under (a), or
(c) R represents the group 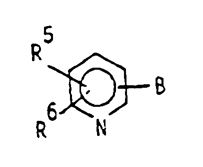 where R⁵ and R⁶ may be the same or different and represent a hydrogen atom, a halogen atom or a linear C₁ -₃ alkyl group or a linear C₁ -₃ alkoxy group, B may be attached in position 2, 3 or 4 of the pyridine ring and represents the group 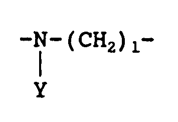 or -CH2)m-, I having the value 2, 3 or 4 and m having the value 3, 4 or 5 and Y being a hydrogen atom or a linear C₁ -₃ alkyl group or
(d) R represents the group 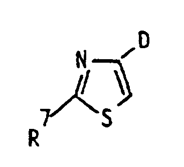 where R⁷ represents the group (R¹R²)N-CH₂-, (H₂N)₂ C =N-, 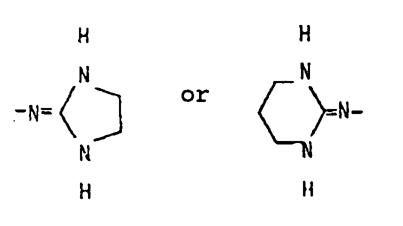 R¹ and R² being as defined above in (a), D represents the group -CH₂-S-(CH₂)ₙ- or -(CH₂)ₒ-, n having the value 2 or 3 and o having the value 2, 3 or 4, or
(e) R represents the group 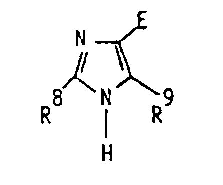 in which R⁸ is a hydrogen atom, an optionally halogen-substituted benzyl group, the group (R¹R²)N-CH₂- or an amino group, R¹ and R² being as defined above in (a), R⁹ is a hydrogen atom, a linear C₁₋₃ alkyl or C₁₋₃ alkylthio group, E represents the group -CH₂-S-(CH₂ₙ-, 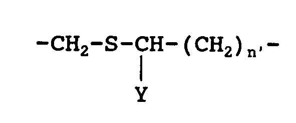 or 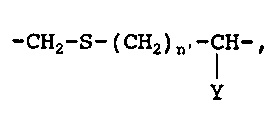 n having the value 2 or 3 and n' having the value 1, 2 or 3 and Y being as defined above in (c), or
(f) R represents the group 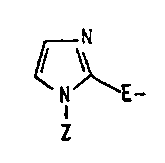 where Z is a hydrogen atom or a linear C₁ -₃ alkyl group and E is as defined above in (e), or
(g) R represents the group R"-A'-B'-, where R" is an unsubstituted or halogen-, linear C₁ -₃ alkyl- or linear C₁ -₃ alkoxy-substituted phenyl group or an unsubstituted or halogen-, linear C₁ -₃ alkyl- or linear C₁₋₃ alkoxy-substituted naphthyl group, A' is a single bond or represents the group -CR^{1'}R^{2'} or a nitrogen atom substituted by a phenyl, naphthyl, benzyl, pyridinyl, furanyl or thiophenyl group - optionally substituted by halogen, linear C₁ -₃ alkyl or linear C₁ -₃ alkoxy - or by a linear C₁ -₃ alkyl group, R^{1'} being a hydrogen atom or a methyl group, R^{2'} being a phenyl group optionally substituted by halogen, linear C₁ -₃ alkyl or linear C₁ -₃ alkoxy or a pyridinyl, furanyl or thiophenyl group optionally substituted by halogen, linear C₁ -₃ alkyl or linear C₁ -₃ alkoxy, B' represents the group -CH(Y)-S-(CH₂)_{m'},-, -CH₂-S-CH₂-CH(Y)-CH₂-, -CH₂-S-CH(Y)-CH₂-, -CH₂-S-CH₂-CH(Y)-, -(CH2)_{n"}-, - (CH₂)-CH(Y)-, -(CH₂)_{n"}-CH(Y)-, -O-(CH₂)₂-, -CH2-0-(CH2)o-, -CH₂-O-CH₂-CH(Y)-CH₂-, -O-CH₂-CH(Y)-, -0-CH(Y)-CH₂-, -S-(CH₂)q-, -S-CH₂-CH(Y)-, -S-CH(Y)-CH₂ or -S-CH₂-CH(Y)-CH₂-, Y being a hydrogen atom or a linear C₁ -₃ alkyl group, m' and o' having the value 2 or 3, n" and q each having the value 2, 3, 4 or 5, or
(h) R represents the group R"'-A"-B"-, where R"' is an unsubstituted or halogen-, linear C₁ -₃ alkyl-or linear C₁₋₃ alkoxy-substituted pyridinyl, imidazolyl, pyrimidinyl, thiophenyl, furanyl, benzimidazolyl or quinolyl group, to which a phenyl ring may optionally be fused, A" is a single bond or represents the group -CR^{1'}R^{2'} or a nitrogen atom substituted by a phenyl, naphthyl, benzyl, pyridinyl, benzimidazolyl, thiophenyl or furanyl group - optionally substituted by halogen, linear C₁ -₃ alkyl or linear C₁₋₃ alkoxy - or by a linear C₁₋₃ alkyl group, R^{1'} being a hydrogen atom or a methyl group, R^{2'} being a phenyl group optionally substituted by halogen, linear C₁ -₃ alkyl or linear C₁ -₃ alkoxy or a benzimidazolyl, pyridinyl, thiophenyl or furanyl group optionally substituted by halogen, linear C₁ -₃ alkyl or linear C₁₋₃ alkoxy, B" represents the group -CH(Y)-S-(CH2)m-, -CH₂-S-CH₂-CH(Y)-CH₂-, -CH₂-S-CH(Y)-CH₂-, -(CH₂)ₙ"CH(Y)-, -CH₂-S-CH₂-CH(Y)-, -(CH₂)ₙ"- -,-O(CH₂)_{n"}- -S-CH2-CH(Y)-, -S-CH(Y)-CH₂-, -S-(CH₂)q-, or -S-CH₂-CH(Y)-CH₂-, Y representing a hydrogen atom or a linear C₁-₃ alkyl group, m' having the value 2 or 3 and n" and q having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group,
and physiologically acceptable salts thereof,
characterized in that
(1) a compound corresponding to general formula II 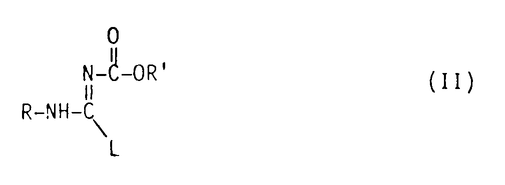 in which R and R' are as defined above and L is a leaving group,
is reacted with a compound corresponding to general formula III 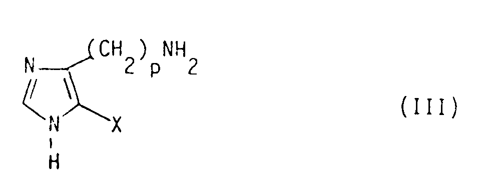 in which X and p are as defined above,
to form a compound corresponding to general formula I or
(2) a compound corresponding to general formula IV 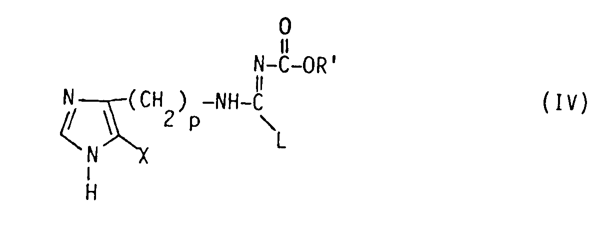 in which R', X and p are as defined above and L is a leaving group,
is reacted with a compound corresponding to general formula (V) 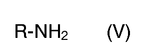 in which R is as defined above,
to form a compound corresponding to general formula I
and in that the compound obtained in accordance with (1) or (2) is converted into a physiologically acceptable salt by methods known per se.

27. A medicament, characterized in that it contains a compound according to claims 1 to 25 and at least one inert, pharmaceutically acceptable carrier or an inert, pharmaceutically acceptable diluent.

## Claims (Claims for the following Contracting State(s) : ES, GR)

1. A process for the production of guanidine derivatives corresponding to general formula I 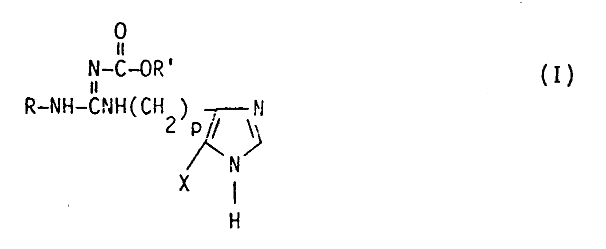 in which
(a) R represents the group 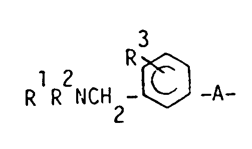 where R¹ and R² may be the same or different and represent hydrogen, linear C₁₋₆ alkyl or C₅₋₆ cycloalkyl or R¹ and R² together with the nitrogen atoms to which they are attached form a 5- to 10- membered, nitrogen-containing, alicyclic heterocyclic ring, R³ is a hydrogen atom, a halogen atom or a C₁₋₃ alkoxy group, A represents the group -O-(CH₂)ₖ-, -O-CH₂CH(OH)CH₂-, -0-CH2CH(CH3)-CH2-, -CH₂-O-CH₂-CH(OH)-CH₂- or -0-CH₂-CH(OH)-CH(OH)-CH₂, k having the value 3 or 4, or
(b) R represents the group 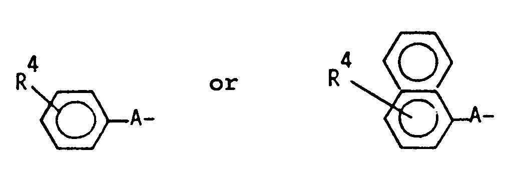 where R⁴ is a hydrogen atom, a halogen atom attached to A preferably in the para position, a C₁ -₃ alkoxy group or a C₁ -₃ alkyl group and A is as defined above under (a), or
(c) R represents the group 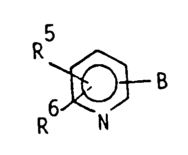 where R⁵ and R⁶ may be the same or different and represent a hydrogen atom, a halogen atom or a linear C₁ -₃ alkyl group or a linear C₁ -₃ alkoxy group, B may be attached in position 2, 3 or 4 of the pyridine ring and represents the group 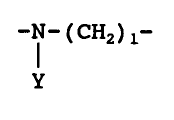 or -(CH2)m-, I having the value 2, 3 or 4 and m having the value 3, 4 or 5 and Y being a hydrogen atom or a linear C₁ -₃ alkyl group or
(d) R represents the group 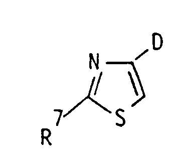 where R⁷ represents the group (R¹R²)N-CH₂-, (H₂N)₂ C = N-, 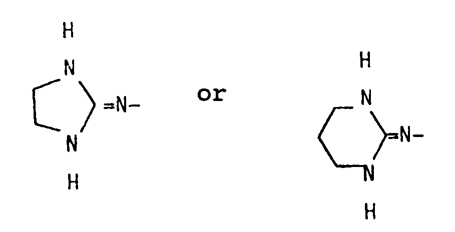 R¹ and R² being as defined above in (a), D represents the group -CH₂-S-(CH₂)ₙ- or -(CH₂)o-, n having the value 2 or 3 and o having the value 2, 3 or 4, or
(e) R represents the group 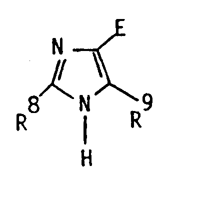 in which R⁸ is a hydrogen atom, an optionally halogen-substituted benzyl group, the group (R¹R²)N-CH₂- or an amino group, R¹ and R² being as defined above in (a), R⁹ is a hydrogen atom, a linear C₁₋₃ alkyl or C₁₋₃ alkylthio group, E represents the group -CH₂-S-(CH₂)ₙ-, 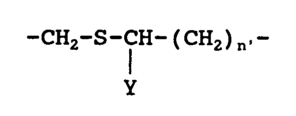 or 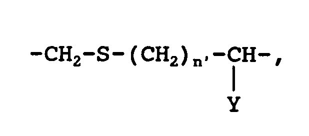 n having the value 2 or 3 and n' having the value 1, 2 or 3 and Y being as defined above in (c), or
(f) R represents the group 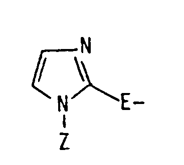 where Z is a hydrogen atom or a linear C₁ -₃ alkyl group and E is as defined above in (e), or
(g) R represents the group R"-A'-B'-, where R" is an unsubstituted or halogen-, linear C₁ -₃ alkyl- or linear C₁ -₃ alkoxy-substituted phenyl group or an unsubstituted or halogen-, linear C₁ -₃ alkyl- or linear C₁₋₃ alkoxy-substituted naphthyl group, A' is a single bond or represents the group -CR^{1'}R^{2'} or a nitrogen atom substituted by a phenyl, naphthyl, benzyl, pyridinyl, furanyl or thiophenyl group - optionally substituted by halogen, linear C₁ -₃ alkyl or linear C₁ -₃ alkoxy - or by a linear C₁ -₃ alkyl group, R^{1'} being a hydrogen atom or a methyl group, R being a phenyl group optionally substituted by halogen, linear C₁ -₃ alkyl or linear C₁ -₃ alkoxy or a pyridinyl, furanyl or thiophenyl group optionally substituted by halogen, linear C₁ -₃ alkyl or linear C₁ -₃ alkoxy, B' represents the group -CH(Y)-S-(CH2)m-, -CH₂-S-CH₂-CH(Y)-CH₂-, -CH₂-S-CH(Y)-CH₂-, -CH₂-S-CH₂-CH(Y)-, -(CH₂)_{n"}-, - (CH₂)-CH(Y)-, -(CH₂)_{n"}-CH(Y)-, -O-(CH₂)₂-, -CH₂-O-(CH₂)ₒ-, -CH₂-O-CH₂-CH(Y)-CH₂-, -O-CH₂-CH(Y)-, -0-CH(Y)-CH₂-, -S-(CH₂)q-, -S-CH₂-CH(Y)-, -S-CH(Y)-CH₂ or -S-CH₂-CH(Y)-CH₂-, Y being a hydrogen atom or a linear C₁ -₃ alkyl group, m' and o' having the value 2 or 3, n" and q each having the value 2, 3, 4 or 5, or
(h) R represents the group R"'-A"-B"-, where R"' is an unsubstituted or halogen-, linear C₁ -₃ alkyl-or linear C₁₋₃ alkoxy-substituted pyridinyl, imidazolyl, pyrimidinyl, thiophenyl, furanyl, benzimidazolyl or quinolyl group, to which a phenyl ring may optionally be fused, A" is a single bond or represents the group -CR^{1'}R^{2'} or a nitrogen atom substituted by a phenyl, naphthyl, benzyl, pyridinyl, benzimidazolyl, thiophenyl or furanyl group - optionally substituted by halogen, linear C₁ -₃ alkyl or linear C₁₋₃ alkoxy - or by a linear C₁₋₃ alkyl group, R^{1'} being a hydrogen atom or a methyl group, R^{2'} being a phenyl group optionally substituted by halogen, linear C₁ -₃ alkyl or linear C₁ -₃ alkoxy or a benzimidazolyl, pyridinyl, thiophenyl or furanyl group optionally substituted by halogen, linear C₁ -₃ alkyl or linear C₁₋₃ alkoxy, B" represents the group -CH(Y)-S-(CH2)m-, -CH₂-S-CH₂-CH(Y)-CH₂-, -CH₂-S-CH(Y)-CH₂-, -(CH₂)_{n"} CH(Y)-, -CH₂-S-CH₂-CH(Y)-, (CH2)_{n"}-, O(CH₂)_{n"}-, -S-CH2-CH(Y)-, -S-CH-(Y)-CH₂-, -S-(CH₂)q-, or -S-CH₂-CH(Y)-CH₂-, Y representing a hydrogen atom or a linear C₁₋₃ alkyl group, m' having the value 2 or 3 and n" and q having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group,
and physiologically acceptable salts thereof, characterized in that
(1) a compound corresponding to general formula II 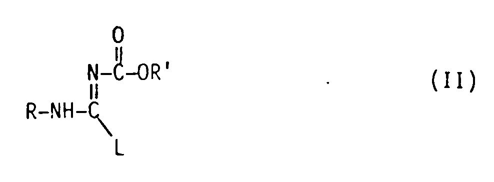 in which R and R' are as defined above and L is a leaving group,
is reacted with a compound corresponding to general formula III 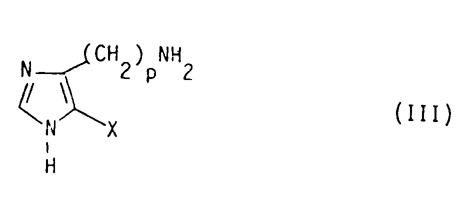 in which X and p are as defined above,
to form a compound corresponding to general formula I or
(2) a compound corresponding to general formula IV 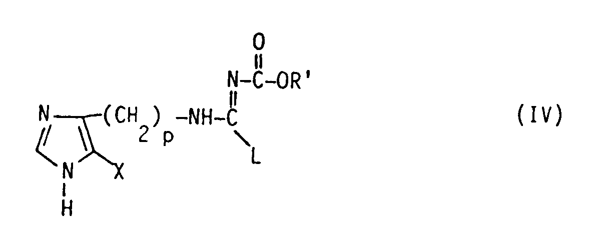
in which R', X and p are as defined above and L is a leaving group, is reacted with a compound corresponding to general formula (V) 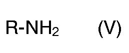 in which R is as defined above,
to form a compound corresponding to general formula I
and in that the compound obtained in accordance with (1) or (2) is converted into a physiologically acceptable salt by methods known per se.

2. A process as claimed in claim 1 for the production of compounds in which R represents the group 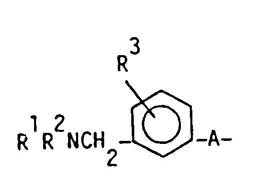 where R¹ and R² together with the nitrogen atom form a pyrrolidine or piperidine ring, R³ represents a hydrogen atom, A is the group -O(CH₂)ₖ, OCH₂CH(OH)CH₂- or OCH₂CH(CH₃)-CH₂-,
R' is a linear or branched C₁₋₆ alkyl or cycloalkyl group optionally substituted one or times by a halogen atom, a C₁ -₃ alkoxy group and/or a phenyl or naphthyl radical, k and p having the value 3 and X is a hydrogen atom.

3. A process as claimed in claim 1 for the production of compounds in which R represents the group 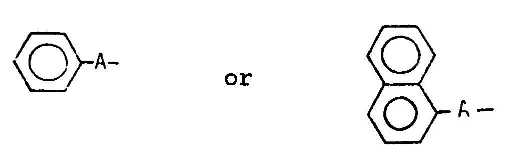 where A is the group -O-(CH₂)ₖ, -O-CH₂CH(OH)CH₂-, -0-CH₂CH(CH₃)-CH₂-, -CH₂-0-CH₂-CH(OH)-CH₂-or -0-CH₂-CH(OH)-CH(OH)-CH₂,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

4. A process as claimed in claim 1 for the production of compounds in which R represents the group 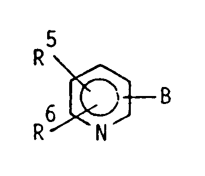 where R⁵ is a halogen atom or hydrogen atom attached in the 5 position of the pyridine ring, R⁶ is a hydrogen atom attached in the 3 position of the pyridine ring, a methyl group or a methoxy group, B may be attached in position 2, 3 or 4 of the pyridine ring and represents the group 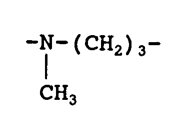 or -(CH₂)₃-₄- or -NH-(CH₂)₁, I having the value 2, 3 or 4,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

5. A process as claimed in claim 1 for the production of compounds in which R represents the group 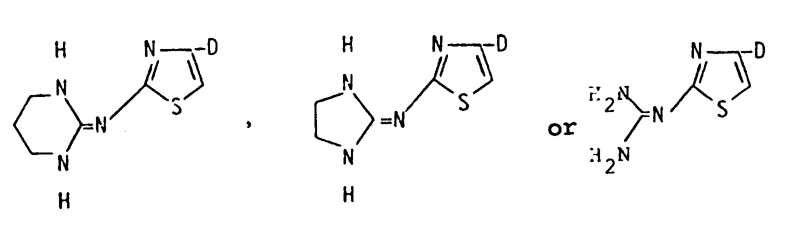 where D is the group -CH₂-S-(CH₂)₂- or -(CH₂)₃- or -(CH₂)₄-and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

6. A process as claimed in claim 1 for the production of compounds in which R represents the group 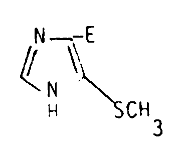 where E is the group -CH₂-S-(CH₂)₂-, 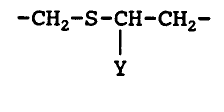 or 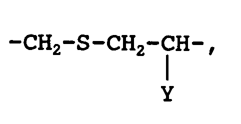 Y representing a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

7. A process as claimed in claim 1 for the production of compounds in which R represents the group 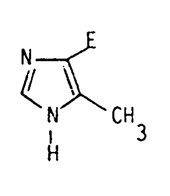 where E is the group -CH₂-S-(CH₂)₂-, 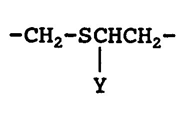 or 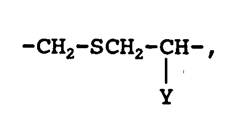 Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

8. A process as claimed in claim 1 for the production of compounds in which R represents the group 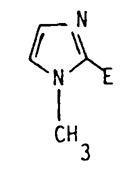 where E is the group 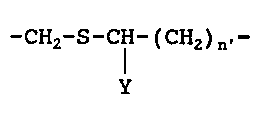 or 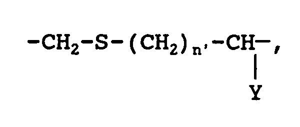 n' having the value 1, 2 or 3 and Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

9. A process as claimed in claim 1 for the production of compounds in which R represents the group R"-A'-B'-, where R" is an unsubstituted phenyl group, A' is a single bond, B' is the group -CH₂-S-(CH₂)ₘ-or -CH₂-S-CH₂-CH(Y)-CH₂-, m' having the value 2 or 3 and Y representing a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

10. A process as claimed in claim 1 for the production of compounds in which R represents the group R"-A'-B'-, where R" is an unsubstituted or halogen-, linear C₁ -₃ alkyl- or linear C₁ -₃ alkoxy-substituted phenyl group, A' is a nitrogen atom substituted by a phenyl, naphthyl, pyridinyl, furanyl or thiophenyl group, B' represents the group -(CH₂)_{n"}-, n" having the value 2 or 3,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

11. A process as claimed in claim 1 for the production of compounds in which R represents the group R"-A'-B'-, where R" is an unsubstituted or halogen-, linear C₁ -₃ alkyl- or linear C₁ -₃ alkoxy-substituted phenyl group, A' represents the group -CR^{1'}R^{2'}-, R^{1'} being a hydrogen atom or a methyl group, R being a phenyl group optionally substituted by halogen, linear Cᵢ -₃ alkyl or linear Ci -₃ alkoxy or a pyridinyl, furanyl or thiophenyl group optionally substituted by halogen, linear C₁ -₃ alkyl or linear C₁ -₃ alkoxy, B' represents the group -(CH₂)_{n"}-, n" having the value 2, 3 or 4,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

12. A process as claimed in claim 1 for the production of compounds in which R represents the group R"'-A"-B"-, where R"' is an unsubstituted or halogen-, linear C₁ -₃ alkyl- or linear C₁ -₃ alkoxy-substituted pyridine ring, A" is a single bond in position 2, 3 or 4 of the pyridine ring and B" is the group -CH₂-S-(CH₂)ₘ,- or -CH₂-S-CH₂-CH(Y)-CH₂-, m' having the value 2 or 3 and Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

13. A process as claimed in claim 1 for the production of compounds in which R represents the group R"'-A"-B"-, where R"' is an unsubstituted or halogen-, linear C₁ -₃ alkyl- or linear C₁ -₃ alkoxy-substituted thiophene ring, A" is a single bond, B" is the group -CH₂-S-(CH₂)m'- or -CH₂-S-CH₂-CH(Y)-CH₂-, m' having the value 2 or 3 and Y being a hydrogen atom or a linear C₁ -₃ alkyl group,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

14. A process as claimed in claim 1 for the production of compounds in which R represents the group R"'-A"-B"-, where R"' is an unsubstituted or halogen-, linear C₁ -₃ alkyl- or linear C₁ -₃ alkoxy-substituted pyridine ring, A" is the group CR^{1'}R^{2'}, R^{1'} being a hydrogen atom or methyl group and R being a phenyl group optionally substituted by halogen, linear Cᵢ -₃ alkyl or linear C₁₋ₐ alkoxy or a pyridinyl, furanyl, thiophenyl or benzimidazolyl group optionally substituted by halogen, linear C₁ -₃ alkyl or linear Ci -₃ alkoxy, B" is the group -(CH2)ₙ"-, n" having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

15. A process as claimed in claim 1 for the production of compounds in which R represents the group R"'-A"-B", where R"' is an unsubstituted or halogen-, linear C₁ -₃ alkyl- or linear C₁ -₃ alkoxy-substituted pyridine ring, A" is single bond in position 2, 3 or 4 of the pyridine ring and B" is the group -(CH₂)ₙ"-, n" having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

16. A process as claimed in claim 1 for the production of compounds in which R represents the group R"'-A"-B"-, where R"' is an unsubstituted or halogen-, linear C₁ -₃ alkyl- or linear C₁ -₃ alkoxy-substituted pyridine ring, A" is single bond or the group -CR^{1'}R^{2'} or a nitrogen atom substituted by a phenyl, naphthyl, benzyl, pyridinyl, benzimidazolyl, thiophenyl or furanyl group - optionally substituted by halogen, linear C₁₋₃ alkyl or linear C₁₋₃ alkoxy - or by a linear C₁₋₃₋ alkyl group, R^{1'} being a nitrogen atom or a methyl group and R being a phenyl group optionally substituted by halogen, linear C₁₋₃ alkyl or linear Cᵢ -₃ alkoxy or a benzimidazolyl, pyridinyl, thiophenyl or furanyl group optionally substituted by halogen, linear C₁₋₃ alkyl or linear C₁₋₃ alkoxy, B" is the group -(CH₂)ₙ"-, n" having the value 2, 3, 4 or 5,
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

17. A process as claimed in claim 1 for the production of compounds in which R represents the group R"'-A"-B"-, where R"' is the group 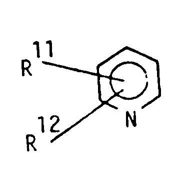 R¹¹ and R¹² independently of one another representing a halogen atom, a linear C₁₋₃ alkyl group or a linear C₁₋₃ alkoxy group, A" is the group CR^{1'}R^{2'}, R¹' being a hydrogen atom or a methyl group and R²' being an unsubstituted phenyl group or a phenyl group substituted once to three times by halogen, linear C₁ -₃ alkyl or linear C₁ -₃ alkoxy, or A" is a nitrogen atom substituted by a phenyl, naphthyl, pyridinyl, furanyl, thiophenyl or benzimidazolyl group or a hydrogen atom, B" represents the group -(CH₂)ₙ-, -O(CH₂)₂- or -SCH₂CH₂-whereA" is the group -CR^{1'}R^{2'} and the group -(CH₂)ₙ- (n = 2 or 3) where A" is a nitrogen atom substituted by a phenyl, naphthyl, pyridinyl, furanyl, thiophenyl or benzimidazolyl group or by a hydrogen atom and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

18. A process as claimed in claim 1 for the production of compounds in which R represents the group R"'-A"-B"-, where R"' is the group 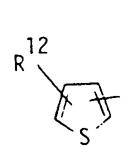 R¹² being a hydrogen atom, a halogen atom, a linear C₁₋₃ alkyl group, the group (CH₃)₂-NCH₂- or 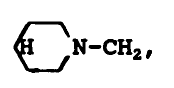 A" is the group -CR^{1'}R^{2'}, R^{1'} being a hydrogen atom or a methyl group and R being an unsubstituted phenyl group or a phenyl group substituted once to three times by halogen, linear C₁ -₃ alkyl or linear C₁ -₃ alkoxy, or A" is a nitrogen atom substituted by a phenyl, naphthyl, pyridinyl, furanyl, thiophenyl or benzimidazolyl group or by a hydrogen atom, B" is the group -CH₂-S-CH₂-CH₂-, -CH₂-S-CH₂-CH(CH₃)-CH₂-, -CH₂-S-CH(CH₃)-CH₂- or -CH₂-S-CH₂-CH(CH₃)- and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

19. A process as claimed in claim 1 for the production of compounds in which R represents the group R"'-A"-B"-, where R"' is the group 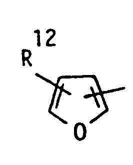 R₁₂ being a hydrogen atom, a halogen atom, a linear a linear C₁₋₃ alkyl group, the group (CH₃)₂-NCH₂- or 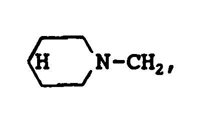 A" is a single bond in position 2, B" is the group -CH₂-S-CH₂-CH₂-, CH₂-S-CH₂-CH(CH₃)-CH₂-, -CH₂-S-CH(CH₃)-CH₂- or -CH₂-S-CH₂-CH(CH₃)- and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

20. A process as claimed in claim 1 for the production of compounds in which R represents the group R"'-A"'B"-, where R"' is the group 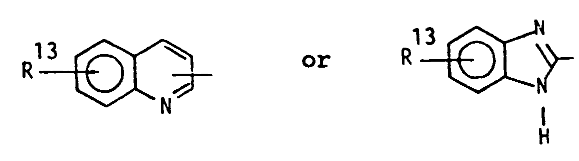 R¹³ being a hydrogen atom, a halogen atom, a linear C₁₋₃ alkyl group, the group (CH₃)₂-NCH₂- or 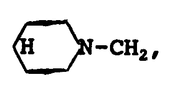 A" is a single bond, B" is the group -CH₂-S-CH₂-CH₂-, -CH₂-S-CH₂-CH(CH₃)-CH₂-,-CH₂-S-CH(CH₃)-CH₂- or -CH₂-S-CH₂-CH(CH₃) and
R' is a linear or branched C₁ -₆ alkyl or cycloalkyl group optionally substituted one or more times by a halogen atom, a C₁ -₃ alkoxy group and/or a phenyl or naphthyl radical, p has the value 2 or 3 and X is a hydrogen atom or a methyl group.

21. A process as claimed in claim 1 for the production of N'-[3-(imidazol-4-yl)propyl]-N²-[4-pyridin-2-yl)-butyl]-N³⁻methoxycarbonyl guanidine and physiologically acceptable salts thereof.

22. A process as claimed in claim 1 for the production of N'-[3-(imidazol-4-yl)propyl]-N²-[4-pyridin-2-yl)-butyl]-N³⁻ethoxycarbonyl guanidine and physiologically acceptable salts thereof.

23. A process as claimed in claim 1 for the production of N'-[3-(imidazol-4-yl)propyl]-N²-[3-(4-fluorophenyl)-3-(pyridin-2-yl)propyl]-N³⁻methoxycarbonyl guanidine and physiologically acceptable salts thereof.

24. A process as claimed in claim 1 for the production of N'-[3-(imidazol-4-yl)propyl]-N²-[3-(4-fluorophenyl)-3-(pyridin-2-yl)propyl]-N³⁻ethoxycarbonyl guanidine and physiologically acceptable salts thereof.

25. A process as claimed in claim 1 for the production of N'-[3-(imidazol-4-yl)propyl]-N²-[3-(4-fluorophenyl)-3-(pyridin-2-yl)propyl]-N³⁻butoxycarbonyl guanidine and physiologically acceptable salts thereof.

## Revendications (Revendications pour l'(les) Etat(s) contractant(s) suivant(s) : suivants : AT, BE, CH, DE, FR, GB, IT, LI, LU, NL, SE)

1. Esters d'acides guanidinecarboxyliques de formule générale (I): 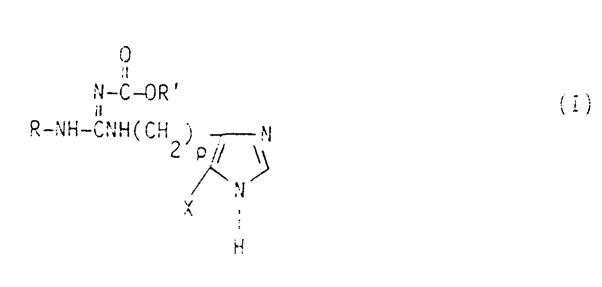 dans laquelle
(a) R représente le groupement 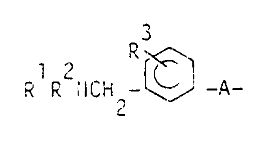 dans lequel R¹ et R², qui peuvent être identiques ou différents, représentent à chaque fois un atome d'hydrogène, un groupe alkyle linéaire en Ci à C₆ ou alkyle en C₅ ou C₆, ou bien R¹ et R² forment avec l'atome d'azote auquel ils sont reliés, un noyau azoté alicyclique, hétérocyclique, comportant 5 à 10 chaînons (pentagonal à décagonal) ; R³ représente un atome d'hydrogène, un atome d'halogène ou un groupe alcoxy en Ci à C₃ ; A représente le groupement -O-(CH₂)ₖ-,-O-CH₂CH-(OH)CH₂-, -0-CH₂CH(CH₃)-CH₂-, -CH₂-O-CH₂-CH(OH)-CH₂- ou -0-CH₂-CH(OH)-CH(OH)-CH₂, l'indice a valant 3 ou 4, ou bien
(b) R représente le groupement 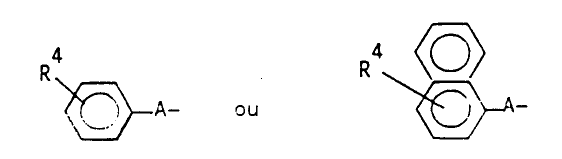 dans lequel R⁴ représente un atome d'hydrogène, un atome d'halogène fixé de préférence en position para par rapport à A, un groupe alcoxy en Ci à C₃ ou alkyle en Ci à C₃, et A a le sens indiqué ci-dessus en (a), ou bien
(c) R représente un groupement 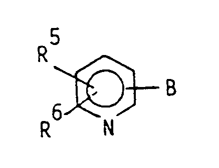 dans lequel R⁵ et R⁶, qui peuvent être identiques ou différents, représentent à chaque fois un atome d'hydrogène, un atome d'halogène ou un groupe alkyle linéaire en Ci à C₃ ou alcoxy linéaire en Ci à C₃ ; B peut être fixé en position 2, 3 ou 4 du noyau pyridine et représente le groupement 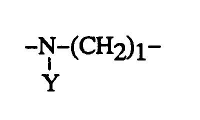 ou -(CH₂)ₘ-, dans lequel I vaut 2, 3 ou 4 et m vaut 2, 3, 4 ou 5, et Y représente un atome d'hydrogène ou un groupe alkyle linéaire en C₁ à C₃, ou bien
(d) R représente le groupement 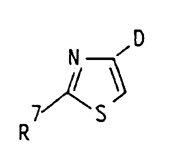 dans lequel R⁷ représente le groupe (R¹R²)N-CH₂-,(H₂ N)₂ C = N-, 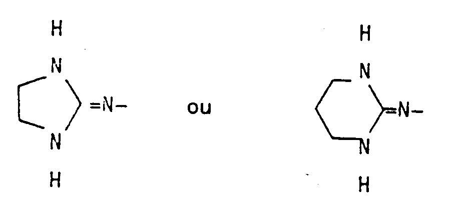 où R¹ et R² ont le sens indiqué ci-dessus en (a); D représente le groupement -CH₂-S-(CH₂)ₙ- ou -(CH₂)ₒ-, l'indice n valant 2 ou 3 et l'indice o valant 2, 3 ou 4, ou bien
(e) R représente le groupement 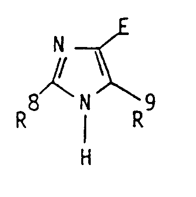 dans lequel R⁸ représente un atome d'hydrogène, un groupe benzyle éventuellement substitué par un atome d'halogène, le groupement (R¹R²)N-CH₂- ou un groupe amino, les symboles R¹ et R² ayant le sens indiqué ci-dessus en (a) ; R⁹ représente un atome d'hydrogène, un groupe alkyle linéaire en Ci à C₃ ou alkylthio en Ci à C₃ ; E représente le groupement -CH₂-S-(CH₂)ₙ-, 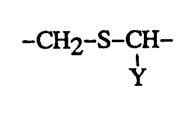 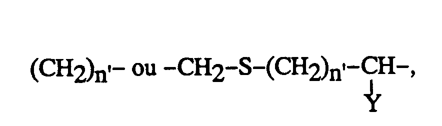 l'indice n valant 2 ou 3 et n' valant 1, 2 ou 3, et Y a le sens indiqué ci-dessus en (c) ; ou bien
(f) R représente le groupement 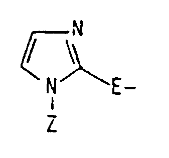 dans lequel Z représente un atome d'hydrogène ou un groupe alkyle linéaire en Ci à C₃, et E a le sens indiqué ci-dessus en (e), ou bien
(g) R représente le groupement R"-A'-B'-, dans lequel R" représente un groupe phényle (non substitué ou substitué par de l'halogène, par un groupe alkyle linéaire en Ci à C₃ ou alcoxy linéaire en Ci à C₃) ou un groupe naphtyle (non substitué ou substitué par de l'halogène, par un groupe alkyle linéaire en Ci à C₃ ou alcoxy linéaire en Ci à C₃) ; A' représente une liaison simple ou le groupement -CR^{1'}R^{2'} ou un atome d'azote (substitué par un groupe phényle, naphtyle, benzyle, pyridinyle, furannyle ou thiophényle, qui est éventuellement substitué par de l'halogène, par un groupe alkyle linéaire en Ci à C₃ ou alcoxy linéaire en Ci à C₃, ou bien un atome d'azote substitué par un groupe alkyle linéaire en Ci à C₃), le symbole R^{1'} représente un atome d'hydrogène ou un groupe méthyle ; R^{2'} représentant un groupe phényle (qui est éventuellement substitué par de l'halogène, par un groupe alkyle linéaire en Ci à C₃ ou alcoxy linéaire en Ci à C₃) ou un groupe pyridinyle, furannyle ou thiophényle (qui est éventuellement substitué par de l'halogène, par un groupe alkyle linéaire en C₁ à C₃ ou par un groupe alcoxy linéaire en C₁ à C₃) ; B' représente le groupement -CH(Y)-S-(CH2)ₘ',-,-CH₂-S-CH₂-CH(Y)-CH₂-, -CH₂-S-CH(Y)-CH₂-, -CH₂-S-CH₂-CH(Y)-, - (CH2)ₙ"-, -(CH2-CH(Y)-, -(CH₂)ₙ"-CH(Y), -O-CH₂)₂-, -CH₂-O-(CH₂)_{o'}-, -CH₂-O-CH₂-CH(Y)-CH₂-, -O-CH₂-CH(Y)-, -0-CH(Y)-CH₂-, -S-(CH₂)q-, -S-CH₂-CH(Y)-, -S-CH(Y)-CH₂ ou -S-CH₂-CH(Y)-CH₂-, où Y représente un atome d'hydrogène ou un groupe alkyle linéaire en C₁ à C₃, n' et o' valent chacun 2 ou 3 ; n" et q valent chacun 2, 3, 4 ou 5 ; ou bien
(h) R représente le groupement R"'-A"-B"-, dans lequel R"' représente un groupe pyridinyle, imidazolyle, pyrimidinyle, thiophényle, furannyle, benzimidazolyle ou quinolinyle (non substitués ou substitués par de l'halogène, par un groupe alkyle linéaire en C₁ à C₃ ou alcoxy linéaire en C₁ à C₃), sur lequel un noyau phénylique peut éventuellement être condensé ; A" représente une liaison simple ou le groupe -CR^{1'}(R^{2'} ou un atome d'azote (substitué par un groupe phényle, naphtyle, benzyle, pyridinyle, benzimidazolyle, thiophényle ou furannyle, qui est éventuellement substitué par de l'halogène, par un groupe alkyle linéaire en Ci à C₃ ou alcoxy linéaire en Ci à C₃) ou un atome d'azote substitué par un groupe alkyle linéaire en Ci à C₃, le symbole R^{1'} représentant un atome d'hydrogène ou un groupe méthyle, R^{2'} représentant un groupe phényle (qui est éventuellement substitué par de l'halogène, par un groupe alkyle linéaire en Ci à C₃ ou par un groupe alcoxy linéaire en Ci à C₃) ou un groupe benzimidazolyle, pyridinyle, thiophényle ou furannyle (qui est éventuellement substitué par de l'halogène, par un groupe alkyle linéaire en Ci à C₃ ou alcoxy linéaire en Ci à C₃) ; B" représente le groupement -CH(Y)-S-(CH₂)ₘ'-, -CH₂-S-CH₂-CH(Y)-CH₂-, CH₂-S-CH(Y)-CH₂-, -(CH₂)ₙ -CH(Y), -CH₂-S-CH₂-CH(Y)-, -(CH2)_{n"} -,O(CH₂)_{n"}-, -S-CH2-CH(Y)-, -S-CH-(Y)-CH₂-, -S-(CH₂)q- ou -S-CH₂-CH(Y)-CH₂-, le symbole Y représentant un atome d'hydrogène ou un groupe alkyle linéaire en Ci à C₃, m' vaut 2 ou 3 et n" et q valent chacun 2, 3, 4 ou 5,
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle ; ainsi que les sels physiologiquement acceptables de cet ester.

2. Esters d'acides guanidinecarboxyliques selon la revendication 1, caractérisés en ce que R représente le groupement 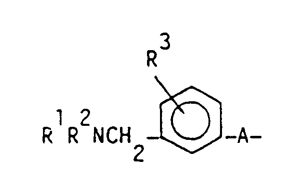 dans lequel R¹ et R², pris avec l'atome d'azote, forment un noyau pyrrolidine ou pipéridine ; R³ représente un atome d'hydrogène ; A représente le groupement -O(CH₂)ₖ-, -OCH₂CH(OH)CH₂-, ou OCH₂CH(CH₃)CH₂- ; R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle)ou R' représente un groupe cycloalkyle ; k et p valent 3 et X représente un atome d'hydrogène.

3. Esters d'acides guanidinecarboxyliques selon la revendication 1, caractérisés en ce que R représente le groupement 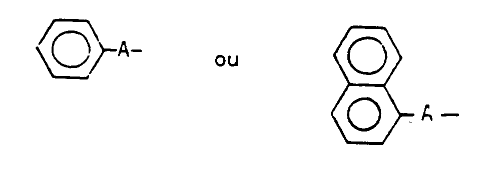 dans lequel, A représente le groupement -O-(CH₂)ₖ-, -0-CH₂CH(OH)CH₂-, -0-CH₂CH(CH₃)-CH₂-, -CH₂-0-CH₂-CH(OH)-CH₂- ou -0-CH₂-CH(OH)-CH(OH-CH₂- ;
R' représente un groupe alkyle en C₁ à C₆, linéaire ou ramifié (éventuellement substitué une ou plusieurs fois par un atome d'halogène, par un groupe alcoxy en C₁ à C₃ et/ou par un reste phényle ou naphtyle) ou représente un groupe cycloalkyle ; p vaut 2 ou 3 et X représente un atome d'hydrogène ou un groupe méthyle.

4. Esters d'acides guanidinecarboxyliques selon la revendication 1, caractérisés en ce que R représente le groupement 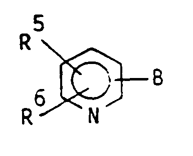 dans lequel R⁵ représente un atome d'halogène ou un atome d'hydrogène fixé en position 5 du noyau pyridine ; R⁶ représente un atome d'hydrogène ou un groupe méthyle ou méthoxy fixé en position 3 du noyau pyridine ; B peut être fixé en position 2, 3 ou 4 du noyau pyridine et représente le groupement 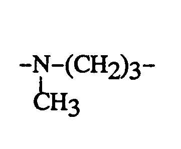 ou bien -(CH₂)₃-₄- ou -NH-(CH₂)₁, où I vaut 2, 3 ou 4, R' représente un reste alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

5. Esters d'acides guanidinecarboxyliques selon la revendication 1, caractérisés en ce que R représente le groupement 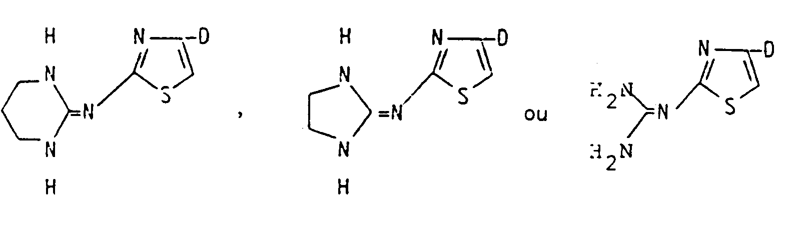 dans lequel D représente le groupement -CH₂-S-(CH₂)₂- ou -(CH₂)₃- ou -(CH₂)₄-, et
R' représente un groupe alkyle en Ci à C₆, linéaire ou ramifié (éventuellement substitué une ou plusieurs fois par un atome d'halogène, par un groupe alkyle en Ci à C₃ et/ou par un reste phényle ou naphtyle) ou un groupe cycloalkyle ; p vaut 2 ou 3 et X représente un atome d'hydrogène ou un groupe méthyle.

6. Esters d'acides guanidinecarboxyliques selon la revendication 1, caractérisés en ce que R représente le groupement 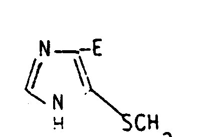 dans lequel E représente le groupement -CH₂-S-(CH₂)₂-, 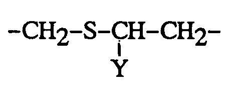 ou 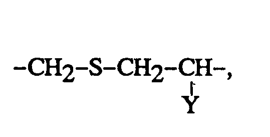 Y représente un atome d'hydrogène ou un groupe alkyle linéaire en Ci à C₃ ;
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou nahtyle) ou un groupe cycloalkyle ; p vaut 2 ou 3 et X représente un atome d'hydrogène ou un groupe méthyle.

7. Esters d'acides guanidinecarboxyliques selon la revendication 1, caractérisés en ce que R représente le groupement 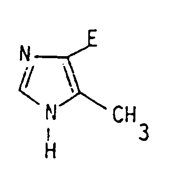 dans lequel E représente le groupement -CH₂-S-(CH₂)₂- 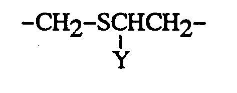 ou 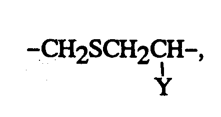 où Y représente un atome d'hydrogène ou un groupe alkyle linéaire en Ci à C₃;
R' représente un groupe alkyle en C₁ à C₆, linéaire ou ramifié (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 ; et X représente un atome d'hydrogène ou un groupe méthyle.

8. Esters d'acides guanidinecarboxyliques selon la revendication 1, caractérisés en ce que R représente le groupement 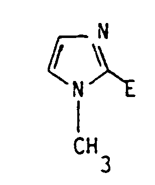 dans lequel E représente le groupement 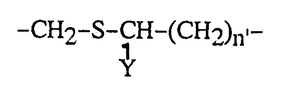 ou -CH₂-S- 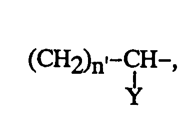 où n' vaut 1, 2 ou 3 et Y représente un atome d'hydrogène ou un groupe alkyle linéaire en Ci à C₃,
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

9. Esters d'acides guanidinecarboxyliques selon la revendication 1, caractérisés en ce que R représente le groupement R"-A'-B'-, dans lequel R" représente un groupe phényle non substitué ; A' représente une liaison simple ; B' repré-sente le groupement -CH₂-S-(CH₂)_{m'}- ou -CH₂-S-CH₂-CH(Y)-CH₂-, m valant 2 ou 3 et Y représentant un atome d'hydrogène ou un groupe alkyle linéaire en Ci à C₃,
R' représente un groupe alkyle linéaire ou ramifié en Ci à C₆ (éventuellement substitué une ou plusieurs fois par un atome d'halogène, par un groupe alcoxy en Ci à Ca et/ou par un reste phényle ou naphtyle) ou un groupe cycloalkyle ; p vaut 2 ou 3 et X représente un atome d'hydrogène ou un groupe méthyle.

10. Esters d'acides guanidinecarboxyliques selon la revendication 1, caractérisés en ce que R représente le groupement R"-A'-B'-, dans lequel R" représente un groupe phényle (non substitué ou substitué par de l'halogène, par un groupe alkyle linéaire en C₁ à C₃ ou par un groupe alcoxy linéaire en Ci à C₃) ; A' représente un atome d'azote (substitué par un groupe phényle, naphtyle, pyridinyle, furannyle ou thiophényle) ; B' représente le groupement -(CH₂)ₙ"-, dans lequel n" vaut 2 ou 3,
R' représente un groupe alkyle en Ci à C₆, linéaire ou ramifié (éventuellement substitué une ou plusieurs fois par un atome d'halogène, par un groupe alkyle en Ci à C₃ et/ou par un reste phényle ou naphtyle) ou un groupe cycloalkyle ; p vaut 2 ou 3 et X représente un atome d'hydrogène ou un groupe méthyle.

11. Esters d'acides guanidinecarboxyliques selon la revendication 1, caractérisés en ce que R représente le groupement R"-A'-B'-, dans lequel R" représente un groupe phényle (non substitué ou substitué par de l'halogène, par un groupe alkyle linéaire en C₁ à C₃ ou par un groupe alcoxy linéaire en Ci à C₃) ; A' représente le groupement -CR^{1'}R^{2'}-, dans lequel R1' représente un atome d'hydrogène ou un groupe méthyle, R2' représente un groupe phényle (qui est éventuellement substitué par de l'halogène, par un groupe alkyle linéaire en Ci à C₃ ou par un groupe alcoxy linéaire en Ci à C₃), ou un groupe pyridinyle, furannyle ou thiophényle (qui est éventuellement substitué par de l'halogène, par un groupe alkyle linéaire en Ci à C₃ ou par un groupe alcoxy linéaire en Ci à C₃) ; B' représente le groupement -(CH2)_{n"}-, dans lequel n" vaut 2, 3 ou 4,
R' représente un groupe alkyle, linéaire ou ramifié, en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

12. Esters d'acides guanidinecarboxyliques selon la revendication 1, caractérisés en ce que R représente le groupement R"'-A"-B"-, dans lequel R"' représente un noyau pyridine (non substitué ou substitué par de l'halogène, par un groupe alkyle linaire en Ci à C₃ ou par un groupe alcoxy linéaire en Ci à C₃), A" représente une liaison simple en position 2, 3 ou 4 du noyau pyridine et B" représente le groupement -CH₂-S-(CH2)_{m'}- ou -CH₂-S-CH₂-CH(Y)-CH₂-, dans lequel m' vaut 2 ou 3 et Y représente un atome d'hydrogène ou un groupe alkyle linéaire en C₁ à C₃,
R' représente un groupe alkyle en C₁ à C₆, linéaire ou ramifié (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 et X représente un atome d'hydrogène ou un groupe méthyle.

13. Esters d'acides guanidinecarboxyliques selon la revendication 1, caractérisés en ce que R représente le groupement R"'-A"-B"-, dans lequel R"' représente un noyau thiophène (non substitué ou substitué par de l'halogène, par un groupe alkyle linéaire en Ci à C₃ ou par un groupe alcoxy linéaire en Ci à C₃),A" représente une liaison simple, B" représente le groupement -CH₂-S-(CH₂)_{m'}- ou -CH₂-S-CH₂-CH(Y)-CH₂-, dans lequel m' vaut 2 ou 3 et Y représente un atome d'hydrogène ou un groupe alkyle linéaire en Ci à C₃,
R' représente un groupe alkyle, linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle, p vaut 2 ou 3 et X représente un atome d'hydrogène ou un groupe méthyle.

14. Esters d'acides guanidinecarboxyliques selon la revendication 1, caractérisés en ce que R représente le groupement R"'-A"-B"-, dans lequel R"' représente un noyau pyridine (non substitué ou substitué par de l'halogène, par un groupe alkyle linéaire en Ci à C₃ ou par un groupe alcoxy linéaire en Ci à C₃), A" représente le groupement -CR^{1'}R^{2'}, dans lequel R^{1'} représente un atome d'hydrogène ou un groupe alkyle, R^{2'} représente un groupe phényle (qui est éventuellement substitué par de l'halogène, par un groupe alkyle linéaire en Ci à C₃ ou par un groupe alcoxy linéaire en Ci à C₃) ou un groupe pyridinyle, furannyle, thiophényle ou berizimidazolyle (qui est éventuellement substitué par de l'halogène, par un groupe alkyle linéaire en Ci à C₃ ou par un groupe alcoxy linéaire en Ci à C₃, B" représente le groupement -(CH₂)_{n"}-, dans lequel n" vaut 2, 3, 4 ou 5,
R' représente un groupe alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle, p vaut 2 ou 3 et X représente un atome d'hydrogène ou un groupe méthyle.

15. Esters d'acides guanidinecarboxyliques selon la revendication 1, caractérisés en ce que R représente le groupement R"'-A"-B"-, dans lequel R"' représente un noyau pyridine (non substitué ou substitué par de l'halogène, par un groupe alkyle linéaire en Ci à C₃ ou par un groupe alcoxy linéaire en Ci à C₃), A" représente une liaison simple en position 2, 3 ou 4 du noyau pyridine, B" représente le groupement -(CH₂)_{n"}-, dans lequel n" vaut 2, 3, 4 ou 5,
R' représente un groupe alkyle, linéaire ou ramifié, en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

16. Esters d'acides guanidinecarboxyliques selon la revendication 1, caractérisés en ce que R représente le groupement R"'-A"-B"-, dans lequel R"' représente un noyau pyridine (non substitué ou substitué par de l'halogène, par un groupe alkyle linéaire en Ci à C₃ ou alcoxy linéaire en Ci à C₃), A" représente une liaison simple ou le groupement -CR^{1'}R^{2'} ou un atome d'azote (substitué par un groupe phényle, naphtyle, benzyle, pyridinyle, benzimidazolyle, thiophényle ou furannyle, qui est éventuellement lui-même substitué par de l'halogène, par un groupe alkyle linéaire en Ci à C₃ ou par un groupe alcoxy linéaire en C₁ à C₃) ou substitué par un groupe alkyle linéaire en C₁ à C₃, et R^{1'} représente un atome d'hydrogène ou un groupe méthyle, R^{2'} représente un groupe phényle (qui est éventuellement substitué par de l'halogène, par un groupe alkyle linéaire en C₁ à C₃ ou par un groupe alcoxy linéaire en C₁, à C₃) ou un groupe benzimidazolyle, pyridinyle, thiophényle ou furannyle (qui est éventuellement substitué par de l'halogène, par un groupe alkyle linéaire en C₁ à C₃ ou par un groupe alcoxy linéaire en C₁ à C₃), B" représente le groupement -(CH₂)_{n"}-, dans lequel n" vaut 2, 3, 4 ou 5,
R' représente un groupe alkyle linéaire ou ramifié en C₁ à C₆ (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 et X représente un atome d'hydrogène ou un groupe méthyle.

17. Esters d'acides guanidinecarboxyliques selon la revelidication 1, caractérisés en ce que R représente le groupement R"'-A"-B"-, dans lequel R"' représente le groupement 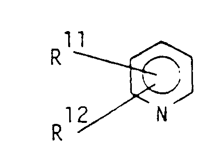 dans lequel, R¹¹ et R¹² représentent chacun, indépendamment l'un de l'autre, un atome d'halogène, un groupe alkyle linéaire en Ci à C₃ ou un groupe alcoxy linéaire en Ci à C₃ ; A" représente le groupement -CR^{1'}R^{2'}, dans lequel R^{1'} représente un atome d'hydrogène ou un groupe méthyle et R^{2'} représente un groupe phényle (non substitué ou substitué une à trois fois par un atome d'halogène, par un groupe alkyle linéaire en Ci à C₃ ou par un groupe alcoxy linéaire en Ci à C₃) ; ou bien A" représente un atome d'azote (qui est substitué par un groupe phényle, naphtyle, pyridinyle, furannyle, thiophényle ou benzimidazolyle ou par un atome d'hydrogène) ; B" représente, quand A" représente le groupe -CR^{1'}R^{2'}, le groupement -(CH₂)n-, -O(CH₂)₂- ou -SCH₂CH₂-, et B" représente, quand A" représente un atome d'azote (substitué par un groupe phényle, naphtyle, pyridinyle, furannyle, thiophényle ou benzimidazolyle ou par un atome d'hydrogène), le groupement -(CH₂)ₙ-, dans lequel n vaut 2 ou 3, et
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

18. Esters d'acides guanidinecarboxyliques selon la revendication 1, caractérisés en ce que R représente le groupement R"'-A"-B"-, dans lequel R"' représente le groupement 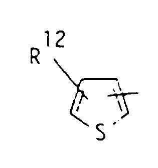 dans lequel R¹² représente un atome d'hydrogène, un atome d'halogène, un groupe alkyle linéaire en C₁ à C₃, le groupement (CH₃)₂-NCH₂-ou 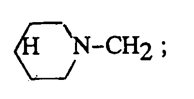 A" représente le groupement -CR^{1'}R^{2'}, dans lequel R^{1'} représente un atome d'hydrogène ou un groupe méthyle et R²' représente un groupe phényle (non substitué ou substitué une à trois fois par de l'halogène, par un groupe alkyle linéaire en Ci à C₃ ou par un groupe alcoxy linéaire en Ci à C₃) ou bien A" représente un atome d'azote (qui est substitué par un groupe phényle, naphtyle, pyridinyle, furannyle, thiophényle ou benzimidazolyle ou par un atome d'hydrogène) ; B" représente le groupement -CH₂-S-CH₂-CH₂-, -CH₂-S-CH₂-CH(CH₃)-CH₂-, -CH₂-S-CH(CH₃)-CH₂- ou -CH₂-S-CH₂-CH(CH₃)-, et
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

19. Esters d'acides guanidinecarboxyliques selon la revendication 1, caractérisés en ce que R représente le groupement R"'-A"-B"-, dans lequel R"' représente le groupement 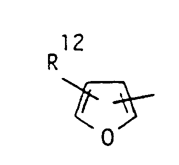 dans lequel R¹² représente un atome d'hydrogène, un atome d'halogène groupe alkyle linéaire en Ci à C₃, le groupement (CH₃)₂-NCH₂-ou 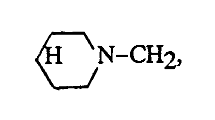 A" représente une liaison simple en position 2 ; B" représente le groupement -CH₂, A" S-CH₂-CH₂-, -CH₂-S-CH₂-CH(CH₃)-CH₂-, -CH₂-S-CH(CH₃)-CH₂- ou -CH₂-S-CH₂-CH(CH₃), et
R' représente un groupe alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

20. Esters d'acides guanidinecarboxyliques selon la revendication 1, caractérisés en ce que R représente le groupement R"'-A"-B"-, dans lequel R"' représente le groupement 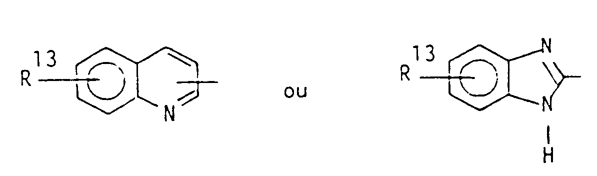 où R¹³ représente un atome d'hydrogène, un atome d'halogène, un groupe alkyle linéaire en Ci à C₃, le groupement (CH₃)₂-NCH₂ ou 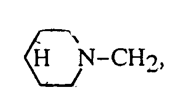 A" représente une liaison simple, B" représente le groupement -CH₂-S-CH₂-CH₂-, -CH₂-S-CH₂-CH-(CH₃)-CH₂-, -CH₂-S-CH(CH₃)-CH₂-, ou -CH₂-S-CH₂-CH-(CH₃), et
R' représente un groupe alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

21. La N'-[3-(imidazol-4-yl)propyl]-N²⁻[4-(pyridine-2-yl)-butyl]-N³⁻méthoxycarbonylguanidine et ses sels physiologiquement acceptables.

22. La N'-[3-(imidazol-4-yl)propyl]-N²-[4-(pyridine-2-yl)-butyl]-N³-éthoxycarbonylguanidine et ses sels physiologiquement acceptables.

23. La Ni⁻[3-(imidazol-4-yl)propyl]-N²⁻[3-(4-fluorophényl)-3-(pyridine-2-yl)propyl]-N³⁻méthoxycarbonylguani- dine et ses sels physiologiquement acceptables.

24. La N'-[3-(imidazol-4-yl)propyl]-N²⁻[3-(4-fluorophényl)-3-(pyridine-2-yl)propyl]-N³-éthoxycarbonylguanidi- ne et ses sels physiologiquement acceptables.

25. La N'-[3-(imidazol-4-yl)propyl]-N²⁻[3-(4-fluorophényl)-3-(pyridine-2-yl)propyl]-N³-butoxycarbonylguanidi- ne et ses sels physiologiquement acceptables.

26. Procédé de préparation de dérivés de la guanidine répondant à la formule générale (I): 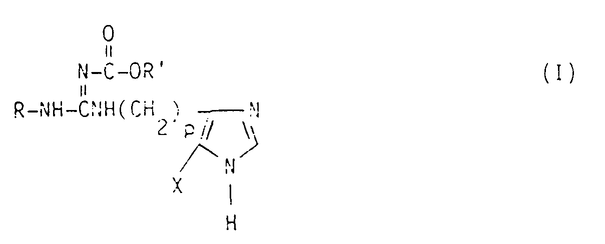 dans laquelle
(a) R représente le groupe 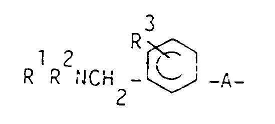 dans lequel R¹ et R², qui peuvent être identiques ou différents, représentent à chaque fois un atome d'hydrogène, un groupe alkyle linéaire en Ci à C₆ ou alkyle en C₅ ou C₆, ou bien R¹ et R² forment avec l'atome d'azote auquel ils sont reliés, un noyau azoté alicyclique, hétérocyclique, comportant 5 à 10 chaînons (pentagonal à décagonal) ; R³ représente un atome d'hydrogène, un atome d'halogène ou un groupe alcoxy en Ci à C₃ ; A représente le groupement -O-(CH₂)ₖ-,-O-CH₂CH-(OH)CH₂-, -0-CH₂CH(CH₃)-CH₂-, -CH₂-O-CH₂-CH(OH)-CH₂- ou -O-CH₂-CH(OH)-CH(OH-CH₂, l'indice a valant 3 ou 4, ou bien
(b) R représente le groupement 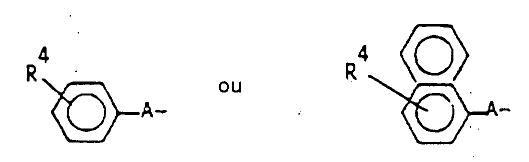 dans lequel R⁴ représente un atome d'hydrogène, un atome d'halogène fixé de préférence en position para par rapport à A, un groupe alcoxy en Ci à C₃ ou alkyle en Ci à C₃, et A a le sens indiqué ci-dessus en (a), ou bien
(c) R représente un groupement 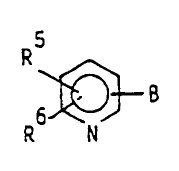 dans lequel R⁵ et R⁶, qui peuvent être identiques ou différents, représentent à chaque fois un atome d'hydrogène, un atome d'halogène ou un groupe alkyle linéaire en C₁ à C₃ ou alcoxy linéaire en C₁ à C₃ ; B peut être fixé en position 2, 3 ou 4 du noyau pyridine et représente le groupement 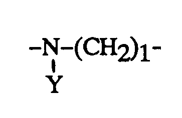 ou -(CH₂)ₘ-, dans lequel I vaut 2, 3 ou 4 et m vaut 2, 3, 4 ou 5, et Y représente un atome d'hydrogène ou un groupe alkyle linéaire en Ci à C₃, ou bien
(d) R représente le groupement 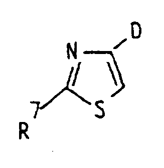 dans lequel R⁷ représente le groupe (R¹R²)N-CH₂-, (H2 N)₂C = N-, 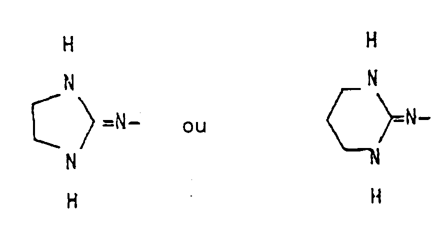 où R¹ et R² ont le sens indiqué ci-dessus en (a) ; D représente le groupement -CH₂-S-(CH₂)ₙ- ou -(CH₂)ₒ-, l'indice n valant 2 ou 3 et l'indice o valant 2, 3 ou 4, ou bien
(e) R représente le groupement 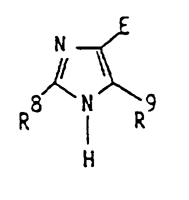 dans lequel R⁸ représente un atome d'hydrogène, un groupe benzyle éventuellement substitué par un atome d'halogène, le groupement (R¹R²)N-CH₂- ou un groupe amino, les symboles R¹ et R² ayant le sens indiqué ci-dessus en (a); R⁹ représente un atome d'hydrogène, un groupe alkyle linéaire en Ci à C₃ ou alkylthio en Ci à C₃ ; E représente le groupement -CH₂-S-(CH₂)ₙ-, 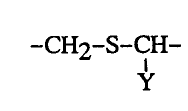 (CH₂)ₙ'- ou 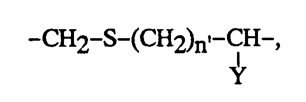 l'indice n valant 2 ou 3 et n' valant 1, 2 ou 3, et Y a le sens indiqué ci-dessus en (c) ; ou bien
(f) R représente le groupement 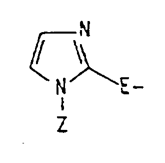 dans lequel Z représente un atome d'hydrogène ou un groupe alkyle linéaire en Ci à C₃, et E a le sens indiqué ci-dessus en (e), ou bien
(g) R représente le groupement R"-A'-B'-, dans lequel R" représente un groupe phényle (non substitué ou substitué par de l'halogène, par un groupe alkyle linéaire en Ci à C₃ ou alcoxy linéaire en Ci à C₃) ou un groupe naphtyle (non substitué ou substitué par de l'halogène, par un groupe alkyle linéaire en Ci à C₃ ou alcoxy linéaire en Ci à C₃) ; A' représente une liaison simple ou le groupement -CR^{1'}R^{2'} ou un atome d'azote (substitué par un groupe phényle, naphtyle, benzyle, pyridinyle, furannyle ou thiophényle, qui est éventuellement substitué par de l'halogène, par un groupe alkyle linéaire en Ci à C₃ ou alcoxy linéaire en Ci à C₃, ou bien un atome d'azote substitué par un groupe alkyle linéaire en Ci à C₃), le symbole R^{1'} représente un atome d'hydrogène ou un groupe méthyle ; R^{2'} représentant un groupe phényle (qui est éventuellement substitué par de l'halogène, par un groupe alkyle linéaire en Ci à C₃ ou alcoxy linéaire en Ci à C₃) ou un groupe pyridinyle, furannyle ou thiophényle (qui est éventuellement substitué par de l'halogène, par un groupe alkyle linéaire on Ci à C₃ ou par un groupe alcoxy linéaire en Ci à C₃) ; B' représente le groupement -CH(Y)-S-(CH2)_{m'},-,-CH₂-S-CH₂-CH(Y)-CH₂-, -CH₂-S-CH(Y)-CH₂-, -CH₂-S-CH₂-CH(Y)-, - (CH₂)n"- -(CH2)-CH(Y)-, -(CH2)ₙ" -CH(Y), -O-CH₂)₂-, -CH₂-O-(CH2)_{o'}-, -CH₂-O-CH₂-CH(Y)-CH₂-, -O-CH₂-CH(Y)-, -0-CH(Y)-CH₂-, -S-(CH₂)q-, -S-CH₂-CH(Y)-, -S-CH(Y)-CH₂ ou -S-CH₂-CH(Y)-CH₂-, où Y représente un atome d'hydrogène ou un groupe alkyle linéaire en Ci à C₃, n' et o' valent chacun 2 ou 3 ; n" et q valent chacun 2, 3, 4 ou 5 ; ou bien
(h) R représente le groupement R"'-A"-B"-, dans lequel R"' représente un groupe pyridinyle, imidazolyle, pyrimidinyle, thiophényle, furannyle, benzimidazolyle ou quinolinyle (non substitués ou substitués par de l'halogène, par un groupe alkyle linéaire en Ci à C₃ ou alcoxy linéaire en Ci à C₃), sur lequel un noyau phénylique peut éventuellement être condensé ; A" représente une liaison simple ou le groupe -CR^{1'}R^{2'} ou un atome d'azote (substitué par un groupe phényle, naphtyle, benzyle, pyridinyle, benzimidazolyle, thiophényle ou furannyle, qui est éventuellement substitué par de l'halogène, par un groupe alkyle linéaire en Ci à C₃ ou alcoxy linéaire en C₁ à C₃) ou un atome d'azote substitué par un groupe alkyle linéaire en C₁ à C₃, le symbole R^{1'} représentant un atome d'hydrogène ou un groupe méthyle, R^{2'} représentant un groupe phényle (qui est éventuellement substitué par de l'halogène, par un groupe alkyle linéaire en C₁ à C₃ ou par un groupe alcoxy linéaire en C₁ à C₃) ou un groupe benzimidazolyle, pyridinyle, thiophényle ou furannyle (qui est éventuellement substitué par de l'halogène, par un groupe alkyle linéaire en C₁ à C₃ ou alcoxy linéaire en C₁ à C₃) ; B" représente le groupement -CH(Y)-S-(CH2)_{m'}-, -CH₂-S-CH₂-CH(Y)-CH₂-, CH₂-S-CH(Y)-CH₂-, -(CH₂)ₙ"-CH(Y)-, -CH₂-S-CH₂-CH(Y)-, -(CH₂)ₙ" -,O(CH₂)ₙ" -, -S-CH2-CH(Y)-, -S-CH-(Y)-CH₂-, -S-(CH₂)q- ou -S-CH₂-CH(Y)-CH₂-, le symbole Y représentant un atome d'hydrogène ou un groupe alkyle linéaire en C₁ à C_{3,} m' vaut 2 ou 3 et n" et q valent chacun 2, 3, 4 ou 5, R' représente un groupe alkyle en C₁ à C₆, linéaire ou ramifié (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 et X représente un atome d'hydrogène ou un groupe méthyle ; ainsi que les sels physiologiquement acceptables de cet ester,
procédé caractérisé en ce que
(1) on fait réagir un composé de formule générale (II) : 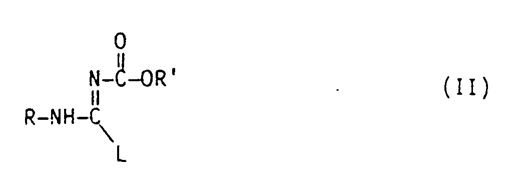 (dans laquelle R et R' ont les sens indiqués ci-dessus, et L représente un groupe partant) avec un composé de formule générale (III) : 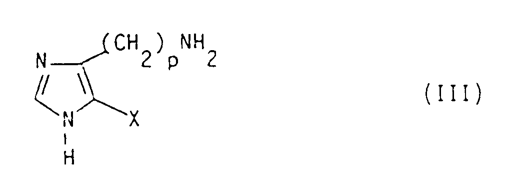 (dans laquelle X et p sont définis comme ci-dessus) pour obtenir un composé de formule générale (1), ou bien
(2) on fait réagir un composé de formule générale (IV) : 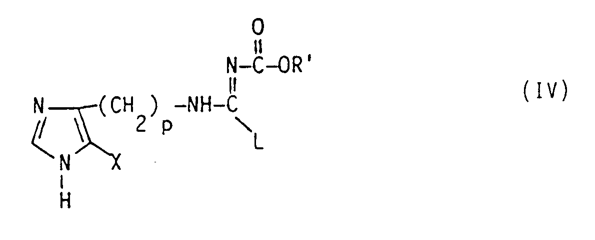 (dans laquelle R', X et p ont les sens indiqués ci-dessus, et L représente un groupe partant) avec un composé de formule générale (V) : 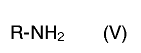 (dans laquelle R a le sens indiqué ci-dessus) pour obtenir un composé de formule générale (1), et en ce qu'on transforme éventuellement les composés obtenus selon (I) ou (II), en opérant de façon connue en soi, en leur sels physiologiquement acceptable.

27. Médicament, caractérisé en ce qu'il contient l'un des composés selon les revendications 1 à 25 et au moins un support ou excipient inerte, pharmaceutiquement acceptable, ou un diluant inerte, pharmaceutiquement acceptable.

## Revendications (Revendications pour l'(les) Etat(s) contractant(s) suivant(s) : suivants : ES, GR)

1. Procédé pour préparer des dérivés de guanidine de formule générale (I): 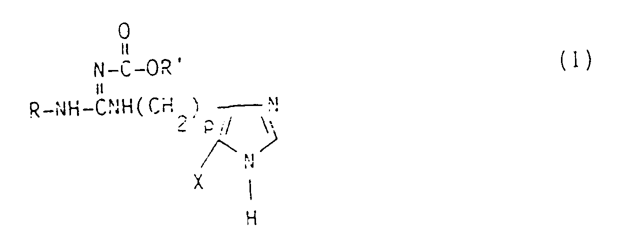 dans laquelle
(a) R représente le groupement 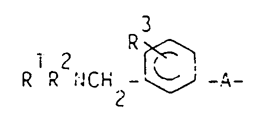 dans lequel R¹ et R², qui peuvent être identiques ou différents, représentent à chaque fois un atome d'hydrogène, un groupe alkyle linéaire en Ci à C₆ ou alkyle en C₅ ou C_{6,} ou bien R¹ et R² forment avec l'atome d'azote auquel ils sont reliés, un noyau coté alicyclique, hétérocyclique, comportant 5 à 10 chaînons (pentagonal à décagonal) ; R³ représente un atome d'hydrogène, un atome d'halogène ou un groupe alcoxy en Ci à C₃ ; A représente le groupement -O-(CH₂)ₖ-,-O-CH₂CH-(OH)CH₂-, -0-CH₂CH(CH₃)-CH₂-, -CH₂-O-CH,-CH(OH)-CH₂- ou -O-CH₂-CH(OH)-CH(OH)-CH₂, l'indice a valant 3 ou 4, ou bien
(b) R représente le groupement 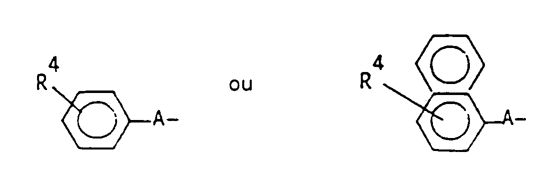 dans lequel R⁴ représente un atome d'hydrogène, un atome d'halogène fixé de préférence en position para par rapport à A, un groupe alcoxy en Ci à C₃ ou alkyle en C₁ à C_{3,} et A a le sens indiqué ci-dessus en (a), ou bien
(c) R représente un groupement 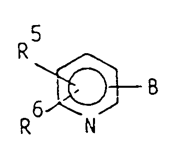 dans lequel R⁵ et R⁶, qui peuvent être identiques ou différents, représentent à chaque fois un atome d'hydrogène, un atome d'halogène ou un groupe alkyle linéaire en C₁ à C₃ ou alcoxy linéaire en C₁ à C₃ ; B peut être fixé en position 2, 3 ou 4 du noyau pyridine et représente le groupement 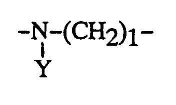 ou -(CH₂)ₘ-, dans lequel I vaut 2, 3 ou 4 et m vaut 2, 3, 4 ou 5, et Y représente un atome d'hydrogène ou un groupe alkyle linéaire en C₁ à C_{3,} ou bien
(d) R représente le groupement 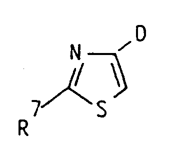 dans lequel R⁷ représente le groupe (R¹ R²)N-CH₂-, (H2 N)₂ C = N-, 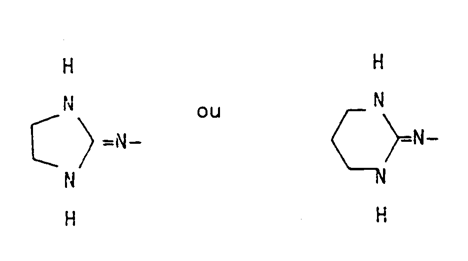 où R¹ et R² ont le sens indiqué ci-dessus en (a) ; D représente le groupement -CH₂-S-(CH₂)ₙ- ou -(CH₂)ₒ-, l'indice n valant 2 ou 3 et l'indice o valant 2, 3 ou 4, ou bien
(e) R représente le groupement 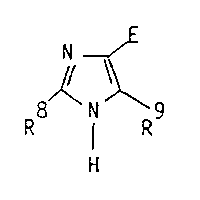 dans lequel R⁸ représente un atome d'hydrogène, un groupe benzyle éventuellement substitué par un atome d'halogène, le groupement (R¹ R²)N-CH₂- ou un groupe amino, les symboles R¹ et R² ayant le sens indiqué ci-dessus en (a); R⁹ représente un atome d'hydrogène, un groupe alkyle linéaire en Ci à C₃ ou alkylthio en Ci à C₃ ; E représente le groupement -CH₂-S-(CH₂)ₙ-, 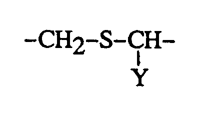 (CH2)_{n'}- ou 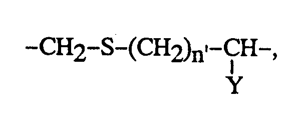 l'indice n valant 2 ou 3 et n' valant 1, 2 ou 3, et Y a le sens indiqué ci-dessus en (c) ; ou bien
(f) R représente le groupement 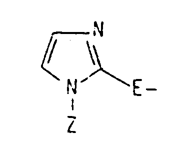 dans lequel Z représente un atome d'hydrogène ou un groupe alkyle linéaire en Ci à C_{3,} et E a le sens indiqué ci-dessus en (e), ou bien
(g) R représente le groupement R"-A'-B'-, dans lequel R" représente un groupe phényle (non substitué ou substitué par de l'halogène, par un groupe alkyle linéaire en Ci à C₃ ou alcoxy linéaire en Ci à C₃) ou un groupe naphtyle (non substitué ou substitué par de l'halogène, par un groupe alkyle linéaire en C₁ à C₃ ou alcoxy linéaire en C₁ à C₃) ; A' représente une liaison simple ou le groupement -CR^{1'}R²' ou un atome d'azote (substitué par un groupe phényle, naphtyle, benzyle, pyridinyle, furannyle ou thiophényle, qui est éventuellement substitué par de l'halogène, par un groupe alkyle linéaire en Ci à C₃ ou alcoxy linéaire en Ci à C_{3,} ou bien un atome d'azote substitué par un groupe alkyle linéaire en Ci à C₃), le symbole R¹' représente un atome d'hydrogène ou un groupe méthyle ; R^{2'} représentant un groupe phényle (qui est éventuellement substitué par de l'halogène, par un groupe alkyle linéaire en Ci à C₃ ou alcoxy linéaire en Ci à C₃) ou un groupe pyridinyle, furannyle ou thiophényle (qui est éventuellement substitué par de l'halogène, par un groupe alkyle linéaire en Ci à C₃ ou par un groupe alcoxy linéaire en Ci à C₃); B' représente le groupement -CH(Y)-S-(CH₂)ₘ-,-CH₂-S-CH₂-CH(Y)-CH₂-, -CH₂-S-CH(Y)-CH₂-, -CH₂-S-CH₂-CH(Y)-, - (CH₂)_{n''}-, -(CH2)-CH(Y)-, -(CH₂)_{n''}-CH(Y)-, -O-CH₂)₂-, -CH₂-O-(CH₂)o'-, -CH₂-O-CH₂-CH(Y)-CH₂-, -O-CH₂-CH(Y)-, -0-CH(Y)-CH₂-, -S-(CH₂)q-, -S-CH₂-CH(Y)-, -S-CH(Y)-CH₂ ou -S-CH₂-CH(Y)-CH₂-, où Y représente un atome d'hydrogène ou un groupe alkyle linéaire en Ci à C_{3,} n' et o' valent chacun 2 ou 3 ; n" et q valent chacun 2, 3, 4 ou 5 ; ou bien
(h) R représente le groupement R"'-A"-B"-, dans lequel R"' représente un groupe pyridinyle, imidazolyle, pyrimidinyle, thiophényle, furannyle, benzimidazolyle ou quinolinyle (non substitués ou substitués par de l'halogène, par un groupe alkyle linéaire en Ci à C₃ ou alcoxy linéaire en Ci à C₃), sur lequel un noyau phénylique peut éventuellement être condensé ; A" représente une liaison simple ou le groupe -CR^{1'}R^{2'} ou un atome d'azote (substitué par un groupe phényle, naphtyle, benzyle, pyridinyle, benzimidazolyle, thiophényle ou furannyle, qui est éventuellement substitué par de l'halogène, par un groupe alkyle linéaire en Ci à C₃ ou alcoxy linéaire en Ci à C₃) ou un atome d'azote substitué par un groupe alkyle linéaire en C₁ à C_{3,} le symbole R^{1'} représentant un atome d'hydrogène ou un groupe méthyle, R^{2'} représentant un groupe phényle (qui est éventuellement substitué par de l'halogène, par un groupe alkyle linéaire en C₁ à C₃ ou par un groupe alcoxy linéaire en C₁ à C₃) ou un groupe benzimidazolyle, pyridinyle, thiophényle ou furannyle (qui est éventuellement substitué par de l'halogène, par un groupe alkyle linéaire en C₁ à C₃ ou alcoxy linéaire en C₁ à C₃); B" représente le groupement -CH(Y)-S-(CH₂)_{m'}-, -CH2-S-CH2-CH(Y)-CH2-, CH₂-S-CH(Y)-CH₂-, -(CH₂)ₙ,,-CH(Y)-, -CH₂-S-CH₂-CH(Y)-, -(CH2)_{n''}-,0(CH2)_{n"}-' -S-CH2-CH(Y)-, -S-CH(Y)-CH₂-, -S-(CH₂)q- ou -S-CH₂-CH(Y)-CH₂-, le symbole Y représentant un atome d'hydrogène ou un groupe alkyle linéaire en C₁ à C_{3,} m' vaut 2 ou 3 et n" et q valent chacun 2, 3, 4 ou 5, R' représente un groupe alkyle en C₁ à C₆, linéaire ou ramifié (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 et X représente un atome d'hydrogène ou un groupe méthyle; ainsi que les sels physiologiquement acceptables de cet ester,
procédé caractérisé en ce que :
(1) on fait réagir un composé de formule générale (II): 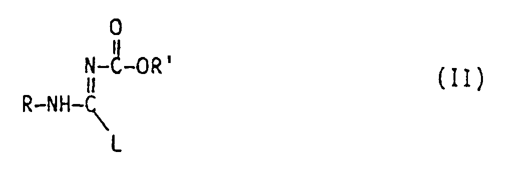 (dans laquelle R et R' ont les sens indiqués ci-dessus, et L représente un groupe partant) avec un composé de formule générale (III): 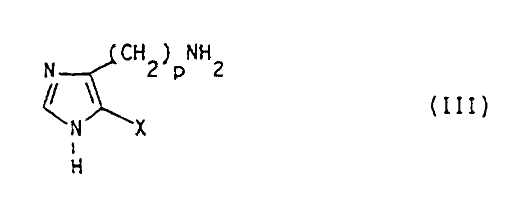 (dans laquelle X et p sont définis comme ci-dessus) pour obtenir un composé de formule générale (1), ou bien
(2) on fait réagir un composé de formule générale (IV) : 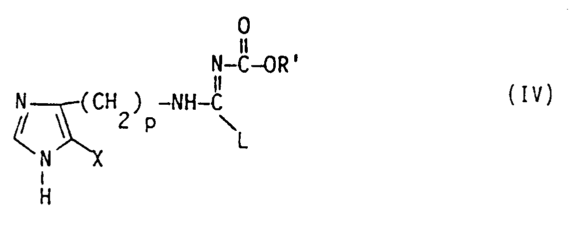 (dans laquelle R', X et p ont les sens indiqués ci-dessus, et L représente un groupe partant) avec un composé de formule générale (V) : 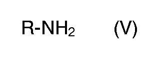 (dans laquelle R a le sens indiqué ci-dessus) pour obtenir un composé de formule générale (I), et en ce qu'on transforme éventuellement les composés obtenus selon (I) ou (II), en opérant de façon connue en soi, en leur sels physiologiquement acceptable.

2. Procédé selon la revendication 1 pour préparer des composés, dans lesquels R représente le groupement 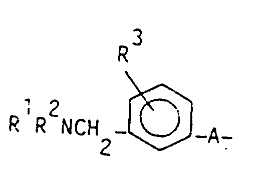 dans lequel R¹ et R², pris avec l'atome d'azote, forment un noyau pyrrolidine ou pipéridine; R³ représente un atome d'hydrogène ; A représente le groupement -O(CH₂)ₖ-, -OCH₂CH(OH)CH₂-, ou OCH₂CH(CH₃)CH₂- ; R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou R' représente un groupe cycloalkyle ; k et p valent 3 et X repré-sente un atome d'hydrogène.

3. Procédé selon la revendication 1 pour préparer des composés, dans lesquels R représente le groupement 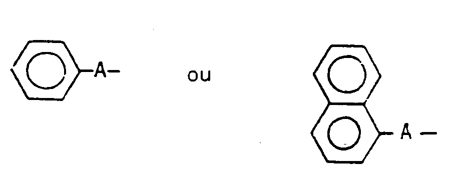 dans lequel, A représente le groupement -O-(CH₂)ₖ-, -0-CH₂CH(OH)CH₂-, -0-CH₂CH(CH₃)-CH₂-, -CH₂-O-CH₂-CH₂-CH(OH)-CH₂- ou -O-CH₂-CH(OH)-CH(OH)-CH₂-;
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou représente un groupe cycloalkyle ; p vaut 2 ou 3 et X représente un atome d'hydrogène ou un groupe méthyle.

4. Procédé selon la revendication 1 pour préparer des composés, dans lesquels R représente le groupement 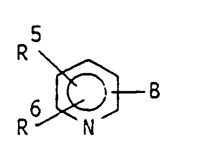 dans lequel R⁵ représente un atome d'halogène ou un atome d'hydrogène fixé en position 5 du noyau pyridine ; R⁶ représente un atome d'hydrogène ou un groupe méthyle ou méthoxy fixé en position 3 du noyau pyridine ; B peut être fixé en position 2, 3 ou 4 du noyau pyridine et représente le groupement 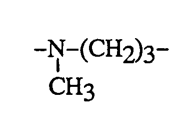 ou bien -(CH₂)₃-₄- ou -NH-(CH₂)₁, où I vaut 2, 3 ou 4, R' représente un reste alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

5. Procédé selon la revendication 1 pour préparer des composés, dans lesquels R représente le groupement 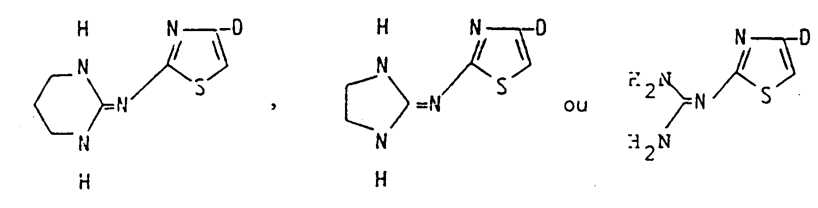 dans lequel D représente le groupement -CH₂-S-(CH₂)₂- ou -(CH₂)₃- ou -(CH₂)₄-, et
R' représente un groupe alkyle en Ci à C₆, linéaire ou ramifié (éventuellement substitué une ou plusieurs fois par un atome d'halogène, par un groupe alkyle en Ci à C₃ et/ou par un reste phényle ou naphtyle) ou un groupe cycloalkyle ; p vaut 2 ou 3 et X représente un atome d'hydrogène ou un groupe méthyle.

6. Procédé selon la revendication 1 pour préparer des composés, dans lesquels R représente le groupement 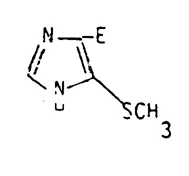 dans lequel E représente le groupement -CH₂-S-(CH₂)₂-, 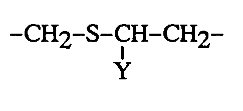 ou 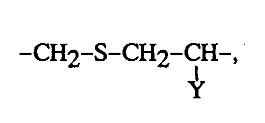 Y représente un atome d'hydrogène ou un groupe alkyle linéaire en Ci à C₃ ;
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou nahtyle) ou un groupe cycloalkyle ; p vaut 2 ou 3 et X représente un atome d'hydrogène ou un groupe méthyle.

7. Procédé selon la revendication 1 pour préparer des composés, dans lesquels R représente le groupement 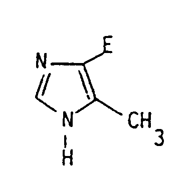 dans lequel E représente le groupement -CH₂-S-(CH₂)₂-, 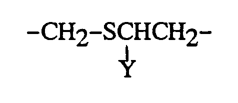 ou 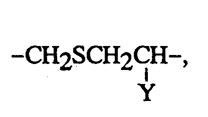 où Y représente un atome d'hydrogène ou un groupe alkyle linéaire en Ci à C_{3;}
R' représente un groupe alkyle en C₁ à C₆, linéaire ou ramifié (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 ; et X représente un atome d'hydrogène ou un groupe méthyle.

8. Procédé selon la revendication 1 pour préparer des composés, dans lesquels R représente le groupement 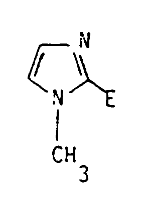 dans lequel E représente le groupement 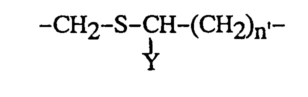 ou -CH₂-S- 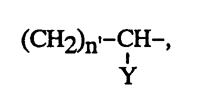 où n' vaut 1, 2 ou 3 et Y représente un atome d'hydrogène ou un groupe alkyle linéaire en Ci à C_{3,}
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

9. Procédé selon la revendication 1 pour préparer des composés, dans lesquels R représente le groupement R"-A'-B'-, par lequel R" représente un groupe phényle non substitué ; A' représente une liaison simple; B' représente le groupement -CH₂-S-(CH₂)_{m'}- ou -CH₂-S-CH₂-CH(Y)-CH₂-,m valant 2 ou 3 et Y représentant un atome d'hydrogène ou un groupe alkyle linéaire en Ci à C_{3,} R' représente un groupe alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

10. Procédé selon la revendication 1 pour préparer des composés, dans lesquels R représente le groupement R"-A'-B'-, dans lequel R" représente un groupe phényle (non substitué ou substitué par de l'halogène, par un groupe alkyle linéaire en Ci à C₃ ou par un groupe alcoxy linéaire en Ci à C₃) _{;} A' représente un atome d'azote (substitué par un groupe phényle, naphtyle, pyridinyle, furannyle ou thiophényle); B' représente le groupement -(CH2)n"-' dans lequel n" vaut 2 ou 3, R' représente un groupe alkyle en Ci à C₆, linéaire ou ramifié (éventuellement substitué une ou plusieurs fois par un atome d'halogène, par un groupe alkyle en Ci à C₃ et/ou par un reste phényle ou naphtyle) ou un groupe cycloalkyle ; p vaut 2 ou 3 et X représente un atome d'hydrogène ou un groupe méthyle.

11. Procédé selon la revendication 1 pour préparer des composés, dans lesquels R représente le groupement R"-A'-B'-, dans lequel R" représente un groupe phényle (non substitué ou substitué par de l'halogène, par un groupe alkyle linéaire en Ci à C₃ ou par un groupe alcoxy linéaire en Ci à C₃) ; A' représente le groupement -CR^{1'}R^{2'}-, dans lequel R^{1'} représente un atome d'hydrogène ou un groupe méthyle, R^{2'} représente un groupe phényle (qui est éventuellement substitué par de l'halogène, par un groupe alkyle linéaire en Ci à C₃ ou par un groupe alcoxy linéaire en Ci à C₃), ou un groupe pyridinyle, furannyle ou thiophényle (qui est éventuellement substitué par de l'halogène, par un groupe alkyle linéaire en Ci à C₃ ou par un groupe alcoxy linéaire en Ci à C₃) ; B' représente le groupement -(CH2)_{n"}-, dans lequel n" vaut 2, 3 ou 4,
R' représente un groupe alkyle, linéaire ou ramifié, en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

12. Procédé selon la revendication 1 pour préparer des composés, dans lesquels R représente le groupement R"'-A"-B"-, dans lequel R"' représente un noyau pyridine (non substitué ou substitué par de l'halogène, par un groupe alkyle linaire en C₁ à C₃ ou par un groupe alcoxy linéaire en C₁ à C₃), A" représente une liaison simple en position 2, 3 ou 4 du noyau pyridine et B" représente le groupement -CH₂-S-(CH₂)_{m'}- ou -CH₂-S-CH₂-CH(Y)-CH₂-, dans lequel m' vaut 2 ou 3 et Y représente un atome d'hydrogène ou un groupe alkyle linéaire en C₁ à C_{3,}
R' représente un groupe alkyle en C₁ à C₆, linéaire ou ramifié (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 et X représente un atome d'hydrogène ou un groupe méthyle.

13. Procédé selon la revendication 1 pour préparer des composés, dans lesquels R représente le groupement R"'-A"-B"-, dans lequel R"' représente un noyau thiophène (non substitué ou substitué par de l'halogène, par un groupe alkyle linéaire en Ci à C₃ ou par un groupe alcoxy linéaire en Ci à C₃), A" représente une liaison simple, B" représente le groupement -CH₂-S-(CH₂)_{m'}- ou -CH₂-S-CH₂-CH(Y)-CH₂-, dans lequel m' vaut 2 ou 3 et Y représente un atome d'hydrogène ou un groupe alkyle linéaire en C₁ à C₃,
R' représente un groupe alkyle, linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle, p vaut 2 ou 3 et X représente un atome d'hydrogène ou un groupe méthyle.

14. Procédé selon la revendication 1 pour préparer des composés, dans lesquels R représente le groupement R"'-A"-B"-, dans lequel R"' représente un noyau pyridine (non substitué ou substitué par de l'halogène, par un groupe alkyle linéaire en Ci à C₃ ou par un groupe alcoxy linéaire en Ci à C₃), A" représente le groupement -CR^{1'}R^{2'}, dans lequel R1' représente un atome d'hydrogène ou un groupe alkyle, R^{2'} représente un groupe phényle (qui est éventuellement substitué par de l'halogène, par un groupe alkyle linéaire en Ci à C₃ ou par un groupe alcoxy linéaire en Ci à C₃) ou un groupe pyridinyle, furannyle, thiophényle ou benzimidazolyle (qui est éventuellement substitué par de l'halogène, par un groupe alkyle linéaire en Ci à C₃ ou par un groupe alcoxy linéaire en Ci à C_{3,} B" représente le groupement -(CH₂)_{n"}-_{'} dans lequel n" vaut 2, 3, 4 ou 5,
R' représente un groupe alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle, p vaut 2 ou 3 et X représente un atome d'hydrogène ou un groupe méthyle.

15. Procédé selon la revendication 1 pour préparer des composés, dans lesquels R représente le groupement R"'-A"-B"-, dans lequel R"' représente un noyau pyridine (non substitué ou substitué par de l'halogène, par un groupe alkyle linéaire en Ci à C₃ ou par un groupe alcoxy linéaire en Ci à C₃), A" représente une liaison simple en position 2, 3 ou 4 du noyau pyridine, B" représente le groupement -(CH2)_{n"}-_{'} dans lequel n" vaut 2, 3, 4 ou 5,
R' représente un groupe alkyle, linéaire ou ramifié, en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle

16. Procédé selon la revendication 1 pour préparer des composés, dans lesquels R représente le groupement R"'-A"-B"-, dans lequel R"' représente un noyau pyridine (non substitué ou substitué par de l'halogène, par un groupe alkyle linéaire en Ci à C₃ ou alcoxy linéaire en Ci à C₃), A" représente une liaison simple ou le groupement -CR^{1'}R^{2'} ou un atome d'azote (substitué par un groupe phényle, naphtyle, benzyle, pyridinyle, benzimidazolyle, thiophényle ou furannyle, qui est éventuellement lui-même substitué par de l'halogène, par un groupe alkyle linéaire en Ci à C₃ ou par un groupe alcoxy linéaire en Ci à C₃) ou substitué par un groupe alkyle linéaire en C₁ à C_{3,} et R^{1'} représente un atome d'hydrogène ou un groupe méthyle, R^{2'} représente un groupe phényle (qui est éventuellement substitué par de l'halogène, par un groupe alkyle linéaire en C₁ à C₃ ou par un groupe alcoxy linéaire en C₁ à C₃) ou un groupe benzimidazolyle, pyridinyle, thiophényle ou furannyle (qui est éventuellement substitué par de l'halogène, par un groupe alkyle linéaire en C₁ à C₃ ou par un groupe alcoxy linéaire en C₁ à C₃), B" représente le groupement -(CH₂)_{n"}-_{'} dans lequel n" vaut 2, 3, 4 ou 5,
R' représente un groupe alkyle linéaire ou ramifié en C₁ à C₆ (éventuellement substitué une ou plusieurs fois par un atome d'halogène, par un groupe alcoxy en C₁ à C₃ et/ou par un reste phényle ou naphtyle) ou un groupe cycloalkyle ; p vaut 2 ou 3 et X représente un atome d'hydrogène ou un groupe méthyle.

17. Procédé selon la revendication 1 pour préparer des composés, dans lesquels R représente R"'-A"-B"-, dans lequel R"' représente le groupement 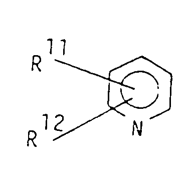 dans lequel, R¹¹ et R¹² représentent chacun, indépendamment l'un de l'autre, un atome d'halogène, un groupe alkyle linéaire en Ci à C₃ ou un groupe alcoxy linéaire en Ci à C₃ ; A" représente le groupement -CR^{1'}R^{2'}, dans lequel R^{1'} représente un atome d'hydrogène ou un groupe méthyle et R^{2'} représente un groupe phényle (non substitué ou substitué une à trois fois par un atome d'halogène, par un groupe alkyle linéaire en Ci à C₃ ou par un groupe alcoxy linéaire en Ci à C₃ ; ou bien A" représente un atome d'azote (qui est substitué par un groupe phényle, naphtyle, pyridinyle, furannyle, thiophényle ou benzimidazolyle ou par un atome d'hydrogène) ; B" représente, quand A" représente le groupe -CR^{1'}R^{2'}, le groupement -(CH₂)ₙ-, -O(CH₂)₂- ou -SCH₂CH₂-, et B" représente, quand A" représente un atome d'azote (substitué par un groupe phényle, naphtyle, pyridinyle, furannyle, thiophényle ou benzimidazolyle ou par un atome d'hydrogène), le groupement -(CH₂)ₙ-, dans lequel n vaut 2 ou 3, et
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

18. Procédé selon la revendication 1 pour préparer des composés, dans lequels R représente le groupement R"'-A"-B"-, dans lequel R"' représente le groupement 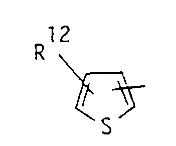 dans lequel R¹² représente un atome d'hydrogène, un atome d'halogène, un groupe alkyle linéaire en C₁ à C_{3 ,} le groupement (CH₃)₂-NCH₂- ou 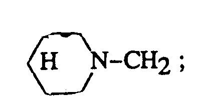 A" représente le groupement -CR^{1'}R^{2'}, dans lequel R^{1'} représente un atome d'hydrogène ou un groupe méthyle et R^{2'} représente un groupe phényle (non substitué ou substitué une à trois fois par de l'halogène, par un groupe alkyle linéaire en Ci à C₃ ou par un groupe alcoxy linéaire en Ci à C₃) ou bien A" représente un atome d'azote (qui est substitué par un groupe phényle, naphtyle, pyridinyle, furannyle, thiophényle ou benzimidazolyle ou par un atome d'hydrogène) ; B" représente le groupement -CH₂-S-CH₂-CH₂-, -CH₂-S-CH₂-CH(CH₃)-CH₂-, -CH₂-S-CH(CH₃)-CH₂- ou -CH₂-S-CH₂-CH(CH₃)-, et
R' représente un groupe alkyle en Ci à C₆, linéaire ou ramifié (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

19. Procédé selon la revendication 1 pour préparer des composés, dans lesquels R représente le groupement R"'-A"-B"-, dans lequel R"' représente le groupement 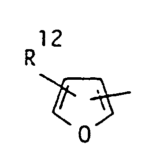 dans lequel R¹² représente un atome d'hydrogène, un atome d'halogène, un groupe alkyle linéaire en Ci à C_{3,} le groupement (CH₃)₂-NCH₂- ou 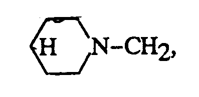 A" représente une liaison simple en position 2 ; B" représente le groupement -CH₂-S-CH₂-CH₂-, -CH₂-S-CH₂-CH(CH₃)-CH₂-, -CH₂-S-CH(CH₃)-CH₂- ou -CH₂-S-CH₂-CH₂-CH(CH₃), et
R' représente un groupe alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

20. Procédé selon la revendication 1 pour préparer des composés, dans lesquels R représente le groupement R"'-A"-B"-, dans lequel R"' représente le groupement 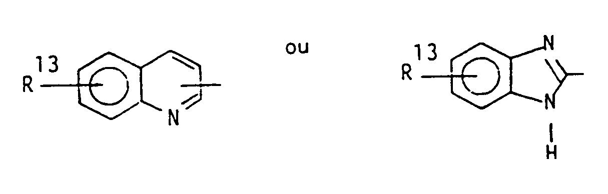 où R¹³ représente un atome d'hydrogène, un atome d'halogène, un groupe alkyle linéaire en Ci à C₃, le groupement (CH₃)-NCH₂ ou H N-CH₂, A" représente une liaison simple, B" représente le groupement -CH₂-S-CH₂-CH₂-, -CH₂-S-CH₂-CH(CH₃)-CH₂-, -CH₂-S-CH(CH₃-CH₂-, ou -CH₂-S-CH₂-CH(CH₃), et
R' représente un groupe alkyle linéaire ou ramifié en Ci à C₆ (éventuellement substitué une ou plusieurs fois par un atome d'halogène, par un groupe alcoxy en Ci à C₃ et/ou par un reste phényle ou naphtyle) ou un groupe cycloalkyle ; p vaut 2 ou 3 et X représente un atome d'hydrogène ou un groupe méthyle.

21. Procédé selon la revendication 1 pour préparer la Ni⁻[3-(imidazol-4-yl)propyl]-N²⁻[4-(pyridine-2-yl)-butyl]-N³⁻méthoxycarbonylguanidine et ses sels physiologiquement acceptables.

22. Procédé selon la revendication 1 pour préparer la Ni⁻[3-(imidazol-4-yl)propyl]-N²⁻[4-(pyridine-2-yl)-butyl]-N³⁻éthoxycarbonylguanidine et ses sels physiologiquement acceptables.

23. Procédé selon la revendication 1 pour préparer la N¹-[3-(imidazol-4-yl)propyl]-N²-[3-(4-fluorophényl)-3-(pyridine-2-yl)propyl]-N³⁻méthoxycarbonylguanidine et ses sels physiologiquement acceptables.

24. Procédé selon la revendication 1 pour préparer la N¹-[3-(imidazol-4-yl)propyl]-N²-[3-(4-fluorophényl)-3-(pyridine-2-yl)propyl]-N³⁻éthoxycarbonylguanidine et ses sels physiologiquement acceptables.

25. Procédé selon la revendication 1 pour préparer la N¹-[3-(imidazol-4-yl)propyl]-N²-[3-(4-fluorophényl)-3-(pyridine-2-yl)propyl]-N³⁻butoxycarbonylguanidine et ses sels physiologiquement acceptables.
